# EUROPEAN PATENT APPLICATION

(11) **EP 3 805 260 A1**
(43) Date of publication of application: **14.04.2021**
(21) Application number: 20184442.0
(22) Date of filing: 27.05.2015
(51) Int. Cl.: C07K 14/72, G16B 15/00

(54) **WATER-SOLUBLE TRANS-MEMBRANE PROTEINS AND METHODS FOR THE PREPARATION AND USE THEREOF**

(30) Priority: 18.02.2015 US 201562117550 P; 26.03.2015 US 201514669753; 26.03.2015 WO PCT/US2015/022780
(62) Divisional of application: 15729292.1
(71) Applicant: Massachusetts Institute of Technology, Cambridge, MA 02139 (US)
(72) Inventor: ZHANG, Shuguang, Lexington, MA 02421-4142 (US); TAO, Fei, Cambridge, MA 02140 (US)
(74) Representative: Atkins, James Gordon John

(57) **Abstract**

The present invention is directed to water-soluble membrane proteins, methods for the preparation thereof and methods of use thereof.

## Description

### RELATED APPLICATIONS

This application claims priority to of U.S. Patent Application No. 14/669,753 and to International Application No. PCT/US2015/022780, both filed on March 26, 2015; both of which claim the benefit of the filing dates under 35 U.S.C. 119(e) of U.S. Provisional Application No. 62/117,550 filed on February 18, 2015, U.S. Provisional Application No. 61/993,783 filed on May 15, 2014, and U.S. Provisional Application No. 61/971,388 filed on March 27, 2014.

This application also claims the benefit of the filing date under 35 U.S.C. 119(e) to U.S. Provisional Application No. 62/117,550 filed on February 18, 2015, U.S. Provisional Application No. 61/993,783 filed on May 15, 2014, and U.S. Provisional Application No. 61/971,388 filed on March 27, 2014.

The entire contents of each of the above referenced applications, including all drawings and sequence listings, are incorporated herein by reference.

### BACKGROUND OF THE INVENTION

Membrane proteins play vital roles in all living systems. Approximately ∼30% of all genes in almost all sequenced genomes code for membrane proteins. However, our detailed understanding of their structure and function lags far behind that of soluble proteins. As of March 2015, there are over 100,000 structures in the Protein Data Bank. However, there are only 945 membrane protein structures with 530 unique structures including 28 G-protein coupled receptors and no tetraspanin membrane proteins.

There are several bottlenecks in elucidating the structure and function of membrane receptors and their recognition and ligand-binding properties although they are of great interest. The most critical and challenging task is that it is extremely difficult to produce milligram quantities of soluble and stable receptors. Inexpensive large-scale production methods are desperately needed, and have thus been the focus of extensive research. It is only possible to conduct detailed structural studies once these preliminary obstacles have been surmounted.

Zhang *et al.* (U.S. Patent No.: 8,637,452), incorporated herein by reference, describes an improved process for water solubilizing GPCRs wherein certain hydrophobic amino acids located in the transmembrane regions were substituted by polar amino acids. However, the process is labor-intensive. Further, while the modified transmembrane regions met the water-soluble criteria, improvements in water solubility and ligand binding are desired. Therefore, there is a need in the art for improved methods of studying G-protein coupled receptors.

### SUMMARY OF THE INVENTION

The present invention is directed to a method of designing, selecting and/or producing water-soluble membrane proteins and peptides, peptides (and transmembrane domains) designed, selected or produced therefrom, compositions comprising said peptides, and methods of use thereof. In particular, the method relates to a process for designing a library of water soluble membrane peptides, such as GPCR variants and tetraspanin membrane proteins, using the "QTY Principle," changing the water-insoluble amino acids (Leu, Ile, Val and Phe, or the simple letter code L, I, V, F) into water-soluble, non-ionic amino acids (Gln, Thr and Tyr, or the simple letter code Q, T, Y). Furthermore, two additional non-ionic amino acids Asn (N) and Ser (S) may also be used for the substitution for L, I and V but not for F. In the embodiments discussed below, it is to be understood that Asn (N) and Ser (S) are envisioned as being substitutable for Q and T (as a variant is described) or L, I or V (as a native protein is described). For the purposes of brevity, however, the application does not further elaborate the details of these alternative embodiments as these are known to those skilled in the art as a result of the teaching herein.

The invention encompasses a modified, synthetic, and/or non-naturally occurring, α-helical domain(s) and water-soluble polypeptide (e.g., "sGPCR") comprising such modified α-helical domain(s), wherein the modified α-helical domain(s) comprise an amino acid sequence in which a plurality of hydrophobic amino acid residues (L, IV, F) within a α-helical domain of a native membrane protein are replaced with hydrophilic, non-ionic amino acid residues (Q, T, T, Y, respectively, or "Q, T, Y") and/or N and S. The invention also encompasses a method of preparing a water-soluble polypeptide comprising replacing a plurality of hydrophobic amino acid residues (L, I, V, F) within the α-helical domain(s) of a native membrane protein with hydrophilic, non-ionic amino acid residues (Q/N/S, T/N/S, Y). The invention additionally encompasses a polypeptide prepared by replacing a plurality of hydrophobic amino acid residues (L, I, V, F) within the α-helical domain of a native membrane protein with hydrophilic, non-ionic amino acid residues (Q/N/S, T/N/S, Y, respectively). The variant can be characterized by the name of the parent or native protein (e.g., CXCR4) followed by the abbreviation "QTY" (e.g., CXCR4-QTY).

Thus one aspect of the invention provides a method of operating a computer program to execute a scripted procedure to design a water-soluble variant of a membrane protein (e.g., a G Protein-Coupled Receptor (GPCR)), the method comprising: (1) entering a sequence of the membrane protein (e.g., GPCR) for analysis; (2) obtaining a variant of the membrane protein (e.g., GPCR), wherein a plurality of hydrophobic amino acids in the transmembrane (TM) domain alpha-helical segments ("TM regions") of the membrane protein (e.g., GPCR) are substituted, wherein: (a) said hydrophobic amino acids are selected from the group consisting of Leucine (L), Isoleucine (I), Valine (V), and Phenylalanine (F); (b) each said Leucine (L) is independently substituted by Glutamine (Q), Asparagine (N), or Serine (S); (c) each said Isoleucine (I) and said Valine (V) are independently substituted by Threonine (T), Asparagine (N), or Serine (S); and, (d) each said Phenylalanine is substituted by Tyrosine (Y); and, subsequently, (3) obtaining an α-helical secondary structure result for the variant to verify maintenance of α-helical secondary structures in the variant; (4) obtaining a trans-membrane region result for the variant to verify water solubility of the variant, thereby designing the water-soluble variant of the membrane protein (e.g., GPCR).

In certain embodiments, step (3) is performed prior to, concurrently with, or after step (4). Additional steps, as described herein, can be incorporated into the above processing sequence. Processing preferably uses computational steps preformed by a data processing system. The system utilizes automated computational systems and methods to select protein variants.

In certain embodiments, in step (2), one subset of said plurality of hydrophobic amino acids in one and the same TM region of the GPCR are substituted to generate one member of a library of potential variants, and one or more different subsets of said plurality of hydrophobic amino acids are substituted to generate additional members of the library. In certain embodiments, the method may further comprising ranking all members of said library based on a combined score, wherein the combined score is a weighed combination of the α-helical secondary structure prediction result and the trans-membrane region prediction result. In certain embodiments, the method further comprises ranking the variant using a ranking function. In certain embodiments, the ranking function may include a secondary structure component and a water solubility component. For example, the ranking function may include a weighting value for the secondary structure component and/or the water solubility component. In certain embodiments, the method further comprises performing the method with a data processor, which may further comprise a memory connected thereto.

In certain embodiments, the method may further comprising selecting N members with the highest combined scores to form a first library of potential variants for said TM region, wherein N is a pre-determined integer (e.g., 3, 4, 5, 6, 7, 8, 9, 10, 11, 12, 13, 14, 15, 16, 17, 18, 19, 20 or more). In certain embodiments, the method may further comprising generating one library of potential variants for 1, 2, 3, 4, 5, or all 6 other TM regions of the GPCR. In certain embodiments, the method may further comprising replacing two or more TM regions of the GPCR with corresponding TM regions from the libraries of potential variants, to create a library of combinatory variants. In certain embodiments, the method further comprises producing / expressing said combinatory variants. In certain embodiments, the method further comprises testing said combinatory variants for ligand binding (*e.g.*, in yeast two-hybrid system), wherein those having substantially the same ligand binding compared to that of the GPCR are selected. In certain embodiments, the method further comprises testing said combinatory variants for a biological function of the GPCR, wherein those having substantially the same biological function compared to that of the GPCR are selected.

Certain water-soluble polypeptides of the invention possess the ability to bind the ligand which normally binds to the wild type or native membrane protein (e.g., GPCR). In certain embodiments, the amino acids within potential ligand binding sites of the native membrane protein (e.g., GPCRs) are not replaced and/or the sequences of the extracellular and/or intracellular domains of the native membrane proteins (e.g., GPCRs) are identical.

The (non-ionic) hydrophilic residues (which replace one or more hydrophobic residues in the α-helical domain of a native membrane protein) are selected from the group consisting of: glutamine (Q), threonine (T), tyrosine (Y), Asparagine (N), and serine (S), and any combinations thereof. In additional aspects, the hydrophobic residues selected from leucine (L), isoleucine (I), valine (V), and phenylalanine (F) are replaced. In certain embodiments, the phenylalanine residues of the α-helical domain of the protein are replaced with tyrosine; each of the isoleucine and/or valine residues of the α-helical domain of the protein are independently replaced with threonine (or S or N); and/or each of the leucine residues of the α-helical domain of the protein are independently replaced with glutamine (or S or N).

In certain embodiments, substantially all (*e.g.*, 96%, 97%, 98%, 99%, or 100%), or 30%, 40%, 50%, 60%, 70%, 75%, 80%, 85%, 90%, 95% of said leucines are substituted by glutamines. In certain embodiments, substantially all (*e.g.*, 96%, 97%, 98%, 99%, or 100%), or 30%, 40%, 50%, 60%, 70%, 75%, 80%, 85%, 90%, 95% of said isoleucines are substituted by threonines. In certain embodiments, substantially all (*e.g.*, 96%, 97%, 98%, 99%, or 100%), or 30%, 40%, 50%, 60%, 70%, 75%, 80%, 85%, 90%, 95% of said valines are substituted by threonines. In certain embodiments, substantially all (*e.g.*, 96%, 97%, 98%, 99%, or 100%), or 30%, 40%, 50%, 60%, 70%, 75%, 80%, 85%, 90%, 95% of said phenylalanines are substituted by tyrosines. In certain embodiments, one or more (*e.g,*, 1, 2, or 3) said leucines are not substituted. In certain embodiments, one or more (*e.g,*, 1, 2, or 3) said isoleucines are not substituted. In certain embodiments, one or more (*e.g,*, 1, 2, or 3) said valines are not substituted. In certain embodiments, one or more (*e.g,*, 1, 2, or 3) said phenylalanines are not substituted.

In certain embodiments, the library of combinatory variants comprises less than about 2 million members. In certain embodiments, the sequence of the GPCR comprises information about the TM regions of the GPCR. In certain embodiments, the sequence of the GPCR is obtained from a protein structure database (e.g., PDB, UniProt). In certain embodiments, the TM regions of the GPCR are predicted based on the sequence of the GPCR. For example, the TM regions of the GPCR can be predicted using TMHMM 2.0 (TransMembrane prediction using Hidden Markov Models) software module / package. In certain embodiments, the TMHMM 2.0 software module / package utilizes a dynamic baseline for peak searching.

In certain embodiments, the method further comprises providing a polynucleotide sequence for each variants of the GPCR. The polynucleotide sequence may be codon optimized for expression in a host (e.g., a bacterium such as *E. coli,* a yeast such as *S*. *cerevisae* or *S*. *pombe*, an insect cell such as Sf9 cell, a non-human mammalian cell, or a human cell).

In certain embodiments, the scripted procedure can comprise VBA scripts. In certain embodiments, the scripted procedure is operable in a Linux system (e.g., Ubuntu 12.04 LTS), a Unix system, a Microsoft Windows operating system, an Android operating system, or an Apple iOS operating system. Different programming language including C⁺⁺, Java Script, MATLAB, etc. can be used in conjunction with implementations of the present invention. Coded instructions can be stored on a memory device, such as a non-transitory computer readable medium, that can be used with a computer system known to those skilled in the art.

In certain embodiments, the α-helical domain is one of 7-transmembrane α-helical domains in a native membrane protein is a G-protein coupled receptor (GPCR). In some embodiments, the GPCR is selected from the group consisting of: purinergic receptors (P2Y₁, P2Y₂, P2Y₄, P2Y₆), M₁ and M₃ muscarinic acetylcholine receptors, receptors for thrombin (protease-activated receptor (PAR)-1, PAR-2), thromboxane (TXA₂), sphingosine 1-phosphate (S1P₂, S1P₃, S1P₄ and S1P₅), lysophosphatidic acid (LPA₁, LPA₂, LPA₃), angiotensin II (AT₁), serotonin (5-HT_{2c} and 5-HT₄), somatostatin (sst₅), endothelin (ET_{A} and ET_{B}), cholecystokinin (CCK₁), V₁ₐ vasopressin receptors, D₅ dopamine receptors, fMLP formyl peptide receptors, GAL₂ galanin receptors, EP₃ prostanoid receptors, A₁ adenosine receptors, α₁ adrenergic receptors, BB₂ bombesin receptors, B₂ bradykinin receptors, calcium-sensing receptors, chemokine receptors, KSHV-ORF74 chemokine receptors, NK₁ tachykinin receptors, thyroid-stimulating hormone (TSH) receptors, protease-activated receptors, neuropeptide receptors, adenosine A2B receptors, P2Y purinoceptors, metabolic glutamate receptors, GRK5, GPCR-30, and CXCR4.

In other embodiments, the native membrane protein or membrane protein is an integral membrane protein. In a further aspect, the native membrane protein is a mammalian protein. The proteins of the invention are preferably human. In certain embodiments, references to specific GPCR proteins (e.g., CXCR4) refer to mammalian GPCRs, such as non-human mammalian GPCRs, or human GPCRs.

In some embodiments, the α-helical domain is one of 7-transmembrane α-helical domains in a G-protein coupled receptor (GPCR) variant modified, for example, in the extracellular or intracellular loops to improve or alter ligand binding, as described elsewhere in the literature. For the purposes of this invention, the word "native" or "wild type" is intended to refer to the protein (or α-helical domain) prior to water solubilization in accordance with the methods described herein.

In certain embodiments, the membrane protein can be a tetraspanin membrane protein characterized by 4 transmembrane alpha-helices. Approximately 54 human tetraspanin membrane proteins have been reviewed and annotated. Many are known to mediate cellular signal transduction events that play a critical role in regulation of cell development, activation, growth and motility. For example, CD81 receptor plays a critical role as the receptor for Hepatitis C virus entry and plasmodium infection. CD81 gene is localized in the tumor-suppressor gene region and can be a candidate for mediating cancer malignancies. CD151 is involved in enhanced cell motility, invasion and metastasis of cancer cells. Expression of CD63 correlates with the invasiveness of ovarian cancer. A characteristic of a tetraspanin membrane protein is a Cysteine-cysteine-glycine motif in the second, or large, extracellular loop.

Another aspect of the invention provides a water-soluble variant of a G Protein-Coupled Receptor (GPCR), wherein: (1) a plurality of hydrophobic amino acids in the transmembrane (TM) domain alpha-helical segments ("TM regions") of the GPCR are substituted, wherein: (a) said hydrophobic amino acids are selected from the group consisting of Leucine (L), Isoleucine (I), Valine (V), and Phenylalanine (F); (b) each said Leucine (L) is independently substituted by Glutamine (Q), Asparagine (N), or Serine (S); (c) each said Isoleucine (I) and said Valine (V) are independently substituted by Threonine (T), Asparagine (N), or Serine (S); and, (d) each said Phenylalanine is substituted by Tyrosine (Y); and, subsequently, (2) all seven TM regions of the variant maintains α-helical secondary structures; and, (3) there is no predicted trans-membrane region.

In certain embodiments, the water-soluble variant comprises one or more amino acid sequences selected from the group consisting of SEQ ID NOs: 4-11, 13-20, 22-29, 31-38, 40-47, 49-56, and 58-64. It may further comprise one or more amino acid sequences selected from the group consisting of SEQ ID NOs: 3, 12, 21, 30, 39, 48, and 57. In certain embodiments, the water-soluble variant binds to a CXCR4 ligand.

In certain embodiments, the water-soluble variant comprises one or more amino acid sequences selected from the group consisting of SEQ ID NOs: 69-76, 78-85, 87, 89-96, 98-105, 107-114 and 116-123. It may further comprise one or more amino acid sequences selected from the group consisting of SEQ ID NOs: 68, 77, 86, 88, 97, 106, 115 and 124. In certain embodiments, the water-soluble variant binds to a CX3CR1 ligand.

In certain embodiments, the water-soluble variant comprises one or more amino acid sequences selected from the group consisting of SEQ ID NOs: 128-135, 137-144, 146-153, 155-162, 164-171, 173 and 175-182. It may further comprise one or more amino acid sequences selected from the group consisting of SEQ ID NOs: 127, 136, 145, 154, 163, 172, 174 and 183. In certain embodiments, the water-soluble variant binds to a CCR3 ligand.

In certain embodiments, the water-soluble variant comprises one or more amino acid sequences selected from the group consisting of SEQ ID NOs: 187-194, 196-203, 205-206, 208, 210-217, 219-225, 227-234. It may further comprise one or more amino acid sequences selected from the group consisting of SEQ ID NOs: 186, 195, 204, 207, 209, 218, 226, and 235. In certain embodiments, the water-soluble variant binds to a CCR5 ligand.

In certain embodiments, the water-soluble variant comprises one or more amino acid sequences selected from the group consisting of SEQ ID NOs: 236-243, 245-252, 254-261, 263-270, 272, 274-281, and 283-290. It may further comprise one or more amino acid sequences selected from the group consisting of SEQ ID NOs: 235, 244, 253, 262, 271, 273, 282 and 291. In certain embodiments, the water-soluble variant binds to a CXCR3 ligand.

In certain embodiments, the water-soluble variant comprises one or more transmembrane domains as set forth in any one of SEQ ID NOs: 2, 67, 126, 185, 327, 293, 295, 297, 299, 301, 303, 305, 307, 309, 311, 313, 315, 317, 319, 321, 323 or 325. In certain embodiments, the variant is water soluble and binds a ligand of a homologous native transmembrane protein.

Another aspect of the invention provides a method of producing a protein in a bacterium (e.g., an *E. coli*), comprising: (a) culturing the bacterium in a growth medium under a condition suitable for protein production; (b) fractioning a lysate of the bacterium to produce a soluble fraction and the insoluble pellet fraction; and, (c) isolating the protein from the soluble fraction; wherein: (1) the protein is a variant G-protein couple receptor (GPCR) of any one of claims 29-46; and, (2) the yield of the protein is at least 20 mg/L (e.g., 30 mg/L, 40 mg/L, 50 mg/L or more) of growth medium.

In certain embodiments, the bacterium is *E. coli* BL21, and the growth medium is LB medium. In certain embodiments, the protein is encoded by a plasmid in the bacterium. In certain embodiments, expression of the protein is under the control of an inducible promoter, such as an inducible promoter inducible by IPTG. In certain embodiments, the lysate is produced by sonication. In certain embodiments, the soluble fraction is produced by centrifuging the lysate at 14,500 × g or more.

Another aspect of the invention provides a method of treatment for a disorder or disease that is mediated by the activity a membrane protein in a subject in need thereof, comprising administering to said subject an effective amount of a water-soluble polypeptide described herein.

In certain embodiments, the water-soluble polypeptide retains the ligand-binding activity of the membrane protein. Examples of disorders and diseases that can be treated by administering a water-soluble peptide of the invention include, but are not limited to, cancer (such as, small cell lung cancer, melanoma, triple negative breast cancer), Parkinson's disease, cardiovascular disease, hypertension, and bronchial asthma.

Another aspect of the invention provides a pharmaceutical composition comprising a therapeutically effective amount of a water-soluble polypeptide of the invention and pharmaceutically acceptable carrier or diluent.

In yet another aspect, the invention provides a cell transfected with a subject water-soluble peptide comprising a modified α-helical domain. In certain embodiments, the cell is an animal cell (e.g., human, non-human mammalian, insect, avian, fish, reptile, amphibian, or other cell), yeast or a bacterial cell.

The invention also includes a computer implemented method performed on a computer system, the method comprising one or more of the methods (or steps thereof) as described herein. Computer systems including a non-transient computer readable medium having computer-executable instructions stored thereon, the computer-executable instructions when executed by the computer system causing the computer system to perform the methods the computer-executable instructions when executed by the computer system causing the computer system to perform the methods contemplated herein. Additionally, computer systems comprising at least one memory to store sequence data and quantitative results described herein and at least one processor coupled to the memory, the processor being configured to perform the methods described herein are contemplated. A user interface, such as a graphical user interface (GUI) in conjunction with an electronic display device can be used to select processing parameters that are operative to control the selection process, including computational methods described herein.

Another aspect of the invention provides a non-transitory computer readable medium having stored thereon a sequence of instructions to perform any of the methods of the invention.

A further aspect of the invention provides a data processing system operative to select a water-soluble variant of a G Protein-Coupled Receptor comprising: a data processor operative to perform substitution of amino acids as in any of the methods of the invention, wherein the system ranks a protein variant with a ranking function.

It should be understood that all embodiments of the invention, including those described only under one aspect of the invention (e.g., screening method), are to be construed to be applicable to all aspects of the invention (e.g., water-soluble proteins or methods of use), and are to be construed to be combinable with any one or more additional embodiments of the invention unless explicitly disclaimed or otherwise improper, as should be readily understood by one of skill in the art.

### BRIEF DESCRIPTION OF THE DRAWINGS

The foregoing and other objects, features and advantages of the invention will be apparent from the following more particular description of the representative embodiments of the invention, as illustrated in the accompanying drawings in which like reference characters refer to the same parts throughout the different views. The drawings are not necessarily to scale, emphasis instead being placed upon illustrating the principles of the invention.

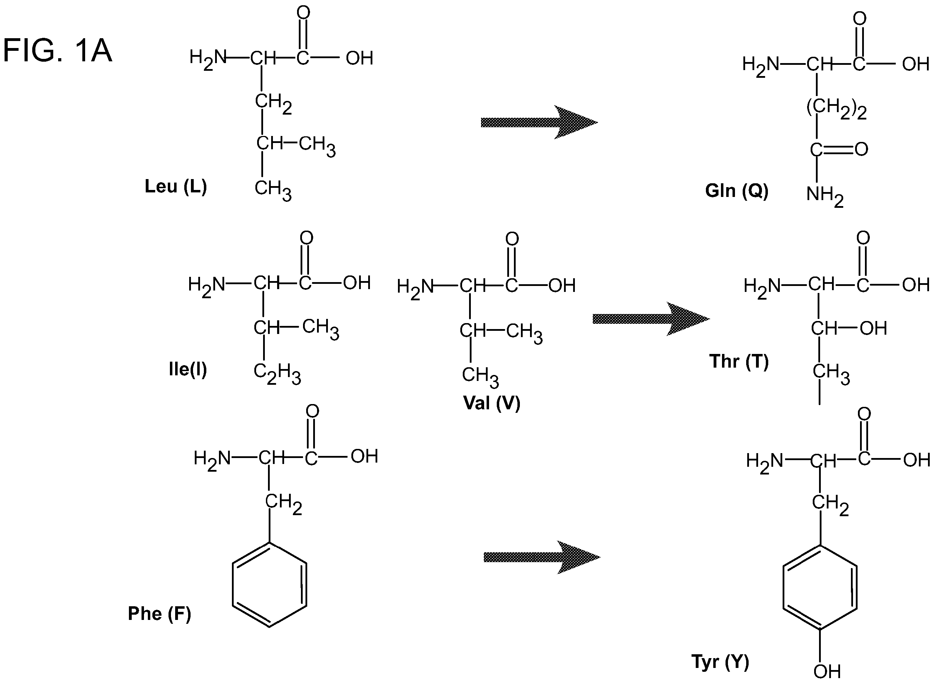

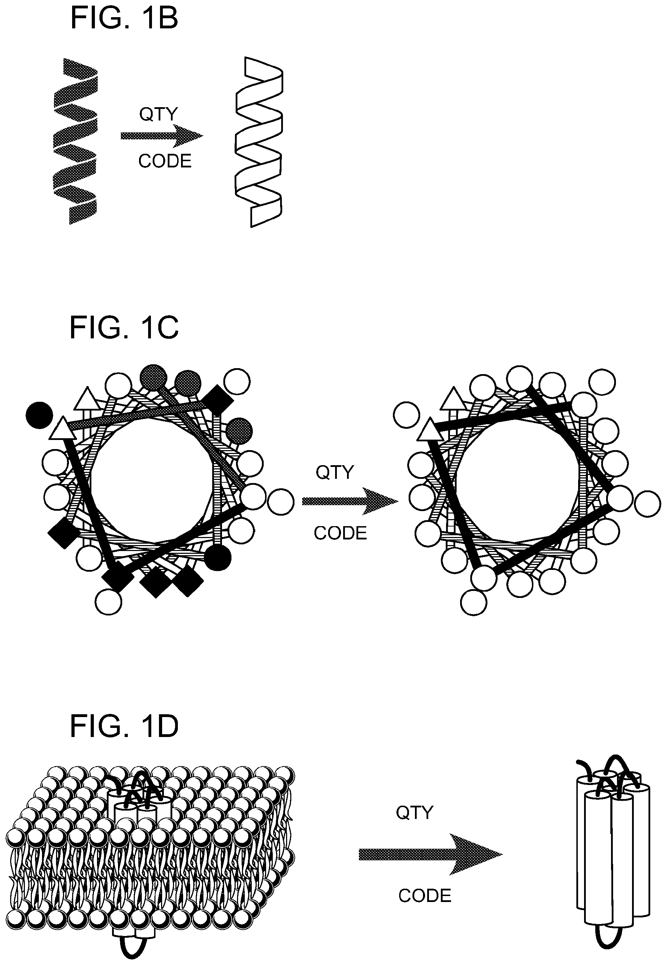

FIGs. 1A-1D is the general illustration for the QTY Code that systematically substitutes the hydrophobic amino acids L, I, V and F to Q, T, T, Y, respectively (FIG. 1A). The molecular shapes of amino acids leucine and glutamine are similar; likewise, molecular shapes of isoleucine and valine are similar to threonine; and molecular shapes of phenylalanine and tyrosine are similar. Leucine, isoleucine, valine and phenylalanine are hydrophobic and cannot bind with water molecules. In contrast, glutamine can bind with 4 water molecules, 2 hydrogen donors, and 2 hydrogen acceptors; the -OH group on threonine and tyrosine can bind to 3 water molecules, 1 hydrogen donor and 2 acceptors. FIG. 1B is a side view of an alpha helix. After applying the QTY Code of systematic amino acid changes, the alpha helix become water-soluble. FIG. 1C is a top view of an alpha helix before and after QTY Code substitution: the helix on the left is the natural membrane helix with mostly hydrophobic amino acids, the helix on the right is the same helix after applying QTY Code substitution. The helix now has most hydrophilic amino acids (FIG. 1D). Before QTY Code, the GPCR membrane proteins are surrounded by hydrophobic lipid molecules to embed them inside the lipid membrane (left portion of FIG. 1D). After applying QTY Code, the GPCR membrane proteins become water-soluble and no long need detergent to surround it for stabilization (right portion of FIG. 1D).

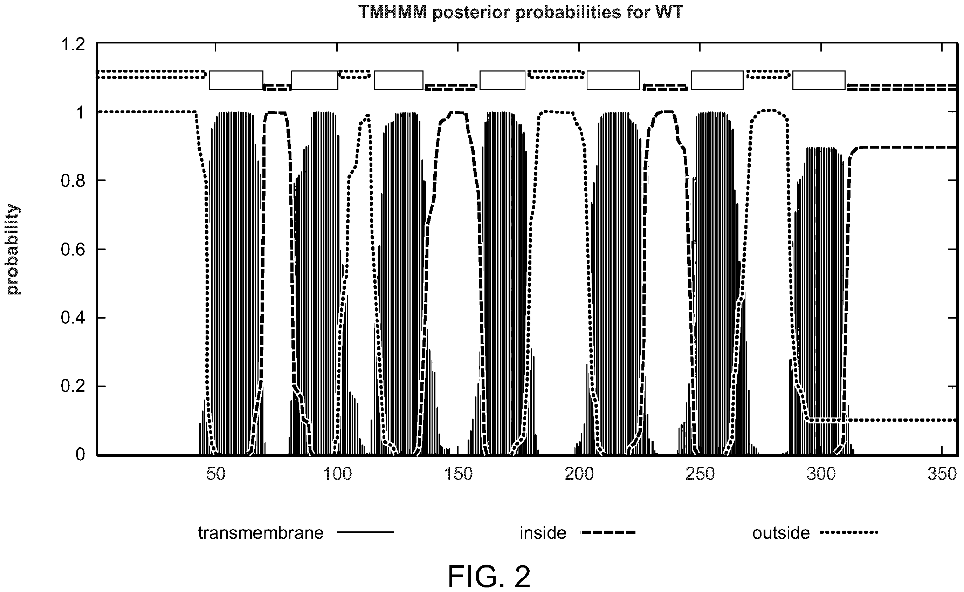

FIG. 2 is a TMHMM prediction for the transmembrane domain regions for CXCR4. The prediction shows 7 distinctive hydrophobic transmembrane segments. In contrast, in a TMHMM prediction for a variant of CXCR4 subject to the QTY substitution method of the invention (CXCR4-QTY), there are no distinctive 7 hydrophobic transmembrane segments visible anymore.
FIG. 3 illustrates the predicted alpha helical wheel structure of the fully QTY Code modified TM1 domain of CXCR4.
FIG. 4 is an illustration of the potential variants in each of the seven TM regions of a GPCR CXCR4.
FIGs. 5, 6, 7, and 8 are sequence alignments of the wild type proteins and QTY variants of CXCR4, CXCR3, CCR3 and CCR5, respectively. QTY Code is only applied to the 7 hydrophobic transmembrane segments, but not the extracellular and intracellular segments.
FIG. 9A is a flowchart of a representative embodiment of the process.
FIG. 9B is another flowchart of a representative embodiment of the process.
FIG. 10 is an illustration of the computer systems of the invention.
FIGs. 11A and 11B are schematic illustration of flowcharts setting forth processing steps of certain preferred embodiments of the invention.

### DETAILED DESCRIPTION OF THE INVENTION

A description of preferred embodiments of the invention follows. The words "a" or "an" are meant to encompass one or more, unless otherwise specified.

In some aspects, the invention is directed to the use of the QTY (Glutamine, threonine and tyrosine) replacement (or "QTY Code") method (or "principle") to change the 7-transmembrane α-helix hydrophobic residues leucine (L), isoleucine (I), valine (V), and phenylalanine (F) of a native protein to the hydrophilic residues glutamine (Q), threonine (T) and tyrosine (Y). In certain embodiments, as described above, Asn (N) and Ser (S) can also be used as substitute residues for L, I and/or V, but not F. This invention can convert a water insoluble, native membrane protein to a more water-soluble counterpart that still maintains some or substantially all functions of the native protein.

The invention includes a process for designing water-soluble peptides. The process is described in terms of GPCR proteins as an illustrative example, with specificity in the first instance to human CCR3, CCR5, CXCR4, and CX3CR1. However, the general principle of the invention also applies to other proteins with transmembrane (α-helical) regions.

GPCRs typically have 7-transmembrane alpha-helices (7TM) and 8 loops (8NTM) connected by the seven TM regions. These transmembrane segments may be referred to as TM1, TM2, TM3, TM4, TM5, TM6 and TM7. The 8 non-transmembrane loops are divided into 4 extracellular loops EL1, EL2, EL3, and EL4, and 4 intracellular loops, IL1, IL2, IL3, and IL4, thus a total of 8 loops (including the N- and C-terminal loops that are each only connected to one TM region, and each has a free end). Thus a 7TM GPCR protein can be divided into 15 fragments based on the transmembrane and non-transmembrane features.

One aspect of the invention provides a process of operating a computer program to execute a scripted procedure to select, or make a water-soluble variant of a membrane protein (e.g., a G Protein-Coupled Receptor (GPCR)), the method comprising:
(1) entering a sequence of the membrane protein (e.g., GPCR) for analysis;
(2) obtaining a variant of the membrane protein (e.g., GPCR), wherein a plurality of hydrophobic amino acids in the transmembrane (TM) domain alpha-helical segments ("TM regions") of the membrane protein (e.g., GPCR) are substituted, wherein:
   (a) said hydrophobic amino acids are selected from the group consisting of Leucine (L), Isoleucine (I), Valine (V), and Phenylalanine (F);
   (b) each said Leucine (L) is independently substituted by Glutamine (Q), Asparagine (N), or Serine (S);
   (c) each said Isoleucine (I) and said Valine (V) are independently substituted by Threonine (T), Asparagine (N), or Serine (S); and,
   (d) each said Phenylalanine is substituted by Tyrosine (Y); and, subsequently,
(3) obtaining an α-helical secondary structure result for the variant to verify maintenance of α-helical secondary structures in the variant;
(4) obtaining a trans-membrane region result for the variant to verify water solubility of the variant,
thereby selecting the water-soluble variant of the membrane protein (e.g., GPCR).

As used herein, "water-soluble variant of the (trans)membrane protein" or "water-soluble (trans)membrane variant" may be used interchangeably.

The exact sequence of carrying out the steps of the invention may be variable. For example, in certain embodiments, step (3) is performed prior to step (4). In certain embodiments, step (3) is performed concurrently with step (4). In certain embodiments, step (3) is performed after step (4).

In certain embodiments, the plurality of hydrophobic amino acids are randomly selected from all potential hydrophobic amino acids L, I, V, and F located on all TM regions of the protein. In certain embodiments, the plurality of hydrophobic amino acids is about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 50%, 55%, 60%, 65%, 70%, 75%, 80%, 85%, 90%, 91%, 92%, 93%, 94%, 95%, 96%, 97%, 98%, 99%, or 100% of all the potential hydrophobic amino acids L, I, V, and F located on all TM regions of the protein. In certain embodiments, the plurality of hydrophobic amino acids is no less than about 10%, 15%, 20%, 25%, 30%, 35%, 40%, 45%, 50% of all the potential hydrophobic amino acids L, I, V, and F located on all TM regions of the protein. In certain embodiments, the plurality of hydrophobic amino acids is no more than about 95%, 90%, 85%, 80%, 75%, 70%, 65%, 60%, or 50% of all the potential hydrophobic amino acids L, I, V, and F located on all TM regions of the protein. In certain embodiments, the randomly selected hydrophobic amino acids L, I, V, and F may be roughly evenly distributed on all TM regions, or may be preferentially or exclusively distributed on 1, 2, 3, 4, 5, or 6 TM regions.

In certain embodiments, every potential hydrophobic amino acids L, I, V, and F on all TM regions of the protein are substituted. For example, all L are independently substituted by Q (or S or N); and/or all I and V are independently substituted by T (or S or N); and/or all F are substituted by Y. In certain embodiments, all L are substituted by Q, all I and V are substituted by T, and all F are substituted by Y.

In certain embodiments, instead of randomly substituting selected hydrophobic amino acids L, I, V, and F in all TM regions, all substitutions can first be limited to any one of the TM regions (such as the most N-terminal or C-terminal TM region), and only desired substitution variants are selected as members of a library of potential variants. All members of the library differ in the substitutions in the chosen TM region, either due to the positions substituted (e.g., the 3^{rd} vs. the 10^{th} residue in the TM region is substituted), or due to the identity of the substituent residues (e.g., S vs. T for an I or V substitution), or both. The desired substitution variants are selected based on a pre-determined criteria, such as a scoring system that takes into consideration the α-helical secondary structure prediction result and/or the trans-membrane region prediction result.

This process can be repeated for 1, 2, 3, 4, 5, 6 additional TM regions of the protein, or all the remaining TM regions of the protein, each iteration creates a library of potential variants that can be stored in an electronic memory or database. Within the same library, all variants differ in the substitutions in the chosen TM region (see above), but are otherwise the same in the remaining TM regions and non-TM regions.

Domain swapping or shuffling using sequences from two or more such libraries creates combinatory variants having hydrophobic amino acids L, I, V, F substitutions in two or more TM regions. Depending on the number of members in each library, the total possible combinations of combinatory variants can approach millions with just a few members in each library. For example, for a GPCR having 7 TM regions, if there are 8 members in each of the seven libraries, the total number of combinatory variants based on the libraries will be 8⁷ or about 2.1 million. In certain embodiments, the library of combinatory variants comprises less than about 5, 4, 3, 2, 1, or 0.5 million members.

Thus in certain embodiments, in step (2), one subset of said plurality of hydrophobic amino acids in one and the same TM region of the protein (e.g., GPCR) are substituted to generate one member of a library of potential variants, and one or more different subsets of said plurality of hydrophobic amino acids are substituted to generate additional members of the library.

In certain embodiments, the method further comprises ranking all members of said library based on a combined score, wherein the combined score is a weighed combination of the α-helical secondary structure prediction result and the trans-membrane region prediction result.

As one of ordinary skill in the art would appreciate, the domains having different sequences will likely predict different water solubilities and propensities for alpha helical formation. One can assign "a score" to a specific predicted water solubility or range of solubilities, propensity to form alpha helical structure or range of propensities. The score can be quantitative (0,1) where 0 can represent, for example, a domain with an unacceptable predicted water solubility and 1 can represent, for example, a domain with an acceptable predicted water solubility. This score can be based on a threshold value, for example. Or, the score can be assessed on a scale, for example, between 1 and 10 establishing characterizing increasing degrees of water solubility. Or, the score can be quantitative, such as in describing the predicted solubility in terms of mg/mL. Upon assessing a score to each domain, the domain variants can be readily compared (or ranked) by one or, preferably, both of the scores to select domain variants that are both water soluble and form alpha helices. Thus, preferred embodiments can utilize a ranking function that can be used to compute the ranking data. Note also that water soluble proteins made based on the currently described system can be analyzed and characterized to provide input to the system such that those combinations of substitution that are not effective to achieve a given biological function can be used to constrain the computational model, thereby enabling a more efficient processing of the information.

For example, using the methods of the invention, one or more variants can be designed and produced in vitro and/or in vivo, and one or more biological functions of the variants can be determined based on any of many art-recognized methods. For GPCR, for example, ligand binding and/or downstream signal transduction by the variants can be compared to that of the wild-type GPCR, and the patterns of QTY substitution used to generate a specific variant can be associated with an enhanced, maintained, or diminished biological activity. Such structural-functional relationship information obtained based on one or more variants can be used for machine learning or impart additional constrain on the computational model of the invention, to more efficiently rank the variants created by the methods of the invention. Thus new potential variants having substitution patterns more closely matching that of a known successful variant can be ranked higher that another potential variant having substitution patterns less closely matching that of the known successful variant, or more closely matching that of a known unsuccessful variant.

The TMHMM program, when run as a standalone version of the software module / package (e.g., one for the Linux system), produces a score of between 0 and 1 that can be used to predict the propensity of forming transmembrane regions / proteins. The score can be used as a quantitative prediction for water solubility in the methods of the invention.

Thus in certain embodiment, the α-helical secondary structure component of a ranking function can be a quantitative score, such as 0.5 or 1 for having no predicted α-helical secondary structures, and 0 for having maintained predicted α-helical secondary structures. In certain embodiments, the trans-membrane region result can be provided by a TM region prediction program, such as TMHMM 2.0, which provides a numeric value between 0 and 1, with 0 being no predicted TM region, and 1 being the strongest propensity of forming TM region(s). Thus the two scores can be combined, either directly or with weighing, such that the combined score represents an overall assessment of maintained secondary structure as well as predicted water solubility (as measured by propensity to form TM regions). For example, a combined score of 0 indicates that the variant has no predicted TM region, while having maintained predicted α-helical secondary structures, and is thus a desired variant. Meanwhile, a variant has strong propensity to form TM region (due to the presence of large number of hydrophobic residues, for example), tends to have a larger combined score and thus undesirable under this scoring scheme.

In certain embodiments, the method includes eliminating variants having an α-helical secondary structure prediction result tending to show that the α-helical secondary structures are destroyed or disrupted. In certain embodiments, the method includes eliminating variants having trans-membrane region prediction result tending to show strong propensity to form TM regions. Thus the system can include a beaming module in which variants can be excluded from further selection processing.

In certain embodiments, the ranking function can be selected to include a weighing scheme that assigns 5%, 10%, 20%, 25%, 30%, 40%, 50%, 60%, 70%, 80%, 90% or 95% weight to the α-helical secondary structure prediction result, and the remaining to the trans-membrane region prediction result. The user can either manually select the weighting features, or the software can automatically select the weighting features depending on the desired characteristics such as biological function.

In certain embodiments, the method further comprises selecting N members with the highest combined scores to form a first library of potential variants for said TM region, wherein N is a pre-determined integer (e.g., 3, 4, 5, 6, 7, 8, 9, 10, 11, 12, 13, 14, 15, 16, 17, 18, 19, 20 or more).

In certain embodiments, the method further comprises generating one library of potential variants for 1, 2, 3, 4, 5, 6, or all the remaining TM regions of the protein (e.g., GPCR). Each entry in the library can include fields used to define attributes of that entry, including the ranking data generated by one or more ranking functions.

In certain embodiments, the method further comprises replacing two or more (e.g., all) TM regions of the protein (e.g., GPCR) with corresponding TM regions from the libraries of potential variants, to create a library of combinatory variants. As used herein, "corresponding TM regions" refer to the TM regions in the libraries of potential variants that are the same or homologous to the TM regions of the protein (e.g., GPCR) that are being combined. For example, if the 2^{nd} and 3^{rd} TM regions from the N-terminal of a GPCR are to be substituted, TM region sequences from the library having substitutions only in the 2^{nd} TM regions, and TM region sequences from the library having substitutions only in the 3^{rd} TM regions, are imported / pasted / transferred into the 2^{nd} and 3^{rd} TM regions of the GPCR to create combinatory variants.

In certain embodiments, substantially all (*e.g.*, 96%, 97%, 98%, 99%, or 100%), or 30%, 40%, 50%, 60%, 70%, 75%, 80%, 85%, 90%, 95% of said leucines are substituted by glutamines. In certain embodiments, substantially all (*e.g.*, 96%, 97%, 98%, 99%, or 100%), or 30%, 40%, 50%, 60%, 70%, 75%, 80%, 85%, 90%, 95% of said isoleucines are substituted by threonines. In certain embodiments, substantially all (*e.g.*, 96%, 97%, 98%, 99%, or 100%), or 30%, 40%, 50%, 60%, 70%, 75%, 80%, 85%, 90%, 95% of said valines are substituted by threonines. In certain embodiments, wherein substantially all (*e.g.*, 96%, 97%, 98%, 99%, or 100%), or 30%, 40%, 50%, 60%, 70%, 75%, 80%, 85%, 90%, 95% of said phenylalanines are substituted by tyrosines. In certain embodiments, one or more (*e.g,*, 1, 2, or 3) said leucines are not substituted. In certain embodiments, one or more (*e.g,*, 1, 2, or 3) said isoleucines are not substituted. In certain embodiments, one or more (*e.g,*, 1, 2, or 3) said valines are not substituted. In certain embodiments, one or more (*e.g,*, 1, 2, or 3) said phenylalanines are not substituted.

In certain embodiments, the method further comprises producing / expressing said combinatory variants. In certain embodiments, the method further comprises testing said combinatory variants for ligand binding (*e.g.*, in vitro, or in a biological system such as yeast two-hybrid system), wherein those having substantially the same ligand binding compared to that of the GPCR are selected. In certain embodiments, the method further comprises testing said combinatory variants for a biological function of the GPCR, wherein those having substantially the same biological function compared to that of the GPCR are selected.

In certain embodiments, the sequence of the TM protein (e.g., GPCR) contains information about the TM regions of the protein, e.g., the location of one or more transmembrane regions of the TM protein, such as the location of all TM regions. Such sequences may belong to proteins having resolved crystal structure with defined TM regions. Such sequences may also belong to proteins having annotated TM region information based on prior research, and such information is readily available from a public or proprietary database, such as PDB, UniProt, GenBank, EMBL, DBJ, etc.

The Protein Data Bank (PDB) is a weekly updated repository for the three-dimensional structural data of large biological molecules, such as proteins and nucleic acids. The data, typically obtained by X-ray crystallography or NMR spectroscopy and submitted by biologists and biochemists from around the world, are freely accessible on the Internet via the websites of its member organizations (PDBe, PDBj, and RCSB). The PDB is overseen by the Worldwide Protein Data Bank, wwPDB. The PDB is a key resource in areas of structural biology, such as structural genomics, and most major scientific journals, and some funding agencies, now require scientists to submit their structure data to the PDB.

If the contents of the PDB are thought of as primary data, then there are hundreds of derived (i.e., secondary) databases that categorize the data differently. For example, both SCOP and CATH categorize structures according to type of structure and assumed evolutionary relations; GO categorize structures based on genes; while crystallographic database store information about the 3D structure of the proteins. All such publically available database may be used to provide input sequence information, including information about the existence and position of transmembrane regions.

Another publically available database that can provide sequence information for use in the methods of the invention is UniProt. UniProt is a comprehensive, high-quality and freely accessible database of protein sequence and functional information, many entries being derived from genome sequencing projects. It contains a large amount of information about the biological function of proteins derived from the research literature. UniProt provides four core databases: UniProtKB (with sub-parts Swiss-Prot and TrEMBL), UniParc, UniRef, and UniMes. Among them, UniProtKB/Swiss-Prot is a manually annotated, non-redundant protein sequence database that combines information extracted from scientific literature and biocurator-evaluated computational analysis. The aim of UniProtKB/Swiss-Prot is to provide all known relevant information about a particular protein. Annotation is regularly reviewed to keep up with current scientific findings. The manual annotation of an entry involves detailed analysis of the protein sequence and of the scientific literature. Sequences from the same gene and the same species are merged into the same database entry. Differences between sequences are identified, and their cause documented (e.g., alternative splicing, natural variation, etc.). Computer-predictions are manually evaluated, and relevant results selected for inclusion in the entry. These predictions include post-translational modifications, transmembrane domains and topology, signal peptides, domain identification, and protein family classification, all may be used to provide useful sequence information pertaining to the TM regions used in the methods of the invention.

In certain embodiments, the sequence of the TM protein (e.g., GPCR) does not contain information about the location of one or more (e.g., any) transmembrane regions. However, the TM region(s) can be predicted based on sequence homology with a related protein having known TM regions. For example, the related protein may be a homologous protein in a different species.

In certain embodiments, the sequence of the TM protein (e.g., GPCR) does not contain information about the location of one or more (e.g., any) transmembrane regions, and such information is not readily available based on known information. In this embodiment, the invention provides computation of TM regions using art-recognized methods, such as the TMHMM 2.0 (TransMembrane prediction using Hidden Markov Models) program, developed by Center for Biological Sequence Analysis. See further details regarding this below.

In certain embodiments, the method further comprises providing a polynucleotide sequence for each variants of the protein (e.g., GPCR). Such polynucleotide sequence can be readily generated based on the protein sequence of the protein (e.g., GPCR), and the known genetic code. In certain embodiments, the polynucleotide sequence is codon optimized for expression in a host. The host may be a bacterium such as *E. coli,* a yeast such as *S*. *cerevisae* or *S*. *pombe,* an insect cell such as Sf9 cell, a non-human mammalian cell, or a human cell.

In certain embodiments, the protein is a GPCR, such as one selected from the group consisting of: purinergic receptors (P2Y₁, P2Y₂, P2Y₄, P2Y₆), M₁ and M₃ muscarinic acetylcholine receptors, receptors for thrombin (protease-activated receptor (PAR)-1, PAR-2), thromboxane (TXA₂), sphingosine 1-phosphate (S1P₂, S1P₃, S1P₄ and S1P₅), lysophosphatidic acid (LPA₁, LPA₂, LPA₃), angiotensin II (AT₁), serotonin (5-HT_{2c} and 5-HT₄), somatostatin (sst₅), endothelin (ET_{A} and ET_{B}), cholecystokinin (CCK₁), V₁ₐ vasopressin receptors, D₅ dopamine receptors, fMLP formyl peptide receptors, GAL₂ galanin receptors, EP₃ prostanoid receptors, A₁ adenosine receptors, α₁ adrenergic receptors, BB₂ bombesin receptors, B₂ bradykinin receptors, calcium-sensing receptors, chemokine receptors, KSHV-ORF74 chemokine receptors, NK₁ tachykinin receptors, thyroid-stimulating hormone (TSH) receptors, protease-activated receptors, neuropeptide receptors, adenosine A2B receptors, P2Y purinoceptors, metabolic glutamate receptors, GRK5, GPCR-30, and CXCR4.

In certain embodiments, the scripted procedure of the method comprises VBA scripts.

In certain embodiments, the scripted procedure is operable in a Linux system (e.g., Ubuntu 12.04 LTS), a Microsoft Windows operative system, or an Apple iOS operative system.

In certain embodiments, the process comprises all, or substantially all, of the following steps:
(1) identifying a first transmembrane region of a (trans)membrane protein, if necessary, by predicting an alpha-helical structure of the protein (e.g., a GPCR);
(2) modifying a plurality of hydrophobic amino acids via the QTY Code, as defined herein to obtain a modified first transmembrane sequence;
(3) scoring the propensity of the alpha-helical structure of the first modified transmembrane sequence of (2) (e.g., in the context of a modified (trans)membrane protein having the first modified transmembrane sequence) to arrive at a structure score;
(4) scoring the water solubility prediction of the first modified transmembrane sequence of (2) (e.g., in the context of a modified (trans)membrane protein having the first modified transmembrane sequence) to arrive at a solubility score;
(5) repeating steps (2) through (4) to arrive at a first library of putative water soluble first modified transmembrane variants;
(6) comparing the structure scores and solubility scores of each putative water soluble first modified transmembrane variants in the first library and, preferably ranking the putative water soluble first modified transmembrane variants using said structure scores and solubility scores;
(7) selecting a plurality of putative water soluble first modified transmembrane variants (wherein the plurality is the integer, H, or preferably less than 10, 9, 8, 7, 6, 5 or 4) to arrive at a second library of putative water soluble first modified transmembrane variants;
(8) repeating steps (1) through (7) for a second, third, fourth, fifth, sixth, seventh or, preferably, all transmembrane regions of the protein (the sum of the transmembrane regions modified by the method being an integer n);
(9) identifying the amino acid sequences of the protein which are not included in any transmembrane region modified in steps (1) through (8), and including any extracellular or intracellular domain of the protein;
(10) generating combinatorial variants of putative water soluble modified transmembrane protein (see above); and,
(11) optionally, identifying a nucleic acid sequence for each putative water soluble modified transmembrane variant.

Using the nucleic acid sequences identified in the above process, nucleic acid sequences for each putative water-soluble modified transmembrane variant and each non-transmembrane domains (including the extracellular and intracellular domains) can be generated and combinatorially expressed to create a library of up to Hⁿ putative water-soluble transmembrane protein variants. For example, where H is 8 and n is 7, a library of approximately 2 million water-soluble protein variants can be designed.

Another aspect of the invention pertains to the expression of the water-soluble variant proteins (e.g., GPCR) designed based on the methods of the invention. This aspect of the invention is partly based on the surprising finding that the water-soluble variant proteins (e.g., GPCR) designed based on the methods of the invention can achieve high levels of expression in both in vitro cell-free expression system and expression in commonly used cell-based expression systems, such as *E. coli.* In addition, the expressed proteins are highly soluble, and can be easily purified from the soluble fraction of the expression system, such as the soluble fraction from the lysate of an *E. coli* culture, as opposed to the insoluble aggregates or pellets in which most membrane proteins are typically found.

Thus one aspect of the invention provides a method of producing a protein in a bacterium (e.g., an *E. coli*), comprising:
(a) culturing the bacterium in a growth medium under a condition suitable for protein production;
(b) fractioning a lysate of the bacterium to produce a soluble fraction and the insoluble pellet fraction; and,
(c) isolating the protein from the soluble fraction;
wherein:
(1) the protein is a subject variant protein (e.g., G-protein couple receptor (GPCR)) of the invention; and,
(2) the yield of the protein is at least 20 mg/L (e.g., 30 mg/L, 40 mg/L, 50 mg/L or more) of growth medium.

In certain embodiments, the bacterium is *E. coli* BL21, and the growth medium is LB medium. In certain embodiments, the protein is encoded by a plasmid in the bacterium. In certain embodiments, expression of the protein is under the control of an inducible promoter. For example, the inducible promoter may be inducible by IPTG. In certain embodiments, the lysate is produced by sonication. In certain embodiments, the soluble fraction is produced by centrifuging the lysate at 14,500 × g or more.

With the general aspects of the inventions described above, certain features or specific embodiments of the invention are further described below.

### Transmembrane Region Prediction

Certain methods of the invention comprise a step of predicting a transmembrane region of a protein, such as GPCR. There are many programs and software known in the art relating to the TM region, and any of which may be used individually or in combination in the methods of the invention where a TM region prediction step is called for. These programs usually have a very simple user interface, typically requiring the user to provide an input sequence of a specified format (such as FASTA or plain text), and provides prediction results using text or graphics or both. Some programs also offer more advanced features, such as allowing the user to specify certain parameters to fine tune the prediction results. All such programs can be used in the methods of the invention.

One exemplary TM region prediction program is TMHMM (hosted by Center for Biological Sequence Analysis, Technical University of Denmark), which method predicts 97-98% TM region helices correctly. It predicts transmembrane helices in proteins using the Hidden Markov Model. The input protein sequence can be the FASTA format, and the output can be presented as an html page with an image of predicted locations for the TM regions. In a study by Moller et al., entitled "Evaluation of Methods for the Prediction of Membrane Spanning Regions," Bioinformatics 17(7):646-653, 2001, TMHMM was determined to be the best performing transmembrane prediction program at the time of evaluation.

The programs compared in that study include the following, all can be used to predict TM region in the methods of the invention: TMHMM 1.0, 2.0, and a retrained version of 2.0 (Sonnhammer et al., Int. Conf. Intell. Syst. Mol. Biol. AAAI Press, Montreal, Canada, pp.176-182, 1998; Krogh et al., J Mol Biol. 305(3):567-80, 2001); MEMSAT 1.5 (Jones et al., Biochemistry 33:3038-3049, 1994); Eisenberg (Eisenberg et al., Nature 299:371-374, 1982); Kyte/Doolittle (Kyte and Doolittle, J. Mol. Biol. 157:105-132, 1982); TMAP (Persson and Argos, J. Protein Chem. 16:453-457, 1997); DAS (Cserzo et al., Protein Eng. 10:673-676, 1997); HMMTOP (Tusnady and Simon, J. Mol. Biol. 283:489-506, 1998); SOSUI (Hirokawa et al., Bioinformatics 14:378-379, 1998); PHD (Rost et al., Int. Conf. Intell. Syst. Mol. Biol. AAAI Press, St. Louis, USA, pp.192-200, 1996); TMpred (Hofmann and Stoffel, Biol. Chem. Hoppe-Seyler 374:166, 1993); KKD (Klein et al., Biochim. Biophys. Acta. 815:468-476, 1985); ALOM2 (Nakai and Kanehisa, Genomics 14:489-911, 1992); and Toppred 2 (Claros and Heijne, Comput. Appl. Biosci. 10:685-686, 1994). All references cited are incorporated herein by reference.

The principals of TMHMM is described in Krogh et al., Predicting transmembrane protein topology with a hidden Markov model: Application to complete genomes. Journal of Molecular Biology, 305(3):567-580, January 2001 (incorporated by reference); and Sonnhammer et al., A hidden Markov model for predicting transmembrane helices in protein sequences. In J. Glasgow, T. Littlejohn, F. Major, R. Lathrop, D. Sankoff, and C. Sensen, editors, Proceedings of the Sixth International Conference on Intelligent Systems for Molecular Biology, pages 175-182, Menlo Park, CA, 1998, AAAI Press (incorporated by reference).

DAS (Dense Alignment Surface, Cserzo et al., "Prediction of transmembrane alpha-helices in procariotic membrane proteins: the Dense Alignment Surface method," Prot. Eng. 10(6): 673-676, 1997, Stockholm University, Sweden) predicts transmembrane regions using the Dense Alignment Surface method. DAS is based on low-stringency dot-plots of the query sequence against a set of library sequences - non-homologous membrane proteins - using a previously derived, special scoring matrix. The method provides a high precision hyrdophobicity profile for the query from which the location of the potential transmembrane segments can be obtained. The novelty of the DAS-TMfilter algorithm is a second prediction cycle to predict TM segments in the sequences of the TM-library. To use the DAS server, user enters a protein sequence at www dot sbc dot su dot se slash ∼miklos slash DAS, and the DA server will predict a TM region of the input sequence.

HMMTOP (Hungarian Academy of Sciences, Budapest) is an automatic server for predicting transmembrane helices and topology of proteins using Hidden Markov Model, developed by G.E. Tusnády, at the Institute of Enzymology. The method used by this prediction server is described in G.E Tusnády and I. Simon (1998) "Principles Governing Amino Acid Composition of Integral Membrane Proteins: Applications to Topology Prediction." J. Mol. Biol. 283: 489-506 (incorporated by reference). The new features of HMMTOP 2.0 version is described in 'G.E Tusnády and I. Simon (2001) "The HMMTOP transmembrane topology prediction server," Bioinformatics 17: 849-850 (incorporated by reference).

MEMSAT2 Transmembrane Prediction Page (www dot sacs dot ucsf dot edu slash cgi-bin slash memsat dot py) predicts transmembrane segments in a protein using FASTA format or plain text as input. A related program, the MEMSAT (1.5) software, is copyrighted by Dr. David Jones (Jones et al., Biochemistry 33:3038-3049, 1994). The latest version of MEMSTAT, MEMSAT V3, is a widely used all-helical membrane protein prediction method MEMSAT. The method was benchmarked on a test set of transmembrane proteins of known topology. From sequence data MEMSAT was estimated to have an accuracy of over 78% at predicting the structure of all-helical transmembrane proteins and the location of their constituent helical elements within a membrane. MEMSATSVM is highly accurate predictor of transmembrane helix topology. It is capable of discriminating signal peptides and identifying the cytosolic and extra-cellular loops. MEMSAT3 and MEMSATSVM are both parts of the PSIPRED Protein Sequence Analysis Workbench, which aggregates several structure prediction methods into one location at the University College London.

The Phobius server (phobius dot sbc dot su dot se) is for prediction of transmembrane topology and signal peptides from the amino acid sequence of a protein in FASTA format. Phobius is described in Lukas et al., "A Combined Transmembrane Topology and Signal Peptide Prediction Method," Journal of Molecular Biology 338(5): 1027-1036, 2004). PoyPhobius is described in: Lukas et al., "An HMM posterior decoder for sequence feature prediction that includes homology information," Bioinformatics, 21 (Suppl 1):i251-i257, 2005. And the Phobius webserver is described in: Lukas et al., "Advantages of combined transmembrane topology and signal peptide prediction--the Phobius web server," Nucleic Acids Res. 35:W429-32, 2007 (all cited art incorporated by reference).

SOSUI is for the discrimination of membrane proteins and soluble ones together with the prediction of transmembrane helices. SOSUI predicts transmembrane regions using Hydrophobicity Analysis for Topology and Probe Helix Method for Tertial Structure. The accuracy of the classification of proteins is said to be as high as 99%, and the corresponding value for the transmembrane helix prediction is said to be about 97%. The system SOSUI is available through internet access www dot tuat dot ac dot jp slash mitaku slash sosui.

TMPred (European Molecular Biology Network, Swiss node) predicts transmembrane regions and protein orientation in a query sequence. Specifically, the TMPred algorithm is based on the statistical analysis of TMbase, a database of naturally occurring transmembrane proteins. The prediction is made using a combination of several weight-matrices for scoring. See Hofmann & Stoffel (1993) "TMbase - A database of membrane spanning proteins segments," Biol. Chem. Hoppe-Seyler, 374:166.

The SPLIT 4.0 server is a membrane protein secondary structure prediction server (split dot pmfst dot hr slash split slash 4) that predicts the transmembrane (TM) secondary structures of membrane proteins in SWISS-PROT format, using the method of preference functions. See Juretic et al., "Basic charge clusters and predictions of membrane protein topology," J. Chem. Inf. Comput. Sci., 42:620-632, 2002 (incorporated by reference).

PRED-TMR predicts transmembrane domains in proteins using solely the protein sequence itself. The algorithm refines a standard hydrophobicity analysis with a detection of potential termini ("edges," starts and ends) of transmembrane regions. This allows both to discard highly hydrophobic regions not delimited by clear start and end configurations and to confirm putative transmembrane segments not distinguishable by their hydrophobic composition. The accuracy obtained on a test set of 101 non-homologous transmembrane proteins with reliable topologies compares well with that of other popular existing methods. Only a slight decrease in prediction accuracy was observed when the algorithm was applied to all transmembrane proteins of the SwissProt database (release 35). See Pasquier et al., "A novel method for predicting transmembrane segments in proteins based on a statistical analysis of the SwissProt database: the PRED-TMR algorithm," Protein Eng., 12(5):381-385, 1999 (incorporated by reference).

In the related PRED-TMR2, the application has been extended with a preprocessing stage represented by an artificial neural network which is able to discriminate with a high accuracy transmembrane proteins from soluble or fibrous ones. Applied on several test sets of transmembrane proteins, the system gives a perfect prediction rating of 100% by classifying all the sequences in the transmembrane class. Applied on 995 non-transmembrane protein extracted from the PDBSELECT database, the neural network predicts falsely 23 of them to be transmembrane (97.7% of correct assignment). See Pasquier and Hamodrakas, "An hierarchical artificial neural network system for the classification of transmembrane proteins," Protein Eng., 12(8):631-634, 1999 (incorporated by reference).

### Protein Alpha Helical Secondary Structure Prediction

Certain methods of the invention comprise a step of predicting alpha helical secondary structure of a protein, such as GPCR. There are many such programs and software known in the art, and any of which may be used individually or in combination in the methods of the invention where alpha helical secondary structure prediction step is called for. All such programs can be used in the methods of the invention.

Early methods of secondary-structure prediction were restricted to predicting the three predominate states: helix, sheet, or random coil. These methods were based on the helix- or sheet-forming propensities of individual amino acids, sometimes coupled with rules for estimating the free energy of forming secondary structure elements. Such methods were typically ∼60% accurate in predicting which of the three states (helix/sheet/coil) a residue adopts. The first widely used technique to predict protein secondary structure from the amino acid sequence was the Chou-Fasman method.

A significant increase in accuracy (to nearly ∼80%) was made by taking advantage of information provided by multiple sequence alignment; knowing the full distribution of amino acids that occur at a position (and in its vicinity, typically ∼7 residues on either side) throughout evolution provides a much better picture of the structural tendencies near that position. For example, a given protein might have a glycine at a given position, which by itself might suggest a random coil. However, multiple sequence alignment might reveal that helix-favoring amino acids occur at that position (and nearby positions) in 95% of homologous proteins throughout evolution. Moreover, by examining the average hydrophobicity at that and nearby positions, the same alignment might also suggest a pattern of residue solvent accessibility consistent with an α-helix. Taken together, these factors would suggest that the glycine of the original protein adopts α-helical structure, rather than random coil. Thus in the methods of the invention, the alpha helical secondary structure prediction program may combine all the available data to form a 3-state prediction, including neural networks, hidden Markov models and support vector machines. Such prediction methods also provide a confidence score for their predictions at every position.

Secondary-structure prediction methods are continuously benchmarked, e.g., EVA (benchmark). EVA is a continuously running benchmark project for assessing the quality of protein structure prediction and secondary structure prediction methods. Methods for predicting both secondary structure and tertiary structure - including homology modeling, protein threading, and contact order prediction - are compared to results from each week's newly solved protein structures deposited in the Protein Data Bank (PDB). The project aims to determine the prediction accuracy that would be expected for non-expert users of common, publicly available prediction webservers.

Based on these tests, the most accurate methods at present are Psipred, SAM (Karplus, "SAM-T08, HMM-based protein structure prediction," Nucleic Acids Res. (2009) 37 (Web Server issue): W492-497. doi:10.1093/nar/gkp403); PORTER (Pollastri & McLysaght, "Porter: a new, accurate server for protein secondary structure prediction," Bioinformatics 21 (8):1719-1720, 2005); PROF (Yachdav et al. (2014). "PredictProtein-an open resource for online prediction of protein structural and functional features," Nucleic Acids Res. 42 (Web Server issue): W337-343. doi:10.1093/nar/gku366); and SABLE (Adamczak et al. (2005) "Combining prediction of secondary structure and solvent accessibility in proteins," Proteins 59 (3): 467-475. doi:10.1002/prot.20441). In addition, the standard method for assigning secondary-structure classes (helix/strand/coil) to PDB structures is DSSP (Kabsch W and Sander (1983) "Dictionary of protein secondary structure: pattern recognition of hydrogen-bonded and geometrical features," Biopolymers 22 (12): 2577-2637. doi:10.1002/bip.360221211), against which the predictions are benchmarked. All incorporated by reference and all can be used in the methods of the invention.

The DSSP algorithm is the standard method for assigning secondary structure to the amino acids of a protein, given the atomic-resolution coordinates of the protein. DSSP begins by identifying the intra-backbone hydrogen bonds of the protein using a purely electrostatic definition, assuming partial charges of -0.42 *e* and +0.20 *e* to the carbonyl oxygen and amide hydrogen respectively, their opposites assigned to the carbonyl carbon and amide nitrogen. A hydrogen bond is identified if *E* in the following equation is less than -0.5 kcal/mol:
Based on this, eight types of secondary structure are assigned. The 3₁₀ helix, α helix and π helix have symbols G, H and I and are recognized by having a repetitive sequence of hydrogen bonds in which the residues are three, four, or five residues apart respectively. Two types of beta sheet structures exist; a beta bridge has symbol B while longer sets of hydrogen bonds and beta bulges have symbol E. T is used for turns, featuring hydrogen bonds typical of helices, S is used for regions of high curvature (where the angle between and is less than 70°), and a blank (or space) is used if no other rule applies, referring to loops. These eight types are usually grouped into three larger classes: helix (G, H and I), strand (E and B) and loop (all others).

PSIPRED (Psi-blast based secondary structure prediction) is a technique used to investigate protein structure. It employs neural network, machine learning methods in its algorithm. It is a server-side program, featuring a website serving as a front-end interface, which can predict a protein's secondary structure (beta sheets, alpha helices and coils) from the primary sequence. See bioinf dot cs dot ucl dot ac dot uk slash psipred. The idea of this method is a machine learning method that uses the information of the evolutionarily related proteins to predict the secondary structure of a new amino acid sequence. Specifically, PSIBLAST is used to find related sequences and to build a position-specific scoring matrix. This matrix is processed by a neural network, which was constructed and trained to predict the secondary structure of the input sequence. The prediction method or algorithm is split into three stages: *Generation of a sequence profile, Prediction of initial secondary structure,* and *Filtering of the predicted structure.* PSIPRED works to normalize the sequence profile generated by PSIBLAST. Then, by using neural networking, initial secondary structure is predicted. For each amino acid in the sequence the neural network is fed with a window of 15 acids. There is additional information attached, indicating if the window spans the N or C terminus of the chain. This results in a final input layer of 315 input units, divided into 15 groups of 21 units. The network has a single hidden layer of 75 units and 3 output nodes (one for each secondary structure element: helix, sheet, coil). A second neural network is used for filtering the predicted structure of the first network. This network is also fed with a window of 15 positions. The indicator on the possible position of the window at a chain terminus is also forwarded. This results in 60 input units, divided into 15 groups of four. The network has a single hidden layer of 60 units and results in three output nodes (one for each secondary structure element: helix, sheet, coil). The three final output nodes deliver a score for each secondary structure element for the central position of the window. Using the secondary structure with the highest score, PSIPRED generates the protein prediction. The Q3 value is the fraction of residues predicted correctly in the secondary structure states, namely helix, strand and coil.

### Step-by-step description of an exemplary embodiment:

With the invention generally described above, certain non-limiting but illustrative embodiments are described below with reference to representative flow charts in the figures.

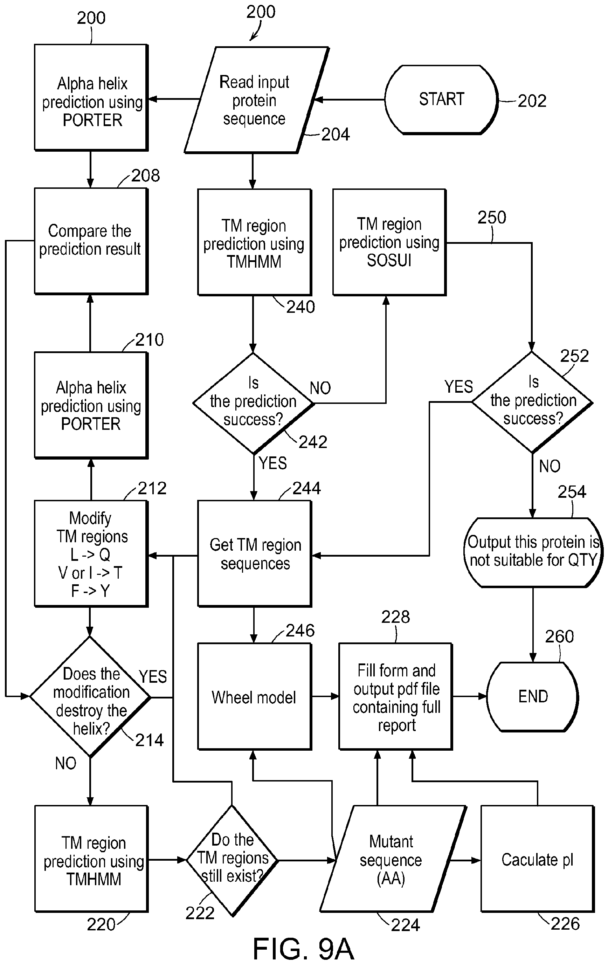

FIG. 9A illustrates one embodiment of the invention that is non-limiting. It generally illustrates a method **200** of the invention in which selected hydrophobic amino acids L, I, V, and F in the TM region of the proteins (e.g., GPCR) are replaced according to the "QTY Code" of the invention, without limiting the substitutions in any particular TM region / domain.

In that specific embodiment, the process starts **202** by acquiring or reading **204** an input of a protein sequence which may or may not be a transmembrane protein. The protein sequence can then be subject to TM region prediction **206** (if such information is not already available from the input protein sequence) and alpha-helical secondary structure prediction based on any of art-recognized methods. The TM region prediction, for example, can be performed using a program **240** such as the TMHMM program. If the prediction does not yield any TM region at **242**, it may be possible that one or more different TM region prediction programs **250**, such as SOSUI, can be used to predict the presence / absence of TM region. If no TM region is predicted based on such programs at **252**, it is likely that no TM region exists in the protein **254**, and the process will terminate **260.**

On the other hand, if one or more TM region(s) are predicted by any of the suitable programs at **242**, the TM region protein sequences are obtained **244**, and the QTY Code of the invention can be applied to the hydrophobic amino acids L, I, V, and F within such TM region(s). More specifically, according to the QTY code, each leucine in the TM regions can be independently substituted **212** by glutamine (Q), serine (S), or asparagine (N), or remain unsubstituted; each isoleucine and valine in the TM regions can be independently substituted by threonine (T), serine (S), or asparagine (N), or remain unsubstituted; and each phenylalanine in the TM regions can be substituted by tyrosine (Y), or remain unsubstituted. The result of such QTY substitution produces one or more putative water-soluble variants of the original transmembrane protein. Note that the number of substitutions made for each amino acid in a region can be selected as a parameter.

Next, the alpha-helical secondary structures in each putative water-soluble variant can be predicted using any art-recognized programs, such as PORTER **210.** The result can be compared to that of the original protein **208**, preferably predicted using the same program (e.g., PORTER). Note that the alpha-helical secondary structure of the original protein can be predicted using any art-recognized program, wither before, concurrently, or after the TM region prediction step of the original protein.

If the result of the alpha-helical secondary structure prediction shows that the potential water-soluble variant has maintained or largely maintained the same alpha-helical secondary structure as the original protein at **214**, it suggests that the specific pattern of QTY substitution in that variant does not or does not significantly affect the alpha-helical secondary structure in the original protein. The TM regions' prediction can then be conducted **220**, verified **222**, and the mutant sequence generated **224.** Optionally, if the result shows that one or more of the alpha-helical secondary structure(s) in the original protein is destroyed at **214**, the variant can be discarded at this step as undesirable, thus terminating the process.

On the other hand, the method of the invention also requires the predicted QTY variant to show less or no propensity to form TM region, as compared to the original protein. Thus the putative water-soluble variant can be subject to TM region prediction, such as using the same TM region prediction program used for the initial TM region prediction (if necessary) in the original protein. If the result shows that significant TM region still exist, the variant may be discarded. On the other hand, if the result shows that no TM region exists, or the propensity of forming TM regions is low, the variant can be selected as the desired variant having enhanced water-solubility over the original protein, while having maintained the alpha-helical secondary structure and hence likely the function of the original protein.

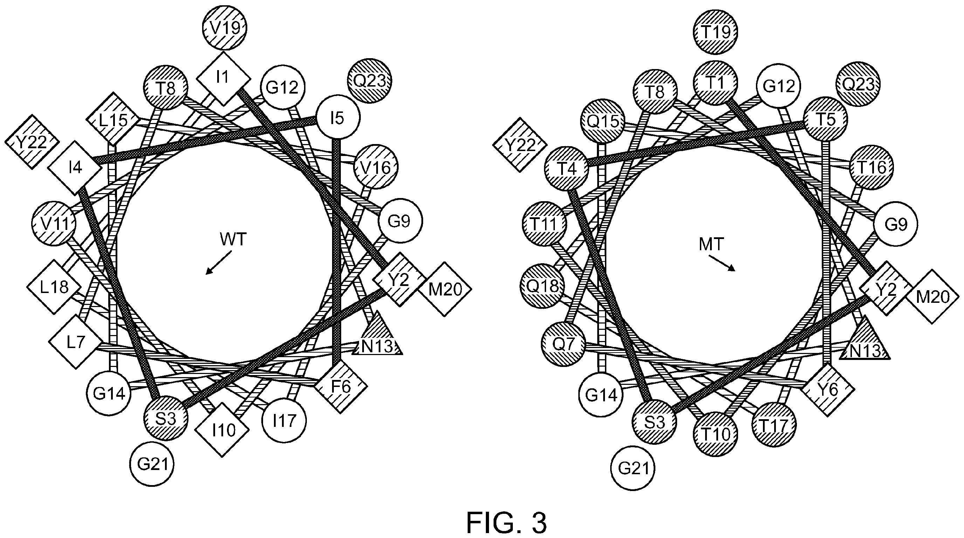

If desired, additional steps can be performed to provide further characterization of the resulting water-soluble variant. Such additional characterization may include calculating **226** the pI of the variant and compare it to that of the original protein. The pI should have no change or very little change (i.e. less than 30 percent, or preferably less than 20 percent or more preferably less than 10 percent). Other additional characterization may include creating a helical wheel model **246** (such as the one shown in FIG. 3) to show the location and any clustering of the QTY substitutions on any particular TM regions.

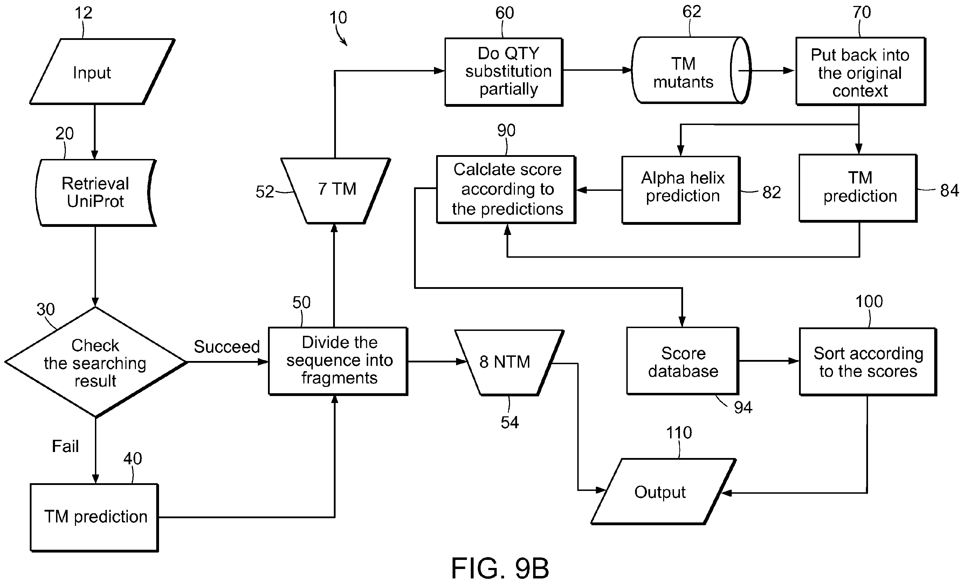

Another illustrative embodiment of the invention for designing the transmembrane regions of a protein (e.g., a GPCR) by the QTY Code of the invention can be performed on a computer system, using the representative process **10** described in FIG. 9B, some of the detailed steps are further described below. Many of the steps are optional or can be combined according to the methods of the invention.
1: In step 1, a computer interface of a computer system receives a protein sequence, selected for analysis, and data descriptive of the protein (e.g., the sequence) entered, uploaded or inputted **12** through a computer interface of a computer system. The data entered can be a protein name, a database reference, or a protein sequence. For example, the protein sequence can be uploaded through a computer interface.
2: In step 2, additional data about the protein can be identified, determined, obtained and/or entered, including its name or sequence and entered via the computer interface. One source to obtain **20** protein data is a database named UniProt (www dot uniprot dot org). Alternatively, the method of the invention can store data relating to the protein, or related sequences to the protein, for later retrieval by the user in this step. In embodiments, the program can prompt the user to select a database or file for retrieving additional data (e.g., sequence data) relating to the protein selected for analysis.
3: In step 3, the user can enter, upload, or obtain data identifying the transmembrane regions. For example, the user can be prompted to obtain the data from a public source, such as from UniProt. The information can be verified **30** and collected from the database for use in Step 5.
4: Alternatively or additionally, if the TM region information is not readily available from the input protein sequence, the transmembrane region can nevertheless be established **40** by any art recognized methods. Transmembrane regions are generally characterized by an alpha helical conformation. Transmembrane helix prediction can be predicted, for example, using a software module / package named TMHMM 2.0 (TransMembrane prediction using Hidden Markov Models), developed by Center for Biological Sequence Analysis (www dot cbs dot dtu dot dk slash services slash TMHMM). A version of the software may have problems on peak finding and sometimes fails to find 7-TM regions for a GPCR. Therefore, a modified version of the program may be used when necessary, wherein the peak searching method executed by the computer system introduces a dynamic baseline. Here, for example, in the case of a GPCR, if all seven TM regions using the initial baseline value are not found, the baseline can be changed to a lower value. For example, the default baseline may be set at 0.2. To identify a missing seventh transmembrane region, one can set the baseline value to 0.1. If more than seven TM regions are found, the baseline can be changed to a higher value, such as 0.15, to eliminate spurious TM prediction. For example, when the CCR-2 amino acid sequence was subjected to the TMHMM 2.0 software, only 6 transmembrane regions were initially identified. When the TMHMM 2.0 baseline value was set to 0.07, however, a correct total of 7 transmembrane regions were identified. The result of the TM region prediction is then provided to step 5.
5: in step 5, after identifying the TM data either through de novo prediction or through obtaining such information through the initial sequence input, the sequence of a GPCR is divided **50** into a total of 15 fragments (i.e., 7-transmembrane segments (7TM) **52** and 8 non-transmembrane segments (8NTM)) **54** according to the TM region information. That is, there should be 7TM and 8 NTM fragments for each typical GPCR.
   It is understood that the system can execute one or more, such as all of the steps described above, using a computer interface for input by a user. It is also understood that the system can omit one or more of the steps described above, or combine two or more steps.
6: In step 6, QTY substitution **60** is performed partially, on a selected subsets of hydrophobic amino acids L, I, V, and F within a given TM region of the protein. Specifically, a first transmembrane region (typically, but not necessarily, the transmembrane region which is most proximal to the N-terminal of the protein) is first selected for variation. Some or all of the hydrophobic amino acids (L, I, V, and F) in the first transmembrane region are then substituted with the corresponding non-ionic hydrophilic amino acids (Q/S/N, T/S/N, T/S/N, or Y). It is understood that the amino acid is not actually substituted into the protein in this context. Rather, the amino acid designation is substituted in the sequence for modeling. Thus, the term "sequence" is intended to include "sequence data." Typically, most or all of the hydrophobic amino acids are selected for substitution. If less than all amino acids are selected, it may be desirable to select the internal hydrophobic amino acids leaving one or more N and/or C terminal amino acids of the transmembrane regions hydrophobic. Additionally or alternatively, it may be desirable to select to replace all of the leucines (L) in a transmembrane region. Additionally or alternatively, it may be desirable to select and replace all of the isoleucines (I) in a transmembrane region. Additionally or alternatively, it may be desirable to select to replace all of the valines (V) in a transmembrane region. Additionally or alternatively, it may be desirable to select to replace all of the phenylalanines (F) in a transmembrane region. Additionally or alternatively, it can be beneficial to retain one or more phenylalanines in the transmembrane region. Additionally or alternatively, it can be beneficial to retain one or more valines in the transmembrane region. Additionally or alternatively, it can be beneficial to retain one or more leucines in the transmembrane region. Additionally or alternatively, it can be beneficial to retain one or more isoleucines in the transmembrane region. Additionally or alternatively, it can be beneficial to retain one or more hydrophobic amino acids in the transmembrane region where the wild type sequence is characterized by three or more contiguous hydrophobic amino acids.
7: In step 7, the transmembrane region so designed is put back into the context of the original protein. That is, the mutated or re-designed TM region **62** with the QTY substitutions is swapped into the corresponding TM region of the original protein, to create the transmembrane variants **70** or "putative variants," since each sets of substitution creates one specific putative variant for that TM region. Together, these related putative variants form a first library of putative variants.
8: In steps **82** and **84**, each putative variant is then subjected to the transmembrane region prediction process (**84**), as discussed herein (e.g., loss of predicted TM region). The variant is also assessed a score for the sequence's propensity to form an alpha helix (**82**). The variant is also subjected to a water solubility prediction process, as discussed herein. For example, the variant is assessed a score for the sequence's propensity to be water soluble. Such score may be based on a predicted propensity to form TM regions, with strong propensity to form TM regions being associated with poor water solubility, and low or now propensity to form TM regions being associated with high water solubility. Of course, complete water solubility at all concentrations is not required for most commercial purposes. Water solubility is preferably determined to be that required for functionality at the predicted conditions of use (e.g., in a ligand binding assay).
9: In step 9, putative variants that predict loss of alpha helical structure and/or "water insolubility" (predicted at the expected conditions of use) are discarded. Putative variants that predict alpha helical structure and water solubility can be selected, such as by using the combined score or rank **90** that is a weighted combination based on a ranking function of the alpha-helical secondary structure prediction result and the TM region / water solubility prediction result. For example, one can select transmembrane variants that are highly water soluble, or are characterized by 0, 1, 2, or 3 hydrophobic amino acids (e.g., higher weight for the water solubility prediction result), with a possible expectation that alpha helical structure can be compromised. Alternatively or additionally, one can select highly alpha-helical structures (e.g., higher weight for the alpha helical secondary structure prediction result), characterized by 3, 4, 5 or 6 hydrophobic amino acids.
10: In step 10, the putative variants in the same library **94** can be sorted or ranked **100** based on the score calculation scheme outlined above. Then a pre-determined number of putative variants can be selected as the final members in the first putative variant library. For example, in the combined score described above, a score of 0 means no propensity to form TM region, and complete maintenance of the original alpha helical secondary structure, and is thus the most desired putative variant. A slightly higher score may indicate a slight propensity to form TM region (or a less propensity of being water soluble). Thus the putative variant is less desirable but may still be selected based on its superior combined score compared to the other putative variants in the library.
   In certain embodiments, a pre-determined number of desired putative variants can be selected, such as 10, 9, 8, 7, 6, 5, 4, 3, 2, or 1.
   These steps (e.g., steps 6-10) can be repeated for a second, third, fourth, fifth, sixth and/or seventh (or more) transmembrane region or domain to create one putative variant library for each such TM regions or domains.
11: In step 11, one can select **110** a combination of the TM regions or domains with the putative variants and the unsubstituted non-TM regions. For example, one, two, three or four domains with putative variants possessing high alpha-helical structure scores and one, two, three, four, five, or six domains with putative variants possessing high water solubility scores can be combined. In another example, one can combine a domain / TM region that is characterized by all hydrophobic amino acids being substituted by a hydrophilic amino acid, thus maximizing the water solubility score, and a second domain / TM region that retains 3, 4, or 5 hydrophobic amino acids in a plurality of variant selections. Such selected putative variants can be "shuffled," as is known in the art, with the extracellular and intracellular domains to create an initial combinatorial library of putative water-soluble protein variants.

In certain embodiments, all or a fraction of the putative water soluble protein variants of the initial combinatorial library designed as described herein can be made (produced or expressed in vitro or in a host cell) and screened for water solubility and/or ligand binding, preferably in a high through-put screen. Amplification of the library, for example, can result in less than 100% of the putative water-soluble protein combinatorial variants from being expressed. A reporter system can be used to screen ligand binding, as is well known in the art. Using the methods of the invention, one can rapidly identify a library of putative water soluble modified transmembrane combinatorial variants that contain functionally combined extracellular and intracellular domains, and generate water soluble protein variants possessing the proper 3 dimensional structure of the wild type protein, and retaining ligand binding function (including binding affinity), or other functions. The software can include a learning module in which verified functionality of protein variants is used to eliminate certain variants or rank them differently.

In certain embodiments, to be practical experimentally, the initial combinatorial library has about 2 million potentially water-soluble GPCR, or CXCR4, variants. Of course, a library of more or less variants can be designed as well. Smaller libraries maybe preferred in certain embodiments since they can be optimized based on analysis of the research results as described herein. Analysis of research results is likely to establish trends to optimize the number of domain variants to shuffle and the assumptions for selecting domain variants.

In certain embodiments, certain hydrophobic amino acids in the TM region of the transmembrane proteins are selected for modification based on the helical forming propensity also known as "the helix prediction score" (see www dot proteopedia dot org slash wiki slash index dot php slash Main_Page). The varied fragments are randomly assembled to form about 2M (8⁷) variants of full-length GPCR genes. The predicted number of variants can generally be characterized by the formula Hⁿ, where n = the number of transmembrane regions modified and/or varied by the method (in the example of GPCR, n=7) and H = the number of putative variants in each transmembrane region available for generating the combinatorial variants.

Once the initial combinatorial library, or selection of the domain variants to be shuffled, is selected, nucleic acid molecules, or DNA or cDNA molecules, encoding the proteins in the initial combinatorial library can be designed. The nucleic acid molecules are preferably designed to provide codon optimization and intron deletions for the expression systems selected to produce a library of coding sequences. For example, if the expression system is *E. coli,* codons optimized for *E. coli* expression can be selected. See www dot dna20 dot com slash resources slash genedesigner. In addition, a promoter region, such as a promoter suitable for expression in the expression system (e.g., *E. coli*) is selected and operatively connected to the coding sequences in the library of coding sequences.

The initial library of coding sequences, or a portion thereof, is then expressed to produce a library of putative water soluble GPCRs. The library is then subjected to a ligand binding assay. In the binding assay, the putative water soluble GPCRs are contacted with the ligand, preferably in an aqueous medium and ligand binding is detected.

The invention includes transmembrane domain variants, and nucleic acid molecules encoding same, obtained, or obtainable, from the methods described herein.

The invention also contemplates water soluble GPCR variants ("sGPCRs") characterized by a plurality of transmembrane domains independently characterized by at least 50%, preferably at least about 60%, more preferably at least about 70% or 80%, such as at least about 90%) of the hydrophobic amino acid residues (L, I, V, and F) of a native transmembrane protein (e.g., GPCR) substituted by a Q, T, T, or Y, respectively). The sGPCRs of the invention are characterized by water solubility and ligand binding. In particular, the sGPCR binds the same natural ligand as the corresponding native GPCR.

The invention further encompasses a method of treatment for a disorder or disease that is mediated by the activity of a membrane protein, comprising the use of a water-soluble polypeptide to treat said disorders and diseases, wherein said water-soluble polypeptide comprises a modified α-helical domain, and wherein said water-soluble polypeptide retains the ligand-binding activity of the native membrane protein. Examples of such disorders and diseases include, but are not limited to, cancer, small cell lung cancer, melanoma, breast cancer, Parkinson's disease, cardiovascular disease, hypertension, and asthma.

As described herein, the water-soluble peptides described herein can be used for the treatment of conditions or diseases mediated by the activity of a membrane protein. In certain aspects, the water-soluble peptides can act as "decoys" for the membrane receptor and bind to the ligand that otherwise activates the membrane receptor. As such, the water-soluble peptides described herein can be used to reduce the activity of a membrane protein. These water-soluble peptides can remain in the circulation and competitively bind to specific ligands, thereby reducing the activity of membrane bound receptors. For example, the GPCR CXCR4 is over-expressed in small cell lung cancer and facilitates metastasis of tumor cells. Binding of this ligand by a water-soluble peptide such as that described herein may significantly reduce metastasis.

The chemokine receptor, CXCR4, is known in viral research as a major coreceptor for the entry of T cell line-tropic HIV (Feng et al. (1996) Science 272: 872-877; Davis et al. (1997) J Exp Med 186: 1793-1798; Zaitseva et al. (1997) Nat Med 3: 1369-1375; Sanchez et al. (1997) J Biol Chem 272: 27529-27531). Stromal cell derived factor 1 (SDF-1) is a chemokine that interacts specifically with CXCR4. When SDF-1 binds to CXCR4, CXCR4 activates Gai protein-mediated signaling (pertussis toxin-sensitive) (Chen et al. (1998) Mol Pharmacol 53: 177-181), including downstream kinase pathways such as Ras/MAP Kinases and phosphatidylinositol 3-kinase (PI3K)/Akt in lymphocyte, megakaryocytes, and hematopoietic stem cells (Bleul et al. (1996) Nature 382: 829-833; Deng et al. (1997) Nature 388: 296-300; Kijowski et al. (2001) Stem Cells 19: 453-466; Majka et al. (2001) Folia. Histochem. Cytobiol. 39: 235-244; Sotsios et al. (1999) J. Immunol. 163: 5954-5963; Vlahakis et al. (2002) J. Immunol. 169: 5546-5554). In mice transplanted with human lymph nodes, SDF-1 induces CXCR4-positive cell migration into the transplanted lymph node (Blades et al. (2002) J. Immunol. 168: 4308-4317).

Recently, studies have shown that CXCR4 interactions may regulate the migration of metastatic cells. Hypoxia, a reduction in partial oxygen pressure, is a microenvironmental change that occurs in most solid tumors and is a major inducer of tumor angiogenesis and therapeutic resistance. Hypoxia increases CXCR4 levels (Staller et al. (2003) Nature 425: 307-311). Microarray analysis on a sub-population of cells from a bone metastatic model with elevated metastatic activity showed that one of the genes increased in the metastatic phenotype was CXCR4. Furthermore, overexpression CXCR4 in isolated cells significantly increased the metastatic activity (Kang et al. (2003) Cancer Cell 3: 537-549). In samples collected from various breast cancer patients, Muller et al. (Muller et al. (2001) Nature 410: 50-56) found that CXCR4 expression level is higher in primary tumors relative to normal mammary gland or epithelial cells. Moreover, CXCR4 antibody treatment has been shown to inhibit metastasis to regional lymph nodes when compared to control isotypes that all metastasized to lymph nodes and lungs (Muller et al. (2001). As such a decoy therapy model is suitable for treating CXCR4 mediated diseases and disorders.

In another embodiment of the invention relates to the treatment of a disease or disorder involving CXCR4-dependent chemotaxis, wherein the disease is associated with aberrant leukocyte recruitment or activation. The disease is selected from the group consisting of arthritis, psoriasis, multiple sclerosis, ulcerative colitis, Crohn's disease, allergy, asthma, AIDS associated encephalitis, AIDS related maculopapular skin eruption, AIDS related interstitial pneumonia, AIDS related enteropathy, AIDS related periportal hepatic inflammation and AIDS related glomerulo nephritis.

In another aspect, the invention relates to the treatment of a disease or disorder selected from arthritis, lymphoma, non-small lung cancer, lung cancer, breast cancer, prostate cancer, multiple sclerosis, central nervous system developmental disease, dementia, Parkinson's disease, Alzheimer's disease, tumor, fibroma, astrocytoma, myeloma, glioblastoma, an inflammatory disease, an organ transplantation rejection, AIDS, HIV-infection or angiogenesis.

The invention also encompasses a pharmaceutical composition comprising said water-soluble polypeptide and a pharmaceutically acceptable carrier or diluent.

The compositions can also include, depending on the formulation desired, pharmaceutically-acceptable, non-toxic carriers or diluents, which are defined as vehicles commonly used to formulate pharmaceutical compositions for animal or human administration. The diluent is selected so as not to affect the biological activity of the pharmacologic agent or composition. Examples of such diluents are distilled water, physiological phosphate-buffered saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like. Pharmaceutical compositions can also include large, slowly metabolized macromolecules such as proteins, polysaccharides such as chitosan, polylactic acids, polyglycolic acids and copolymers (such as latex functionalized SEPHAROSE™, agarose, cellulose, and the like), polymeric amino acids, amino acid copolymers, and lipid aggregates (such as oil droplets or liposomes).

The compositions can be administered parenterally such as, for example, by intravenous, intramuscular, intrathecal or subcutaneous injection. Parenteral administration can be accomplished by incorporating a composition into a solution or suspension. Such solutions or suspensions may also include sterile diluents such as water for injection, saline solution, fixed oils, polyethylene glycols, glycerine, propylene glycol or other synthetic solvents. Parenteral formulations may also include antibacterial agents such as, for example, benzyl alcohol or methyl parabens, antioxidants such as, for example, ascorbic acid or sodium bisulfite and chelating agents such as EDTA. Buffers such as acetates, citrates or phosphates and agents for the adjustment of tonicity such as sodium chloride or dextrose may also be added. The parenteral preparation can be enclosed in ampules, disposable syringes or multiple dose vials made of glass or plastic.

Additionally, auxiliary substances, such as wetting or emulsifying agents, surfactants, pH buffering substances and the like can be present in compositions. Other components of pharmaceutical compositions are those of petroleum, animal, vegetable, or synthetic origin, for example, peanut oil, soybean oil, and mineral oil. In general, glycols such as propylene glycol or polyethylene glycol are preferred liquid carriers, particularly for injectable solutions.

Injectable formulations can be prepared either as liquid solutions or suspensions; solid forms suitable for solution in, or suspension in, liquid vehicles prior to injection can also be prepared. The preparation also can also be emulsified or encapsulated in liposomes or micro particles such as polylactide, polyglycolide, or copolymer for enhanced adjuvant effect, as discussed above. Langer, Science 249: 1527, 1990; and Hanes, Advanced Drug Delivery Reviews 28: 97-119, 1997. The compositions and pharmacologic agents described herein can be administered in the form of a depot injection or implant preparation which can be formulated in such a manner as to permit a sustained or pulsatile release of the active ingredient.

Transdermal administration includes percutaneous absorption of the composition through the skin. Transdermal formulations include patches, ointments, creams, gels, salves and the like. Transdermal delivery can be achieved using a skin patch or using transferosomes. See Paul et al., Eur. J. Immunol. 25: 3521-24, 1995; and Cevc et al., Biochem. Biophys. Acta 1368: 201-15, 1998.

"Treating" or "treatment" includes preventing or delaying the onset of the symptoms, complications, or biochemical indicia of a disease, alleviating or ameliorating the symptoms or arresting or inhibiting further development of the disease, condition, or disorder. A "patient" is a human subject in need of treatment.

An "effective amount" refers to that amount of the therapeutic agent that is sufficient to ameliorate of one or more symptoms of a disorder and/or prevent advancement of a disorder, cause regression of the disorder and/or to achieve a desired effect.

### Computer System

Various aspects and functions described herein may be implemented as specialized hardware or software components executing in one or more computer systems. There are many examples of computer systems that are currently in use. These examples include, among others, network appliances, personal computers, workstations, mainframes, networked clients, servers, media servers, application servers, database servers, and web servers. Other examples of computer systems may include mobile computing devices, such as cellular phones and personal digital assistants, and network equipment, such as load balancers, routers, and switches. Further, aspects may be located on a single computer system or may be distributed among a plurality of computer systems connected to one or more communications networks.

For example, various aspects, functions, and processes may be distributed among one or more computer systems configured to provide a service to one or more client computers, or to perform an overall task as part of a distributed system. Additionally, aspects may be performed on a client-server or multi-tier system that includes components distributed among one or more server systems that perform various functions. Consequently, embodiments are not limited to executing on any particular system or group of systems. Further, aspects, functions, and processes may be implemented in software, hardware or firmware, or any combination thereof. Thus, aspects, functions, and processes may be implemented within methods, acts, systems, system elements and components using a variety of hardware and software configurations, and examples are not limited to any particular distributed architecture, network, or communication protocol.

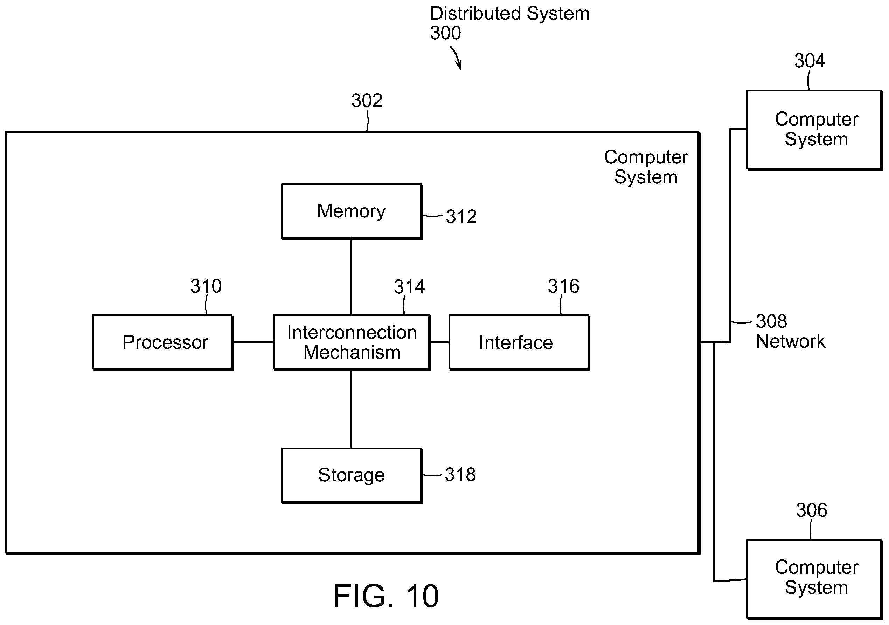

Referring to FIG. 10, there is illustrated a block diagram of a distributed computer system 300, in which various aspects and functions are practiced. As shown, the distributed computer system 300 includes one or more computer systems that exchange information. More specifically, the distributed computer system 300 includes computer systems 302, 304, and 306. As shown, the computer systems 302, 304, and 306 are interconnected by, and may exchange data through, a communication network 308. The network 308 may include any communication network through which computer systems may exchange data. To exchange data using the network 308, the computer systems 302, 304, and 306 and the network 308 may use various methods, protocols and standards. Examples of these protocols and standards include NAS, Web, storage and other data movement protocols suitable for use in a big data environment. To ensure data transfer is secure, the computer systems 302, 304, and 306 may transmit data via the network 308 using a variety of security measures including, for example, SSL or VPN technologies. While the distributed computer system 300 illustrates three networked computer systems, the distributed computer system 300 is not so limited and may include any number of computer systems and computing devices, networked using any medium and communication protocol.

As illustrated in FIG. 10, the computer system 302 includes a processor 310, a memory 312, an interconnection element 314, an interface 316 and data storage element 318. To implement at least some of the aspects, functions, and processes disclosed herein, the processor 310 performs a series of instructions that result in manipulated data. The processor 310 may be any type of processor, multiprocessor or controller. Example processors may include a commercially available processor such as an Intel Xeon, Itanium, Core, Celeron, or Pentium processor; an AMD Opteron processor; an Apple A4 or A5 processor; a Sun UltraSPARC processor; an IBM Power5+ processor; an IBM mainframe chip; or a quantum computer. The processor 310 is connected to other system components, including one or more memory devices 312, by the interconnection element 314.

The memory 312 stores programs (e.g., sequences of instructions coded to be executable by the processor 310) and data during operation of the computer system 302. Thus, the memory 312 may be a relatively high performance, volatile, random access memory such as a dynamic random access memory ("DRAM") or static memory ("SRAM"). However, the memory 312 may include any device for storing data, such as a disk drive or other nonvolatile storage device. Various examples may organize the memory 312 into particularized and, in some cases, unique structures to perform the functions disclosed herein. These data structures may be sized and organized to store values for particular data and types of data.

Components of the computer system 302 are coupled by an interconnection element such as the interconnection element 314. The interconnection element 314 may include any communication coupling between system components such as one or more physical busses in conformance with specialized or standard computing bus technologies such as IDE, SCSI, PCI and InfiniBand. The interconnection element 314 enables communications, including instructions and data, to be exchanged between system components of the computer system 302.

The computer system 302 also includes one or more interface devices 316 such as input devices, output devices and combination input/output devices. Interface devices may receive input or provide output. More particularly, output devices may render information for external presentation. Input devices may accept information from external sources. Examples of interface devices include keyboards, mouse devices, trackballs, microphones, touch screens, printing devices, display screens, speakers, network interface cards, etc. Interface devices allow the computer system 302 to exchange information and to communicate with external entities, such as users and other systems.

The data storage element 318 includes a computer readable and writeable nonvolatile, or non-transitory, data storage medium in which instructions are stored that define a program or other object that is executed by the processor 310. The data storage element 318 also may include information that is recorded, on or in, the medium, and that is processed by the processor 310 during execution of the program. More specifically, the information may be stored in one or more data structures specifically configured to conserve storage space or increase data exchange performance. The instructions may be persistently stored as encoded signals, and the instructions may cause the processor 310 to perform any of the functions described herein. The medium may, for example, be optical disk, magnetic disk or flash memory, among others. In operation, the processor 310 or some other controller causes data to be read from the nonvolatile recording medium into another memory, such as the memory 312, that allows for faster access to the information by the processor 310 than does the storage medium included in the data storage element 318. The memory may be located in the data storage element 318 or in the memory 312, however, the processor 310 manipulates the data within the memory, and then copies the data to the storage medium associated with the data storage element 318 after processing is completed. A variety of components may manage data movement between the storage medium and other memory elements and examples are not limited to particular data management components. Further, examples are not limited to a particular memory system or data storage system.

Although the computer system 302 is shown by way of example as one type of computer system upon which various aspects and functions may be practiced, aspects and functions are not limited to being implemented on the computer system 302 as shown in FIG. 10. Various aspects and functions may be practiced on one or more computers having a different architectures or components than that shown in FIG. 10. For instance, the computer system 302 may include specially programmed, special-purpose hardware, such as an application-specific integrated circuit ("ASIC") tailored to perform a particular operation disclosed herein. While another example may perform the same function using a grid of several general-purpose computing devices running MAC OS System X with Motorola PowerPC processors and several specialized computing devices running proprietary hardware and operating systems.

The computer system 302 may be a computer system including an operating system that manages at least a portion of the hardware elements included in the computer system 302. In some examples, a processor or controller, such as the processor 310, executes an operating system. Examples of a particular operating system that may be executed include a Windows-based operating system, such as, Windows NT, Windows 2000 (Windows ME), Windows XP, Windows Vista or Windows 7 operating systems, available from the Microsoft Corporation, a MAC OS System X operating system or an iOS operating system available from Apple Computer, one of many Linux-based operating system distributions, for example, the Enterprise Linux operating system available from Red Hat Inc., a Solaris operating system available from Oracle Corporation, or a UNIX operating systems available from various sources. Many other operating systems may be used, and examples are not limited to any particular operating system.

The processor 310 and operating system together define a computer platform for which application programs in high-level programming languages are written. These component applications may be executable, intermediate, bytecode or interpreted code which communicates over a communication network, for example, the Internet, using a communication protocol, for example, TCP/IP. Similarly, aspects may be implemented using an object-oriented programming language, such as .Net, SmallTalk, Java, C⁺⁺, Ada, C# (C-Sharp), Python, or JavaScript. Other object-oriented programming languages may also be used. Alternatively, functional, scripting, or logical programming languages may be used.

Additionally, various aspects and functions may be implemented in a non-programmed environment. For example, documents created in HTML, XML or other formats, when viewed in a window of a browser program, can render aspects of a graphical-user interface or perform other functions. Further, various examples may be implemented as programmed or non-programmed elements, or any combination thereof. For example, a web page may be implemented using HTML while a data object called from within the web page may be written in C⁺⁺. Thus, the examples are not limited to a specific programming language and any suitable programming language could be used. Accordingly, the functional components disclosed herein may include a wide variety of elements (e.g., specialized hardware, executable code, data structures or objects) that are configured to perform the functions described herein.

In some examples, the components disclosed herein may read parameters that affect the functions performed by the components. These parameters may be physically stored in any form of suitable memory including volatile memory (such as RAM) or nonvolatile memory (such as a magnetic hard drive). In addition, the parameters may be logically stored in a propriety data structure (such as a database or file defined by a user space application) or in a commonly shared data structure (such as an application registry that is defined by an operating system). In addition, some examples provide for both system and user interfaces that allow external entities to modify the parameters and thereby configure the behavior of the components.

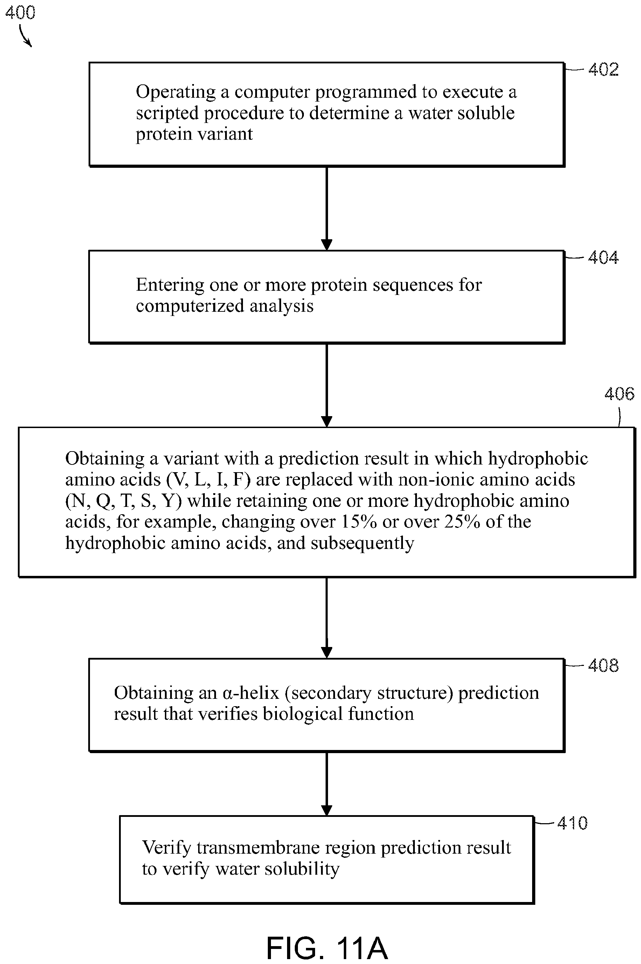

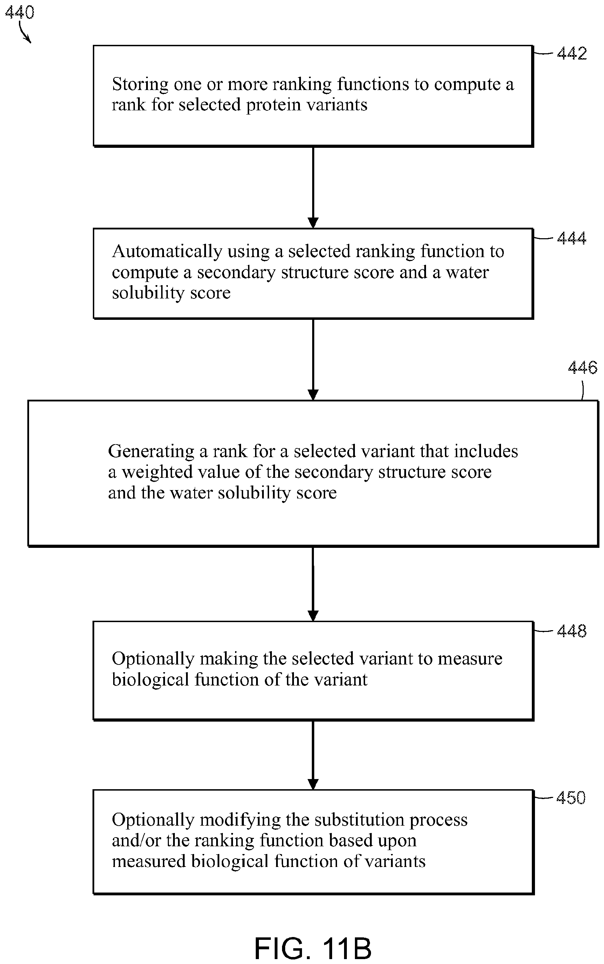

The software is generally depicted in FIG. 11A to perform a computational method in which the user selects operating parameters to execute a procedure on a computer **402**, as previously described herein, where one or more sequences are entered **404**, and substitutions are performed **408.** The system is operative to verify secondary structures **408** and verify water solubility for the one or more variants. As shown in FIG. 11B, the program can include additional processing options in addition to those previously described, wherein one or more ranking functions **442** can be stored, the user can select or the system can automatically select **444** the ranking function to be used. The system can then generate a rank **446** as described herein, and then a user can make **448** a selected variant to measure function **448**, and subsequently enter functional data to modify the processing sequence **450** based thereon.

The invention will be better understood in connection with the following example, which is intended as an illustration only and not limiting of the scope of the invention. Various changes and modifications to the disclosed embodiments will be apparent to those skilled in the art and such changes and may be made without departing from the spirit of the invention and the scope of the appended claims.

### EXAMPLES

### Example 1: CXC chemokine receptor type 4 isoform a (CXCR4):

CXCR4 is a chemokine receptor 356 amino acids in length. It has a pI of about 8.61 and a Molecular Weight of 40221.19 Da. The sequence for CXCR4, as published in the literature, is:

MSIPLPLLQIYTSDNYTEEMGSGDYDSMKEPCFREENANFNKIFLPTIYSIIFLTGIVGN
GLVILVMGYQKKLRSMTDKYRLHLSVADLLFVITLPFWAVDAVANWYFGNFLCKAVHVIY
TVNLYSSVLILAFISLDRYLAIVHATNSQRPRKLLAEKVVYVGVWIPALLLTIPDFIFAN
VSEADDRYICDRFYPNDLWVVVFQFQHIMVGLILPGIVILSCYCIIISKLSHSKGHQKRK
ALKTTVILILAFFACWLPYYIGISIDSFILLEIIKQGCEFENTVHKWISITEALAFFHCC
LNPILYAFLGAKFKTSAQHALTSVSRGSSLKILSKGKRGGHSSVSTESESSSFHSS (SEQ ID NO. 1).

Subjecting the sequence to TMHMM results in the identification of the transmembrane domains as depicted in FIG. 3.

Replacing all or substantially all of the hydrophobic amino acids, L, I V, and F, with Q, T and Y (respectively) results in the following sequence:

1 MSIPLPLLQIYTSDNYTEEMGSGDYDSMKEPCFREENANFNKIFLPTTYSTTYQTGTTGN
61 GQTTQTMGYQKKLRSMTDKYRQHQSTADQQYTTTQPYWATDAVANWYFGNFLCKATHTTY
121 TTNQYSSTQTQAYTSQDRYLAIVHATNSQRPRKLLAEKTTYTGTWTPAQQQTTPDYTYAN
181 VSEADDRYICDRFYPNDLWVVVYQYQHTMTGQTQPGTTTQSCYCTIISKLSHSKGHQKRK
241 ALKTTTIQTQAYYACWQPYYTGTSTDSYILLEIIKQGCEFENTVHKWTSTTEAQAYYHCC
301 QNPTQYAYQGAKFKTSAQHALTSVSRGSSLKILSKGKRGGHSSVSTESESSSFHSS (SEQ ID NO:
2).

The predicted pI of the protein is 8.54 and the Molecular Weight is 40551.64 Da. Each of the predicted transmembrane regions has been underlined and exemplified a fully modified domain of the invention. Thus, for example, the invention includes a transmembrane domain comprising Amino Acids 47-70 of SEQ ID NO: 2 (TM1), and proteins comprising the same. As an example, FIG 3 represents the alpha-helical prediction of the TM1 sequence. Preferably the protein comprising TM1 herein includes one or more (e.g., all) of the extracellular and intracellular loop sequences of SEQ ID NO: 2 (the sequences which have not been underlined). In addition or alternatively, the protein comprising the TM1 herein includes one or more additional transmembrane regions (the underlined sequences) in SEQ ID NO: 2 or homologous sequences retaining one, two, three or, possibly four or more of the native L, I V and F amino acids, as set forth in SEQ ID NO: 1.

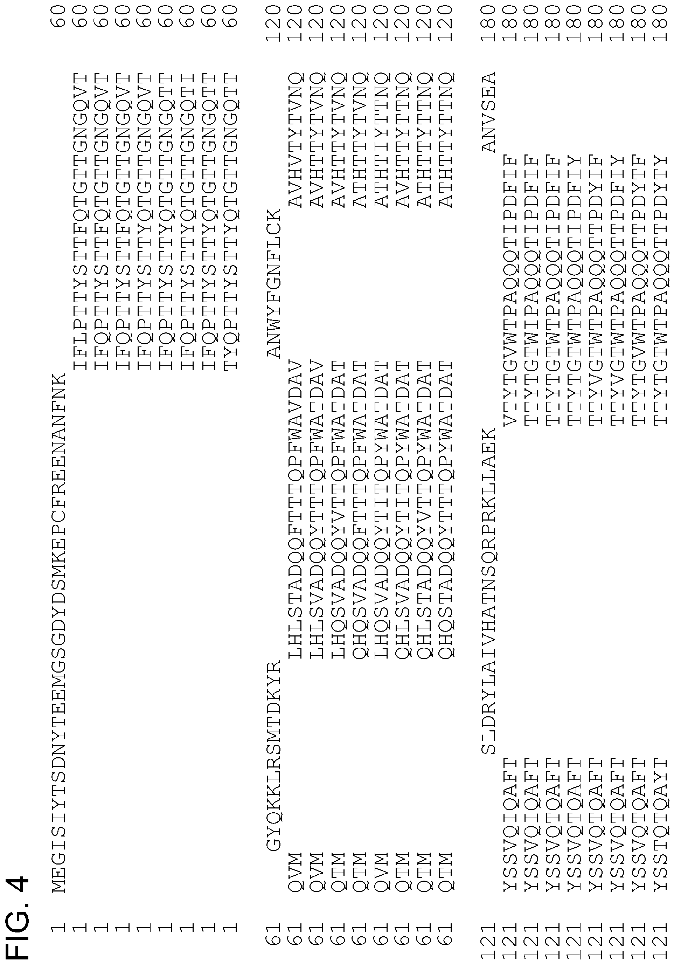

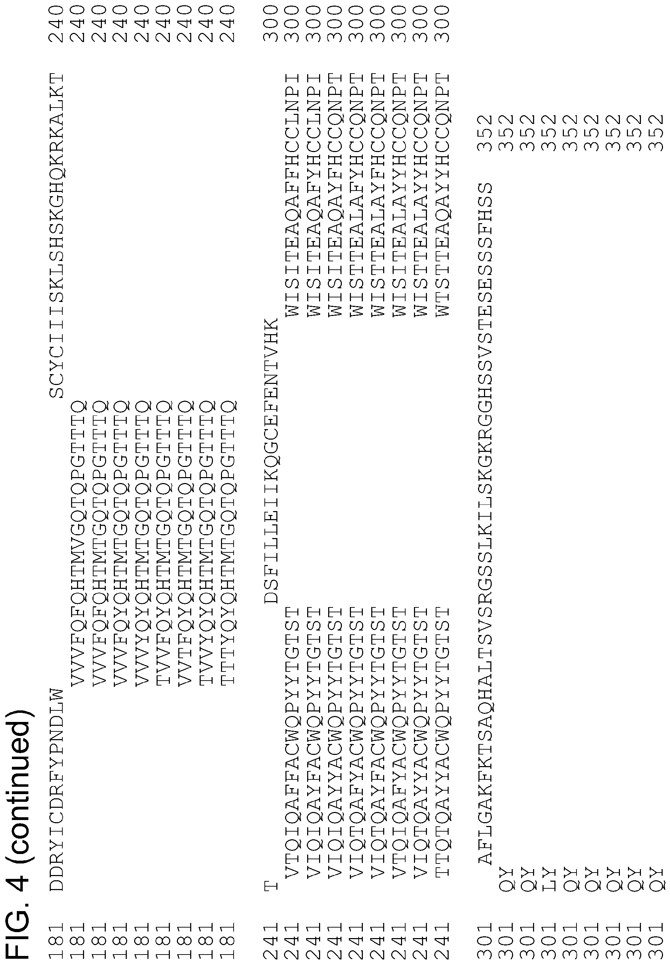

The native protein sequence for CXCR4 (differing in the N-terminal amino acids) was subjected to the method a second time. The program output divided the native sequence into the extracellular and intracellular regions and selected 8 transmembrane domain variants for each transmembrane domain. The results are illustrated in FIG. 4 and in the following table:

| | |
|---|---|
| MEGISIYTSDNYTEEMGSGDYDSMKEPCFREENANFNK | (SEQ ID NO. 3; EC1) |
| | |

| TM 1 Variants: | |
|---|---|
| IFLPTTYSTTFQTGTTGNGQVTQVM | (SEQ ID NO. 4) |
| IFQPTTYSTTFQTGTTGNGQVTQVM | (SEQ ID NO. 5) |
| IFQPTTYSTTFQTGTTGNGQVTQTM | (SEQ ID NO. 6) |
| IFQPTTYSTTYQTGTTGNGQVTQTM | (SEQ ID NO. 7) |
| IFQPTTYSTTYQTGTTGNGQTTQVM | (SEQ ID NO. 8) |
| IFQPTTYSTTYQTGTTGNGQTIQTM | (SEQ ID NO. 9) |
| IFQPTTYSTTYQTGTTGNGQTTQTM | (SEQ ID NO. 10) |
| TYQPTTYSTTYQTGTTGNGQTTQTM | (SEQ ID NO. 11) |
| | |
| GYQKKLRSMTDKYR | (SEQ ID NO. 12; IC1) |
| | |

| TM 2 Variants: | |
|---|---|
| LHLSTADQQFTTTQPFWAVDAV | (SEQ ID NO. 13) |
| LHLSVADQQYTTTQPFWATDAV | (SEQ ID NO. 14) |
| LHQSVADQQYVTTQPFWATDAT | (SEQ ID NO. 15) |
| QHQSVADQQFTTTQPFWATDAT | (SEQ ID NO. 16) |
| LHQSVADQQYTITQPYWATDAT | (SEQ ID NO. 17) |
| QHLSVADQQYTITQPYWATDAT | (SEQ ID NO. 18) |
| QHLSTADQQYVTTQPYWATDAT | (SEQ ID NO. 19) |
| QHQSTADQQYTTTQPYWATDAT | (SEQ ID NO. 20) |
| | |
| ANWYFGNFLCK | (SEQ ID NO. 21; EC2) |
| | |

| TM 3 Variants: | |
|---|---|
| AVHVTYTVNQYSSVQIQAFT | (SEQ ID NO. 22) |
| AVHTTYTVNQYSSVQIQAFT | (SEQ ID NO. 23) |
| AVHTTYTVNQYSSVQTQAFT | (SEQ ID NO. 24) |
| ATHTTYTVNQYSSVQTQAFT | (SEQ ID NO. 25) |
| ATHTIYTTNQYSSVQTQAFT | (SEQ ID NO. 26) |
| AVHTTYTTNQYSSVQTQAFT | (SEQ ID NO. 27) |
| ATHTTYTTNQYSSVQTQAFT | (SEQ ID NO. 28) |
| ATHTTYTTNQYSSTQTQAYT | (SEQ ID NO. 29) |
| | |
| SLDRYLAIVHATNSQRPRKLLAEK | (SEQ ID NO. 30; IC2) |
| | |

| TM 4 Variants: | |
|---|---|
| VTYTGVWTPAQQQTIPDFIF | (SEQ ID NO. 31) |
| TTYTGTWIPAQQQTIPDFIF | (SEQ ID NO. 32) |
| TTYTGTWTPAQQQTIPDFIF | (SEQ ID NO. 33) |
| TTYTGTWTPAQQQTIPDFIY | (SEQ ID NO. 34) |
| TTYVGTWTPAQQQTTPDYIF | (SEQ ID NO. 35) |
| TTYVGTWTPAQQQTTPDFIY | (SEQ ID NO. 36) |
| TTYTGVWTPAQQQTTPDYTF | (SEQ ID NO. 37) |
| TTYTGTWTPAQQQTTPDYTY | (SEQ ID NO. 38) |
| | |
| ANVSEADDRYICDRFYPNDLW | (SEQ ID NO. 39; EC3) |
| | |

| TM 5 Variants: | |
|---|---|
| VVVFQFQHTMVGQTQPGTTTQ | (SEQ ID NO. 40) |
| VVVFQFQHTMTGQTQPGTTTQ | (SEQ ID NO. 41) |
| VVVFQYQHTMTGQTQPGTTTQ | (SEQ ID NO. 42) |
| VVVYQYQHTMTGQTQPGTTTQ | (SEQ ID NO. 43) |
| TVVFQYQHTMTGQTQPGTTTQ | (SEQ ID NO. 44) |
| VVTFQYQHTMTGQTQPGTTTQ | (SEQ ID NO. 45) |
| TVVYQYQHTMTGQTQPGTTTQ | (SEQ ID NO. 46) |
| TTTYQYQHTMTGQTQPGTTTQ | (SEQ ID NO. 47) |
| | |
| SCYCIIISKLSHSKGHQKRKALKTT | (SEQ ID NO. 48; IC3) |
| | |

| TM 6 Variants: | |
|---|---|
| VTQIQAFFACWQPYYTGTST | (SEQ ID NO. 49) |
| VIQIQAYFACWQPYYTGTST | (SEQ ID NO. 50) |
| VIQIQAYYACWQPYYTGTST | (SEQ ID NO. 51) |
| VIQTQAFYACWQPYYTGTST | (SEQ ID NO. 52) |
| VIQTQAYFACWQPYYTGTST | (SEQ ID NO. 53) |
| VTQIQAFYACWQPYYTGTST | (SEQ ID NO. 54) |
| VIQTQAYYACWQPYYTGTST | (SEQ ID NO. 55) |
| TTQTQAYYACWQPYYTGTST | (SEQ ID NO. 56) |
| | |
| DSFILLEIIKQGCEFENTVHK | (SEQ ID NO. 57; EC4) |
| | |

| TM 7 Variants | |
|---|---|
| WISITEAQAFFHCCLNPIQY | (SEQ ID NO. 58) |
| WISITEAQAFYHCCLNPIQY | (SEQ ID NO. 59) |
| WISITEAQAYFHCCQNPTLY | (SEQ ID NO. 60) |
| WISTTEALAFYHCCQNPTQY | (SEQ ID NO. 61) |
| WISTTEALAYFHCCQNPTQY | (SEQ ID NO. 62) |
| WISITEALAYYHCCQNPTQY | (SEQ ID NO. 63) |
| WISTTEALAYYHCCQNPTQY | (SEQ ID NO. 64) |
| WTSTTEAQAYYHCCQNPTQY | |
| AFLGAKFKTSAQHALTSVSRGSSLKILSKGKRGGHSSVSTESESSSFHSS | (SEQ ID NO. 65; IC4). |

It is believed that it is clear from the above, that the sequences (SEQ ID NOs: 3, 12, 21, 30, 39, 48, 57 and 65) before, between and after each list of transmembrane domain variants are the N', intermediary and C' extracellular and intracellular regions, respectively.

The sequences above were then used to generate coding sequences, as is known in the art, suitable for expression in the expression system, in this case yeast. The coding sequences were then shuffled and expressed to produce a library comprising a plurality of proteins each having SEQ ID NOs: 3, 12, 21, 30, 39, 48, 57 and 65 with one transmembrane domain variant from each variant list in between the respective intracellular and extracellular domain.

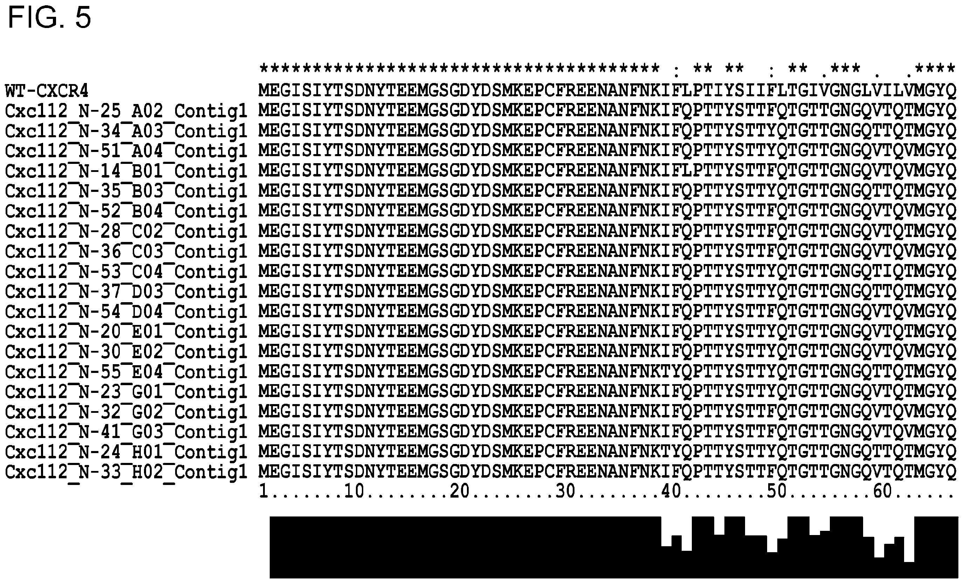

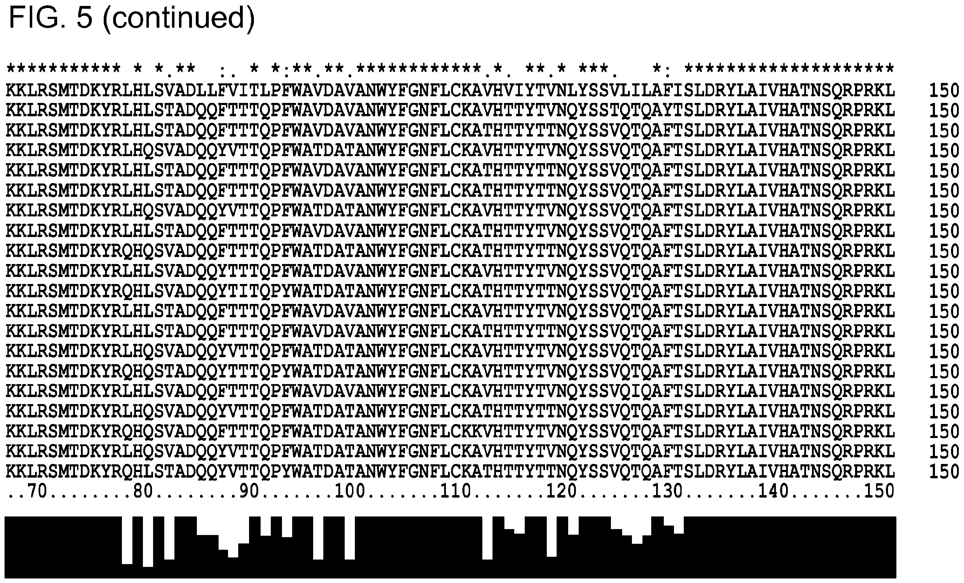

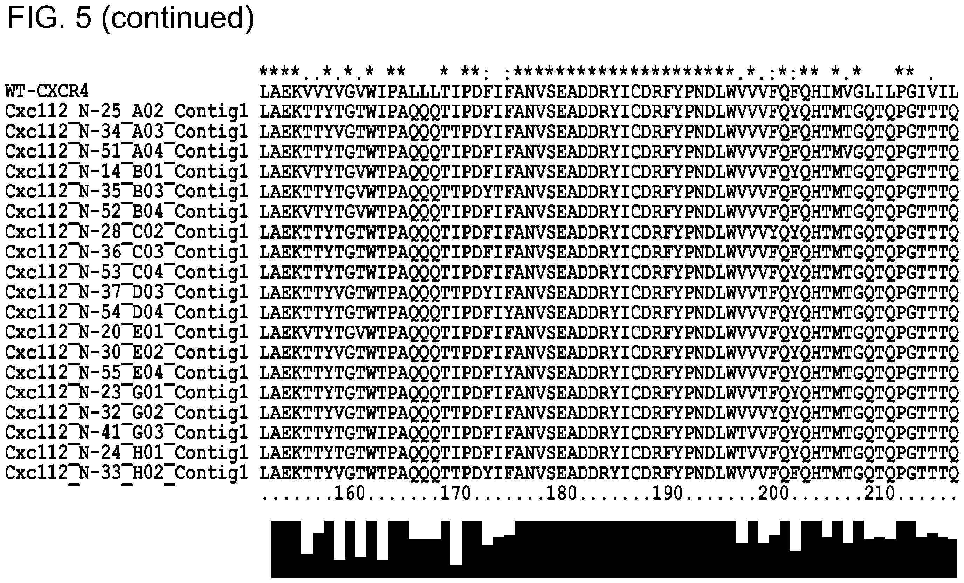

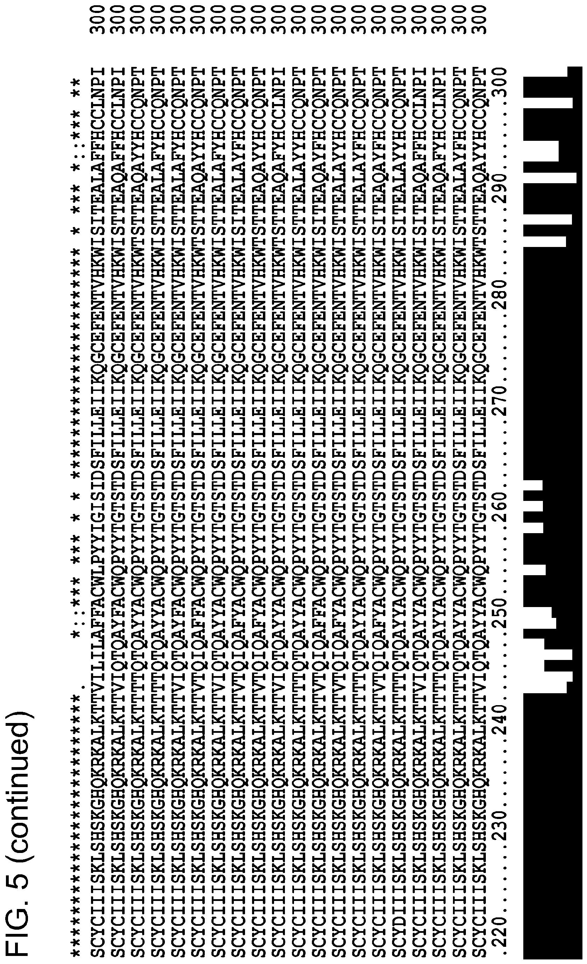

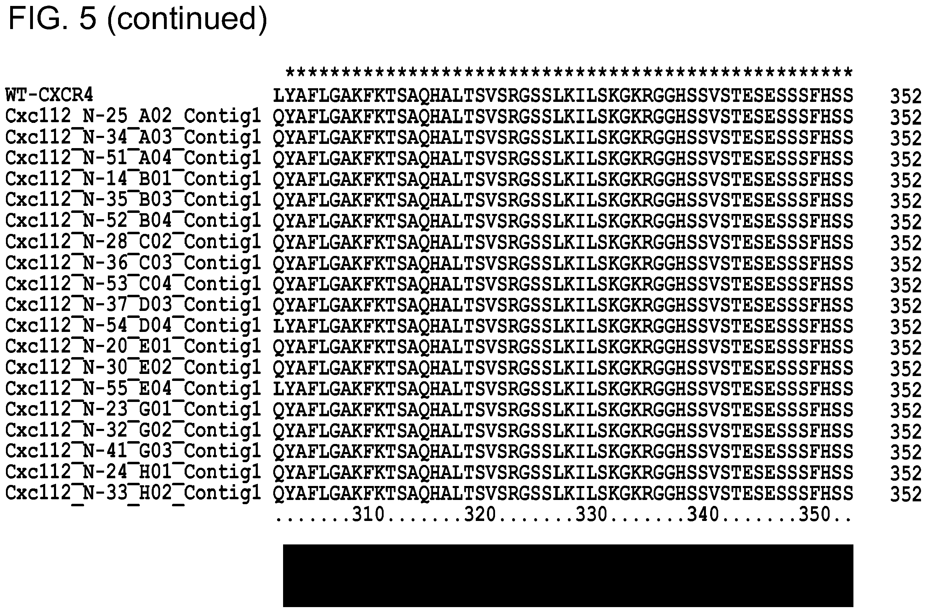

The library so produced was then assayed for CXCR4 cognate ligand, SDF1α (or CCL12) on a plasmid expressed in yeast binding inside living yeast cells. Ligand binding was detected by gene activation from the yeast 2-hybrid system and samples were then sequenced. Nineteen CXCR4 variants were sequenced. The results are shown in FIG. 5.

### Example 2: CXC chemokine receptor type 3 isoform b (CX3CR1):

CX3CR1 is a chemokine receptor 355 amino acids in length. It has a pI of about 6.74 and a Molecular Weight of 40396.4 Da. The subjecting of the sequence to TMHMM results in the identification of the transmembrane domains. Replacing all or substantially all of the hydrophobic amino acids, L, I V, and F, with Q, T and Y (respectively) within the transmembrane domains results in the following sequence (lower line), aligned with the wild type (top line):

MDQFPESVTENFEYDDLAEACYIGDIVVFGTVFLSIFYSVIFAIGLVGNLLVVFALTNSK
|||||||||||||||||||||||||||||||||*|**||***|*|**||*****|*||||
MDQFPESVTENFEYDDLAEACYIGDIVVFGTVFQSTYYSTTYATGQTGNQQTTYAQTNSK

KPKSVTDIYLLNLALSDLLFVATLPFWTHYLINEKGLHNAMCKFTTAFFFIGFFGSIFFI
|||||||*|**|*|*||****||*|*||||*||||||||||||||||****|**||****
KPKSVTDTYQQNQAQSDQQYTATQPYWTHYQINEKGLHNAMCKFTTAYYYTGYYGSTYYT

TVISIDRYLAIVLAANSMNNRTVQHGVTISLGVWAAAILVAAPQFMFTKQKENECLGDYP
|**|*|||||||||||||||||||||*|*|*|*||||***||||*|*|||||||||||||
TTTSTDRYLAIVLAANSMNNRTVQHGTTTSQGTWAAATQTAAPQYMYTKQKENECLGDYP

EVLQEIWPVLRNVETNFLGFLLPLLIMSYCYFRIIQTLFSCKNHKKAKAIKLILLVVIVF
||||||||||||||||**|***|***|||||*|**||**|||||||||||||*********
EVLQEIWPVLRNVETNYQGYQQPQQTMSYCYYRTTQTQYSCKNHKKAKAIKQTQQTTTTY

FLFWTPYNVMIFLETLKLYDFFPSCDMRKDLRLALSVTETVAFSHCCLNPLIYAFAGEKF
***|||||*|***|||||||||||||||||||||*|*|||*|*||||*||**||*|||||
YQYWTPYNTMTYQETLKLYDFFPSCDMRKDLRLAQSTTETTAYSHCCQNPQTYAYAGEKF

RRYLYHLYGKCLAVLCGRSVHVDFSSSESQRSRHGSVLSSNFTYHTSDGDALLLL (SEQ ID NO.66)
|||||||||||||||||||||||||||||||||||||||||||||||||||||||
RRYLYHLYGKCLAVLCGRSVHVDFSSSESQRSRHGSVLSSNFTYHTSDGDALLLL (SEQ ID NO.67).

The predicted pI of the protein variant is 6.74 and the Molecular Weight is 41027.17 Da. Each of the predicted transmembrane regions has been underlined and exemplified a fully modified domain of the invention. Thus, for example, the invention includes a transmembrane domain comprising the underlined Amino Acids of SEQ ID NO: 67. Preferably the protein comprising TM1 herein includes one or more (e.g., all) of the extracellular and intracellular loop sequences of SEQ ID NO: 66 (the sequences which have not been underlined). In addition or alternatively, the protein comprising the TM1 herein includes one or more additional transmembrane regions (the underlined sequences) in SEQ ID NO: 67 or homologous sequences retaining one, two, three or, possibly four or more of the native V, L, I and F amino acids, as set forth in SEQ ID NO: 66.

The native protein sequence for CX3CR1 was subjected to the method a second time. The program output divided the native sequence into the extracellular and intracellular regions and selected 8 transmembrane domain variants for each transmembrane domain. The results are illustrated in the following table:

| | |
|---|---|
| MDQFPESVTENFEYDDLAEACYIGDIVVFGT | (SEQ ID NO.68) |
| | |
| TM 1 Variants: | |
| TYQSTYYSTTFATGQVGNQQVVFALTNS | (SEQ ID NO.69) |
| TYQSTYYSTTYATGQVGNQQVVFALTNS | (SEQ ID NO.70) |
| TYQSTYYSTTYATGQVGNQQVVFAQTNS | (SEQ ID NO.71) |
| | |
| TYQSTYYSTTYATGQTGNLQVTFAQTNS | (SEQ ID NO.72) |
| TYQSTYYSTTYATGQTGNQLVTFAQTNS | (SEQ ID NO.73) |
| TYQSTYYSTTYATGQTGNQQVVFAQTNS | (SEQ ID NO.74) |
| TYQSTYYSTTYATGQTGNLQVTYAQTNS | (SEQ ID NO.75) |
| TYQSTYYSTTYATGQTGNQQTTYAQTNS | (SEQ ID NO.76) |
| | |
| KKPKSVTDIY | (SEQ ID NO.77) |
| | |

| TM 2 Variants | |
|---|---|
| LLNQAQSDQLFVATQPFWTHY | (SEQ ID NO.78) |
| LLNQAQSDQQFVATQPFWTHY | (SEQ ID NO.79) |
| QQNLAQSDQQFVATQPFWTHY | (SEQ ID NO.80) |
| LQNLAQSDQQYTATQPFWTHY | (SEQ ID NO.81) |
| QLNLAQSDQQYTATQPFWTHY | (SEQ ID NO.82) |
| LLNQAQSDQQFTATQPYWTHY | (SEQ ID NO.83) |
| QQNLAQSDQQFTATQPYWTHY | (SEQ ID NO.84) |
| QQNQAQSDQQYTATQPYWTHY | (SEQ ID NO.85) |
| | |
| LINEKGLHNAMCK | (SEQ ID NO.86) |
| | |

| TM3 Variant | |
|---|---|
| YTTAYYYTGYYGSTYYTTTTST | (SEQ ID NO.87) |
| | |
| DRYLAIVLAANSMNNRT | (SEQ ID NO.88) |
| | |

| TM4 Variants: | |
|---|---|
| VQHGTTTSQGTWAAATQVAAPQFMF | (SEQ ID NO.89) |
| VQHGVTTSQGTWAAATQTAAPQFMF | (SEQ ID NO.90) |
| VQHGTTTSQGVWAAATQTAAPQFMY | (SEQ ID NO.91) |
| VQHGTTTSQGTWAAAIQTAAPQFMY | (SEQ ID NO.92) |
| VQHGTTTSQGTWAAATQTAAPQFMF | (SEQ ID NO.93) |
| VQHGTTISQGTWAAATQTAAPQYMF | (SEQ ID NO.94) |
| VQHGTTTSQGTWAAATQTAAPQFMY | (SEQ ID NO.95) |
| TQHGTTTSQGTWAAATQTAAPQYMY | (SEQ ID NO.96) |
| | |
| TKQKENECLGDYPEVLQEIWPVLRNVET | (SEQ ID NO.97) |
| | |

| TM5 Variants: | |
|---|---|
| NFLGFQQPQQIMSYCYFRIT | (SEQ ID NO.98) |
| NFQGFLQPQQTMSYCYFRIT | (SEQ ID NO.99) |
| NFQGFLQPQQTMSYCYFRTT | (SEQ ID NO.100) |
| NFQGFQQPQQTMSYCYYRIT | (SEQ ID NO.101) |
| NFQGFLQPQQTMSYCYYRTT | (SEQ ID NO.102) |
| NFQGYLQPQQTMSYCYFRTT | (SEQ ID NO.103) |
| NYQGFQQPQQTMSYCYFRTT | (SEQ ID NO.104) |
| NYQGYQQPQQTMSYCYYRTT | (SEQ ID NO.105) |
| | |
| QTLFSCKNHKKAKAIK | (SEQ ID NO.106) |
| | |

| TM6 Variants: | |
|---|---|
| LIQQTTTTFYQFWTPYNTMTFQETL | (SEQ ID NO.107) |
| LIQQTTTTFYQYWTPYNVMTFQETQ | (SEQ ID NO.108) |
| LIQQTTTTYYQFWTPYNTMTFQETQ | (SEQ ID NO.109) |
| QIQQTTTTFYQYWTPYNTMTFQETQ | (SEQ ID NO.110) |
| LTQQTTTTYYQFWTPYNTMTFQETQ | (SEQ ID NO.111) |
| QIQQTTTTFFQYWTPYNTMTYQETQ | (SEQ ID NO.112) |
| QIQQTTTTFYQYWTPYNTMTYQETQ | (SEQ ID NO.113) |
| QTQQTTTTYYQYWTPYNTMTYQETQ | (SEQ ID NO.114) |
| | |
| KLYDFFPSCDMRKDLRL (SEQ ID NO.115) | |
| | |

| TM7 Variants: | |
|---|---|
| ALSVTETVAFSHCCQNPQIYAFAG | (SEQ ID NO.116) |
| AQSVTETTAFSHCCQNPLIYAFAG | (SEQ ID NO.117) |
| ALSVTETVAFSHCCQNPQTYAYAG | (SEQ ID NO.118) |
| AQSVTETTAFSHCCQNPQIYAYAG | (SEQ ID NO.119) |
| ALSVTETTAFSHCCQNPQTYAYAG | (SEQ ID NO.120) |
| ALSTTETTAYSHCCQNPQIYAFAG | (SEQ ID NO.121) |
| ALSVTETTAYSHCCQNPQTYAYAG | (SEQ ID NO.122) |
| AQSTTETTAYSHCCQNPQTYAYAG | (SEQ ID NO.123) |
| EKFRRYLYHLYGKCLAVLCGRSVHVDFSSSESQRSRHGSVLSSNFTYHTSDGDALLLL | (SEQ ID NO.124). |

As in Example 1 above, that the sequences before, between and after each list of transmembrane domain variants are the N', intermediary and C' intra or extracellular regions, respectively.

The sequences above were then used to generate coding sequences, as is known in the art, suitable for expression in the expression system, in this case yeast. The coding sequences were then shuffled and expressed to produce a library comprising a plurality of proteins each having SEQ ID NOs: 68, 77, 86, 88, 97, 106, and 115 with one transmembrane domain variant from each variant list in between the respective intracellular and extracellular domain.

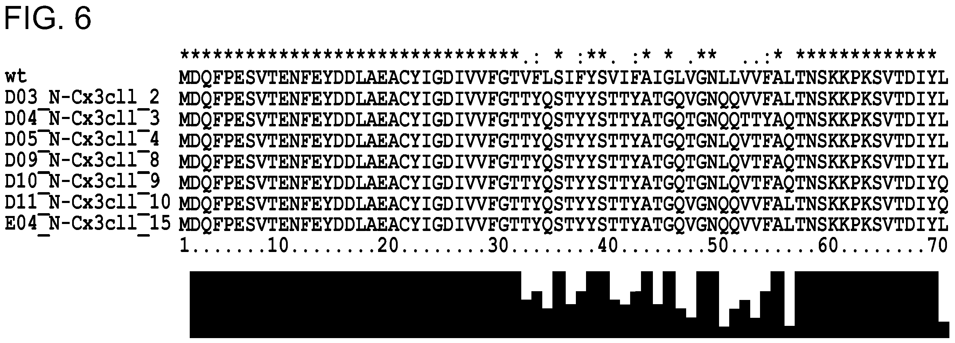

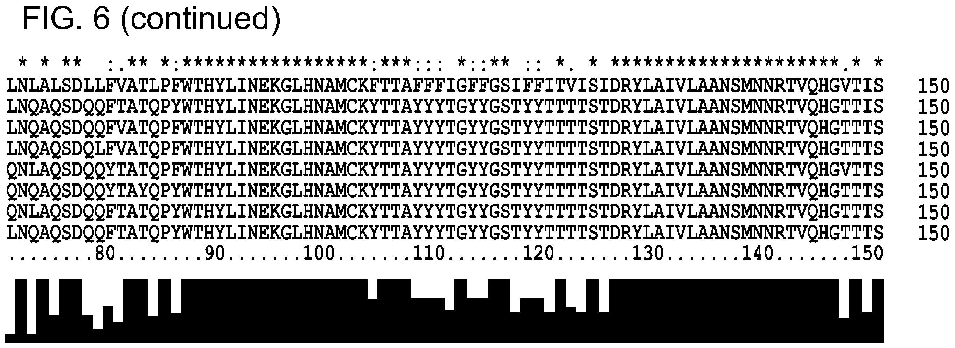

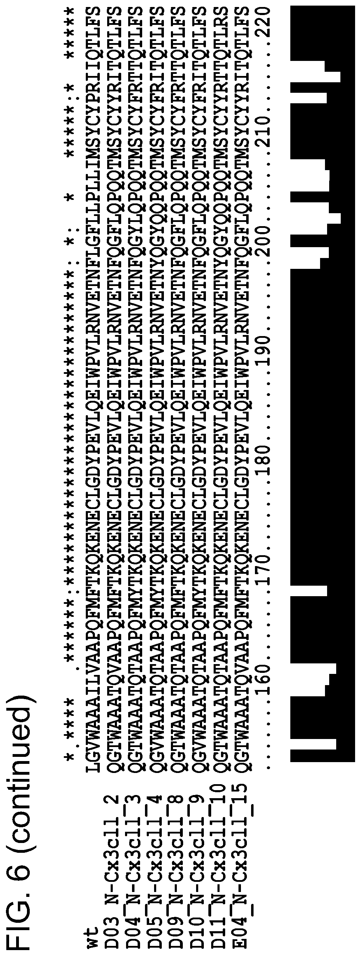

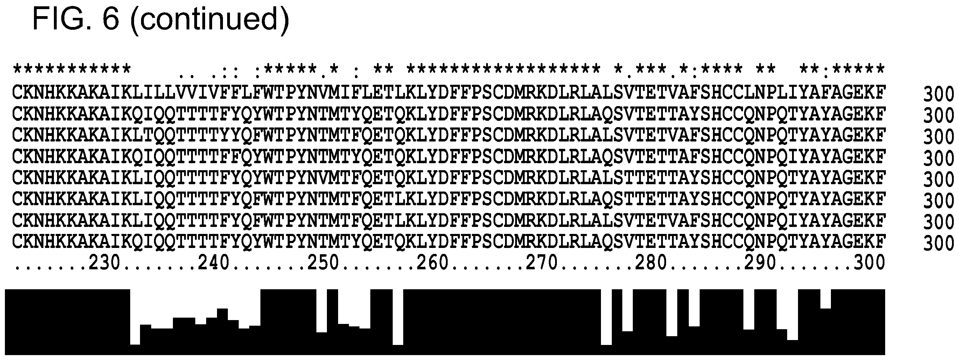

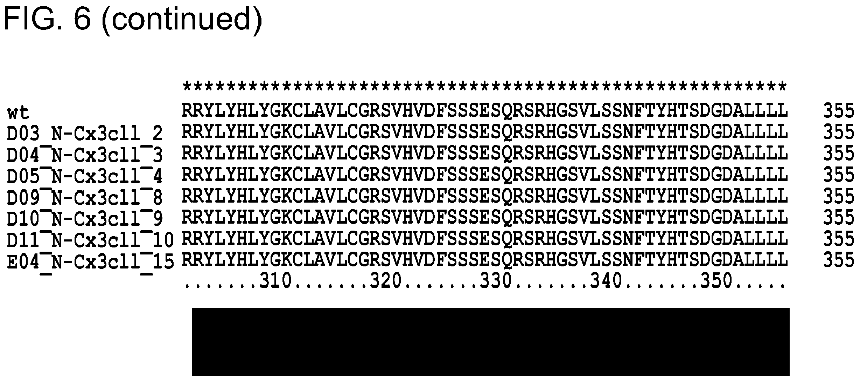

The library so produced was then assayed for CX3CR1 cognate ligand (CXCL1) binding in an aqueous medium, as described in Example 1. Ligand binding was detected and samples were then sequenced. Seven variants were sequenced. The results are shown in FIG. 6.

### Example 3: CCR3 Variants

The method of Example 1 was repeated for Chemokine Receptor Type 3 isoform 3.

| Name | pI | MW (Da) |
|---|---|---|
| WT | 8.87 | 43122.3 |
| MT | 8.78 | 43531.64 |

Replacing all or substantially all of the hydrophobic amino acids, L, I V, and F, with Q, T and Y (respectively) within the transmembrane domains results in the following sequence (lower line), aligned with the wild type (top line):

MPFGIRMLLRAHKPGRSEMTTSLDTVETFGTTSYYDDVGLLCEKADTRALMAQFVPPLYS
|||||||||||||||||||||||||||||||||||||||||||||||||||||||||*||
MPFGIRMLLRAHKPGRSEMTTSLDTVETFGTTSYYDDVGLLCEKADTRALMAQFVPPQYS

LVFTVGLLGNVVVVMILIKYRRLRIMTNIYLLNLAISDLLFLVTLPFWIHYVRGHNWVFG
***|*|**||****|***||||||||||*|**|*|*||*****|*|*|*|||||||||||
QTYTTGQQGNTTTTMTQTKYRRLRIMTNTYQQNQATSDQQYQTTQPYWTHYVRGHNWVFG

HGMCKLLSGFYHTGLYSEIFFIILLTIDRYLAIVHAVFALRARTVTFGVITSIVTWGLAV
||||||||||||||||||*******|*|||*|**||**|*||||||||**||**|||*|*
HGMCKLLSGFYHTGLYSETYYTTQQTTDRYQATTHATYAQRARTVTFGTTTSTTTWGQAT

LAALPEFIFYETEELFEETLCSALYPEDTVYSWRHFHTLRMTIFCLVLPLLVMAICYTGI
*||*||***||||||||||||||||||||||||||||||||||**|***|***||*||||*
QAAQPEYTYYETEELFEETLCSALYPEDTVYSWRHFHTLRMTTYCQTQPQQTMATCYTGT

IKTLLRCPSKKKYKAIRLIFVIMAVFFIFWTPYNVAILLSSYQSILFGNDCERSKHLDLV
*||||||||||||||||*****||*****|||||*|***|||||||||||||||||||**
TKTLLRCPSKKKYKAIRQTYTTMATYYTYWTPYNTATQQSSYQSILFGNDCERSKHLDQT

MLVTEVIAYSHCCMNPVIYAFVGERFRKYLRHFFHRHLLMHLGRYIPFLPSEKLERTSSV
|**||**|||||||||**||**||||||||||||||||||||||||||||||||||||||
MQTTETTAYSHCCMNPTTYAYTGERFRKYLRHFFHRHLLMHLGRYIPFLPSEKLERTSSV

SPSTAEPELSIVF (SEQ ID NO. 125)
|||||||||||||
SPSTAEPELSIVF (SEQ ID NO.
126)

Each of the predicted transmembrane regions have been underlined and exemplify a fully modified domain of the invention. Thus, for example, the invention includes a transmembrane domain comprising the underlined Amino Acids of SEQ ID NO: 126. Preferably the protein comprising TM1 herein includes one or more (e.g., all) of the extracellular and intracellular loop sequences of SEQ ID NO: 126 (the sequences which have not been underlined). In addition or alternatively, the protein comprising the TM1 herein includes one or more additional transmembrane regions (the underlined sequences) in SEQ ID NO: 126 or homologous sequences retaining one, two, three or, possibly four or more of the native V, L I and F amino acids, as set forth in SEQ ID NO: 125.

The native protein sequence for CCR3 was subjected to the method a second time (noting a difference in the N terminal sequence). The program output divided the native sequence into the extracellular and intracellular regions and selected 8 transmembrane domain variants for each transmembrane domain. The results are illustrated in the following table:

| | |
|---|---|
| MTTSLDTVETFGTTSYYDDVGLLCEKADTRALMA | (SEQ ID NO.127) |
| | |

| TM1 Variants: | |
|---|---|
| QFVPPQYSQTFTTGQQGNVTVTMTQIKY | (SEQ ID NO.128) |
| QFVPPQYSQTFTTGQQGNTTVTMTQIKY | (SEQ ID NO.129) |
| QFVPPQYSQTYTTGQQGNTTVTMTQIKY | (SEQ ID NO.130) |
| QFTPPQYSQTYTTGQQGNVTTTMTQIKY | (SEQ ID NO.131) |
| QFTPPQYSQTYTTGQQGNTVTTMTQIKY | (SEQ ID NO.132) |
| QFTPPQYSQTYTTGQQGNTTVTMTQIKY | (SEQ ID NO.133) |
| QFTPPQYSQTYTTGQQGNTTTTMTQIKY | (SEQ ID NO.134) |
| QYTPPQYSQTYTTGQQGNTTTTMTQTKY | (SEQ ID NO.135) |
| | |
| RRLRIMTNIY | (SEQ ID NO.136) |
| | |

| TM2 Variants: | |
|---|---|
| LLNQATSDQQFQVTQPFWIHY | (SEQ ID NO.137) |
| LQNQAISDQLFQTTQPFWTHY | (SEQ ID NO.138) |
| QQNLAISDQQFQTTQPFWTHY | (SEQ ID NO.139) |
| QLNQAISDQQFQTTQPYWTHY | (SEQ ID NO.140) |
| QQNLAISDQQYQVTQPYWTHY | (SEQ ID NO.141) |
| LQNQATSDQLFQTTQPYWTHY | (SEQ ID NO.142) |
| QQNQAISDQQYQVTQPYWTHY | (SEQ ID NO.143) |
| QQNQATSDQQYQTTQPYWTHY | (SEQ ID NO.144) |
| | |
| VRGHNWVFGHGMCK | (SEQ ID NO.145) |
| | |

| TM3 Variants: | |
|---|---|
| LQSGFYHTGQYSETFFTTQQTT | (SEQ ID NO.146) |
| QLSGFYHTGQYSETFFTTQQTT | (SEQ ID NO.147) |
| QLSGFYHTGQYSETFYTTQQTT | (SEQ ID NO.148) |
| QLSGFYHTGQYSETYFTTQQTT | (SEQ ID NO.149) |
| QLSGYYHTGQYSETFFTTQQTT | (SEQ ID NO.150) |
| QQSGFYHTGQYSETFFTTQQTT | (SEQ ID NO.151) |
| QQSGFYHTGQYSETFYTTQQTT | (SEQ ID NO.152) |
| QQSGYYHTGQYSETYYTTQQTT | (SEQ ID NO.153) |
| | |
| DRYLAIVHAVFALRART | (SEQ ID NO.154) |
| | |

| TM4 Variants: | |
|---|---|
| TTFGTTTSTVTWGQAVQAAQPEFIF | (SEQ ID NO.155) |
| TTFGTTTSTTTWGQAVQAAQPEFIF | (SEQ ID NO.156) |
| TTYGTTTSTTTWGQAVQAAQPEFIF | (SEQ ID NO.157) |
| TTYGTTTSTTTWGQAVQAAQPEFTF | (SEQ ID NO.158) |
| TTYGTTTSTTTWGQATQAAQPEFIF | (SEQ ID NO.159) |
| TTFGTTTSTTTWGQATQAAQPEFIY | (SEQ ID NO.160) |
| TTYGTTTSTTTWGQATQAAQPEFIY | (SEQ ID NO.161) |
| TTYGTTTSTTTWGQATQAAQPEYTY | (SEQ ID NO.162) |
| | |
| YETEELFEETLCSALYPEDTVYSWRHFHTLRM | (SEQ ID NO.163) |
| | |

| TM5 Variants: | |
|---|---|
| TIFCQVQPQQTMATCYTGTT | (SEQ ID NO.164) |
| TIFCQTQPQQVMATCYTGTT | (SEQ ID NO.165) |
| TIFCQTQPQQTMATCYTGIT | (SEQ ID NO.166) |
| TIFCQTQPQQTMATCYTGTI | (SEQ ID NO.167) |
| TTFCQVQPQQVMATCYTGTT | (SEQ ID NO.168) |
| TIYCQVQPQQVMATCYTGTT | (SEQ ID NO.169) |
| TIFCQTQPQQTMATCYTGTT | (SEQ ID NO.170) |
| TTYCQTQPQQTMATCYTGTT | (SEQ ID NO.171) |
| | |
| KTLLRCPSKKKYKAIR | (SEQ ID NO.172) |
| | |

| TM 6 Variant: | |
|---|---|
| QTYTTMATYYTYWTPYNTATQQSSY | (SEQ ID NO.173) |
| | |
| QSILFGNDCERSKHLDL | (SEQ ID NO.174) |
| | |

| TM7 Variants: | |
|---|---|
| VMQVTEVTAYSHCCMNPVTYAFTG | (SEQ ID NO.175) |
| VMQVTEVTAYSHCCMNPTTYAYVG | (SEQ ID NO.176) |
| VMLTTEVTAYSHCCMNPTTYAFTG | (SEQ ID NO.177) |
| VMQVTETTAYSHCCMNPVTYAYTG | (SEQ ID NO.178) |
| TMQVTETIAYSHCCMNPTTYAFTG | (SEQ ID NO.179) |
| TMQVTETTAYSHCCMNPTTYAFVG | (SEQ ID NO.180) |
| VMQTTETIAYSHCCMNPTTYAYTG | (SEQ ID NO.181) |
| TMQTTETTAYSHCCMNPTTYAYTG | (SEQ ID NO.182) |
| | |
| ERFRKYLRHFFHRHLLMHLGRYIPFLPSEKLERTSSVSPSTAEPELSIVF | (SEQ ID NO:183). |

As in Example 1 above, the sequences before, between and after each list of transmembrane domain variants are the N', intermediary and C' intra or extracellular regions, respectively.

The sequences above were then used to generate coding sequences, as is known in the art, suitable for expression in the expression system, in this case yeast. The coding sequences were then shuffled and expressed to produce a library comprising a plurality of proteins each having SEQ ID NOs: 127, 136, 145, 154, 163, 172, 174 and 183 with one transmembrane domain variant from each variant list in between the respective intracellular and extracellular domain.

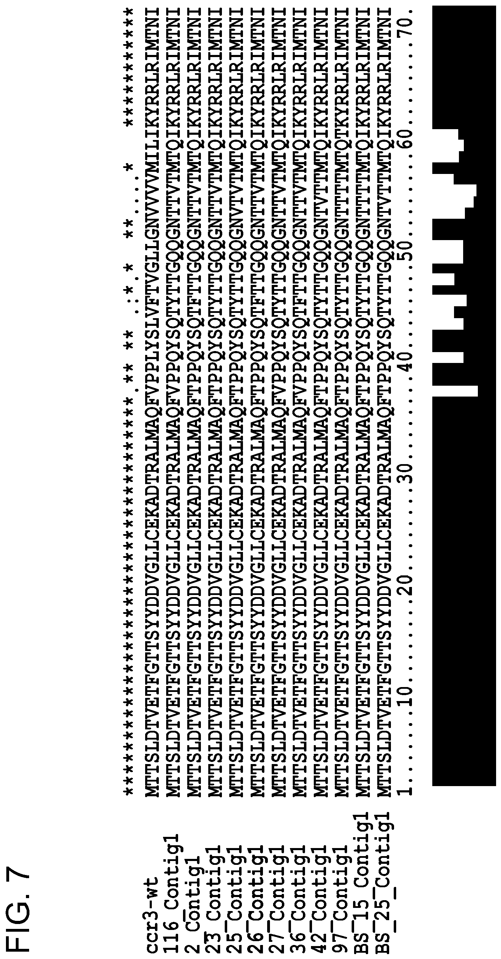

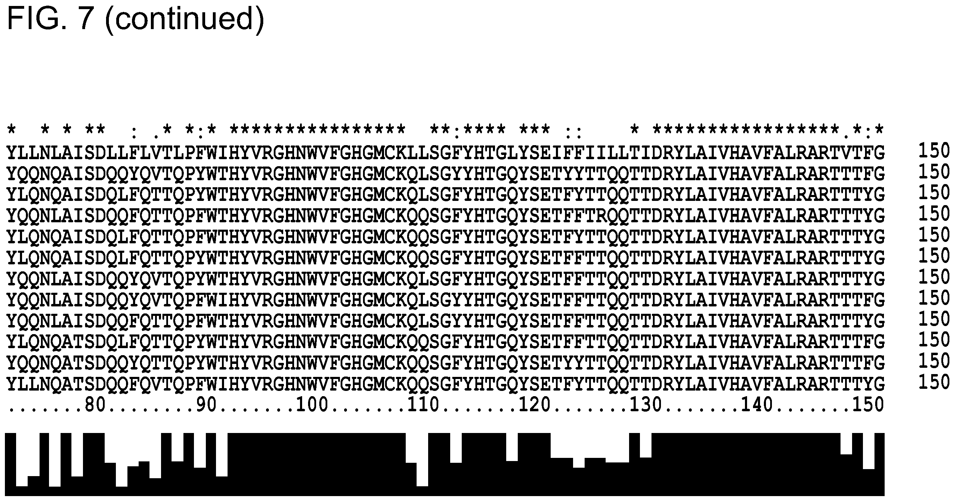

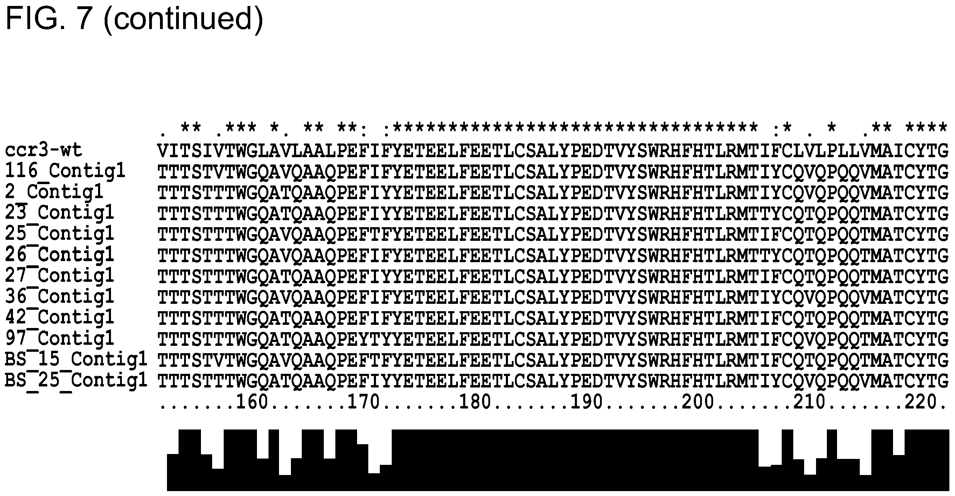

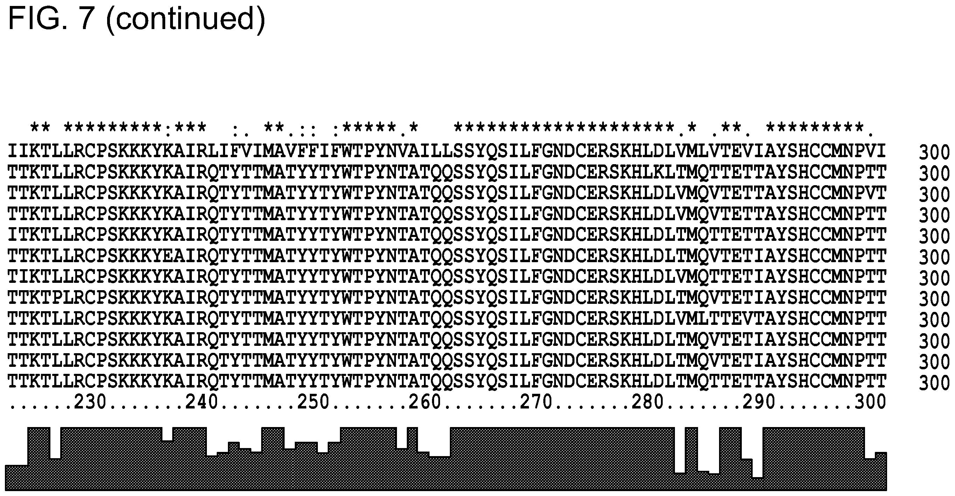

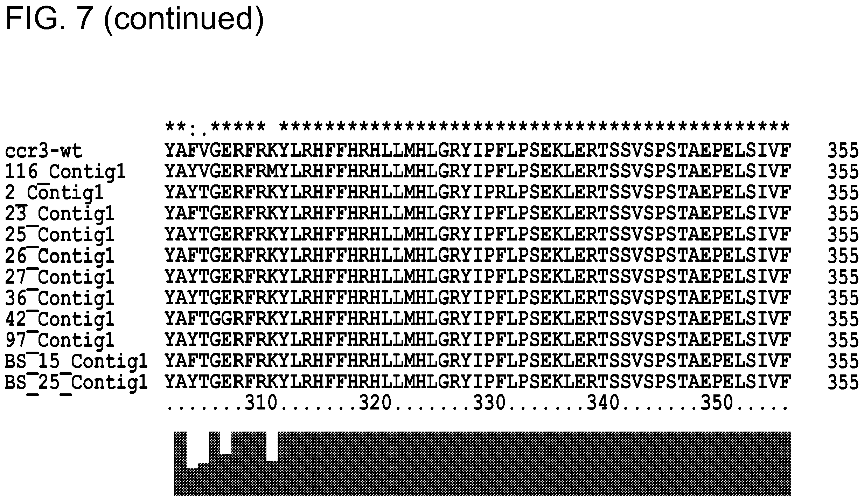

The library so produced was then assayed for CCR3 cognate ligand, CCL3, binding in an aqueous medium, as described in Example 1. Ligand binding was detected and samples were then sequenced. Eleven variants were sequenced. The results are shown in FIG 7.

### Example 4: CCR5 Variants

The method of Example 1 was repeated for Chemokine Receptor Type 5 isoform 3.

| Name | pI | MW (Da) |
|---|---|---|
| WT | 9.21 | 40524.05 |
| MT | 9.06 | 41058.3 |

Replacing all or substantially all of the hydrophobic amino acids, L, I V, and F, with Q, T and Y (respectively) within the transmembrane domains results in the following sequence (lower line), aligned with the wild type (top line):

MDYQVSSPIYDINYYTSEPCQKINVKQIAARLLPPLYSLVFIFGFVGNMLVILILINCKR
|||||||||||||||||||||||||||||||||||*||*****|**|||*******||||
MDYQVSSPIYDINYYTSEPCQKINVKQIAARLLPPQYSQTYTYGYTGNMQTTQTQTNCKR

LKSMTDIYLLNLAISDLFFLLTVPFWAHYAAAQWDFGNTMCQLLTGLYFIGFFSGIFFII
||||||*|**|*|*||*****|*|*|||||||||||||||||**||*|**|**||*****
LKSMTDTYQQNQATSDQYYQQTTPYWAHYAAAQWDFGNTMCQQQTGQYYTGYYSGTYYTT

LLTIDRYLAVVHAVFALKARTVTFGVVTSVITWVVAVFASLPGIIFTRSQKEGLHYTCSS
**|*|||||||||||||||||||*|**||**||**|**||*||***|||||||||||||||
QQTTDRYLAVVHAVFALKARTVTYGTTTSTTTWTTATYASQPGTTYTRSQKEGLHYTCSS

HFPYSQYQFWKNFQTLKIVILGLVLPLLVMVICYSGILKTLLRCRNEKKRHRAVRLIFTI
|||||||||||||||||****|***|***|**||||**||*|||||||||||||||***|*
HFPYSQYQFWKNFQTLKTTTQGQTQPQQTMTTCYSGTQKTQLRCRNEKKRHRAVRQTYTT

MIVYFLFWAPYNIVLLLNTFQEFFGLNNCSSSNRLDQAMQVTETLGMTHCCINPIIYAFV
|**|***|||||*****||||||||||||||||||||||||||||||||||*||**||**
MTTYYQYWAPYNTTQQQNTFQEFFGLNNCSSSNRLDQAMQVTETLGMTHCCTNPTTYAYT

GEKFRNYLLVFFQKHIAKRFCKCCSIFQQEAPERASSVYTRSTGEQEISVGL (SEQ ID NO. 184)
|||*|||*****||||||||||||||||||||||||||||||||||||||||
GEKYRNYQQTYYQKHIAKRFCKCCSIFQQEAPERASSVYTRSTGEQEISVGL (SEQ ID NO. 185).

Each of the predicted transmembrane regions has been underlined and exemplified a fully modified domain of the invention. Thus, for example, the invention includes a transmembrane domain comprising the underlined Amino Acids of SEQ ID NO: 185. Preferably the protein comprising TM1 herein includes one or more (e.g., all) of the extracellular and intracellular loop sequences of SEQ ID NO: 185 (the sequences which have not been underlined). In addition or alternatively, the protein comprising the TM1 herein includes one or more additional transmembrane regions (the underlined sequences) in SEQ ID NO: 185 or homologous sequences retaining one, two, three or, possibly four or more of the native V, L I and F amino acids, as set forth in SEQ ID NO: 184.

The native protein sequence for CCR5 was subjected to the method a second time (noting a difference in the N terminal sequence). The program output divided the native sequence into the extracellular and intracellular regions and selected 8 transmembrane domain variants for each transmembrane domain. The results are illustrated in the following table:

| | |
|---|---|
| MDYQVSSPIYDINYYTSEPCQKINVKQIAA | (SEQ ID NO.186) |
| | |

| TM1 Variants: | |
|---|---|
| RLQPPQYSQTFTFGFTGNMQVTQTQINC | (SEQ ID NO.187) |
| RLQPPQYSQTFTFGYTGNMQVTQTQINC | (SEQ ID NO.188) |
| RQQPPQYSQTFTFGFTGNMQTTQTQINC | (SEQ ID NO.189) |
| RQQPPQYSQTFTYGFTGNMQTTQTQINC | (SEQ ID NO.190) |
| RQQPPQYSQTYTFGFTGNMQTTQTQINC | (SEQ ID NO.191) |
| RQQPPQYSQTFTFGYTGNMQTTQTQINC | (SEQ ID NO.192) |
| RQQPPQYSQTYTFGYTGNMQTTQTQINC | (SEQ ID NO.193) |
| RQQPPQYSQTYTYGYTGNMQTTQTQTNC | (SEQ ID NO.194) |
| KRLKSMTDIY | (SEQ ID NO.195) |
| | |

| TM2 Variants: | |
|---|---|
| LQNQAISDQFFQQTVPFWAHY | (SEQ ID NO.196) |
| LQNQAISDQFFQQTTPFWAHY | (SEQ ID NO.197) |
| LQNQAISDQFFQQTTPYWAHY | (SEQ ID NO.198) |
| LQNQAISDQFYQQTTPYWAHY | (SEQ ID NO.199) |
| LQNQAISDQYFQQTTPYWAHY | (SEQ ID NO.200) |
| LQNQATSDQFFQQTTPYWAHY | (SEQ ID NO.201) |
| LQNQAISDQYYQQTTPYWAHY | (SEQ ID NO.202) |
| QQNQATSDQYYQQTTPYWAHY | (SEQ ID NO.203) |
| | |
| AAAQWDFGNTMCQ | (SEQ ID NO.204) |
| | |

| TM3 Variants: | |
|---|---|
| QQTGQYFTGYYSGTYYTTQQTT | (SEQ ID NO.205) |
| QQTGQYYTGYYSGTYYTTQQTT | (SEQ ID NO.206) |
| | |
| DRYLAVVHAVFALKART | (SEQ ID NO.207) |
| | |

| TM4 Variant: | |
|---|---|
| TTYGTTTSTTTWTTATYASQPGTTY | (SEQ ID NO.208) |
| | |
| TRSQKEGLHYTCSSHFPYSQYQFWKNFQTLKI | (SEQ ID NO.209) |
| | |

| TM5 Variants: | |
|---|---|
| VIQGQVQPQQVMVTCYSGIQ | (SEQ ID NO.210) |
| VIQGQVQPQQVMTTCYSGIQ | (SEQ ID NO.211) |
| VIQGQVQPQQTMTTCYSGIQ | (SEQ ID NO.212) |
| VTQGQVQPQQTMVTCYSGTQ | (SEQ ID NO.213) |
| TIQGQVQPQQVMTTCYSGTQ | (SEQ ID NO.214) |
| TIQGQVQPQQTMVTCYSGTQ | (SEQ ID NO.215) |
| TTQGQVQPQQVMTTCYSGTQ | (SEQ ID NO.216) |
| TTQGQTQPQQTMTTCYSGTQ | (SEQ ID NO.217) |
| | |
| KTLLRCRNEKKRHRAVR | (SEQ ID NO.218) |
| | |

| TM6 Variants: | |
|---|---|
| QTFTTMTTYYQFWAPYNIVQQLNTF | (SEQ ID NO.219) |
| QTFTTMTTYYQFWAPYNTVQQLNTF | (SEQ ID NO.220) |
| QTFTTMTTYYQYWAPYNTVQQLNTF | (SEQ ID NO.221) |
| QTFTTMTTYYQYWAPYNTVQQQNTF | (SEQ ID NO.222) |
| QTYTTMTTYYQYWAPYNTVQQLNTF | (SEQ ID NO.223) |
| QTFTTMTTYYQYWAPYNTTQQLNTF | (SEQ ID NO.224) |
| QTYTTMTTYYQYWAPYNTVQQQNTF | (SEQ ID NO.225) |
| QTYTTMTTYYQYWAPYNTTQQQNTY | (SEQ ID NO.225) |
| | |
| QEFFGLNNCSSSNRLDQ | (SEQ ID NO.226) |
| | |

| TM7 Variants: | |
|---|---|
| AMQVTETQGMTHCCINPIIYAFVG | (SEQ ID NO.227) |
| AMQVTETLGMTHCCTNPIIYAFTG | (SEQ ID NO.228) |
| AMQVTETQGMTHCCINPTIYAYVG | (SEQ ID NO.229) |
| AMQTTETQGMTHCCINPITYAFTG | (SEQ ID NO.230) |
| AMQTTETQGMTHCCINPTIYAFTG | (SEQ ID NO.231) |
| AMQVTETQGMTHCCTNPTIYAYVG | (SEQ ID NO.232) |
| AMQTTETQGMTHCCINPTTYAYVG | (SEQ ID NO.233) |
| AMQTTETQGMTHCCTNPTTYAYTG | (SEQ ID NO.234) |
| | |
| EKFRNYLLVFFQKHIAKRFCKCCSIFQQEAPERASSVYTRSTGEQEISVGL | (SEQ ID NO.235). |

As in Example 1 above, the sequences before, between and after each list of transmembrane domain variants are the N', intermediary and C' intra or extracellular regions, respectively.

The sequences above were then used to generate coding sequences, as is known in the art, suitable for expression in the expression system, in this case yeast. The coding sequences were then shuffled and expressed to produce a library comprising a plurality of proteins each having SEQ ID NO:s, 186, 195, 204, 207, 209, 218, 226, and 235 with one transmembrane domain variant from each variant list in between the respective intracellular and extracellular domain.

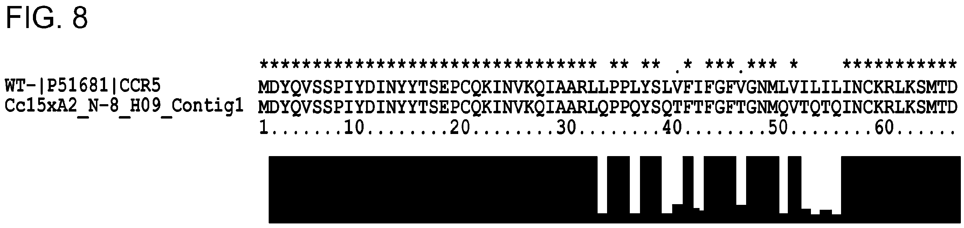

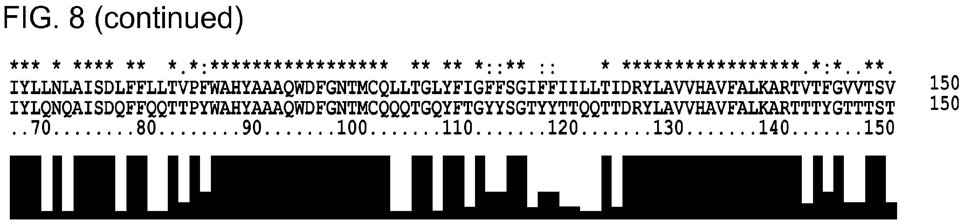

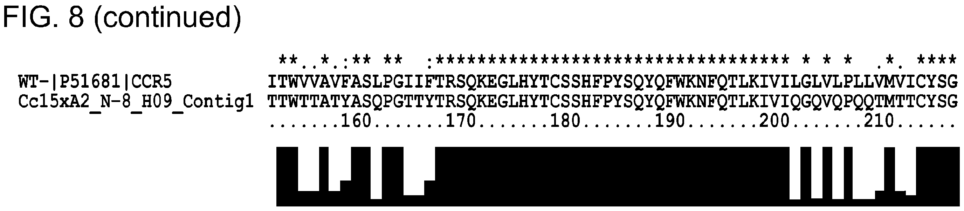

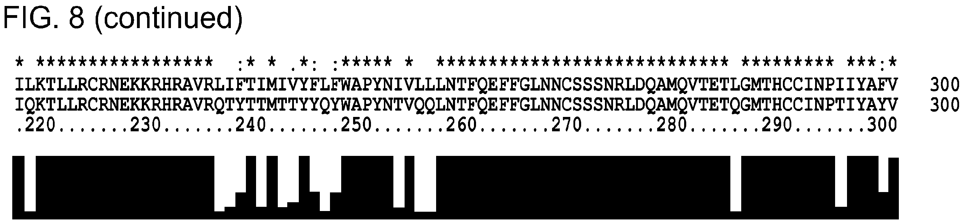

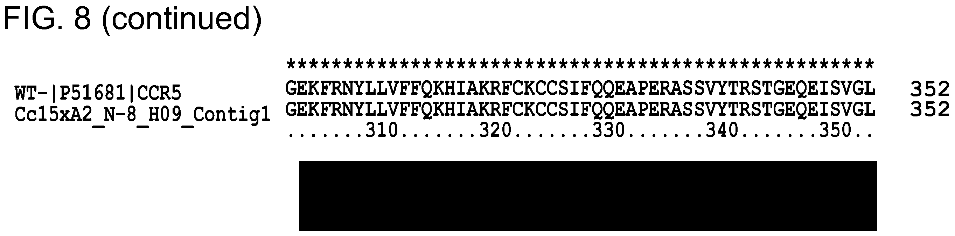

The library so produced was then assayed for CCR5 cognate ligand, CCL5, binding in an aqueous medium, as described in Example 1. Ligand binding was detected and samples were then sequenced. One variant was sequenced. The results are shown in FIG. 8.

### Example 5: CXCR3 Variants

The method of Example 1 was repeated for the CXC chemokine receptor type 3 isoform 2. Replacing all or substantially all of the hydrophobic amino acids, L, I V, and F, with Q, T and Y (respectively) within the transmembrane domains results in the following sequence (SEQ ID NO: 325, lower line), aligned with the wild type (SEQ ID NO: 324, top line):

MELRKYGPGRLAGTVIGGAAQSKSQTKSDSITKEFLPGLYTAPSSPFPPSQVSDHQVLND
||||||||||||||||||||||||||||||||||||||||||||||||||||||||||||
MELRKYGPGRLAGTVIGGAAQSKSQTKSDSITKEFLPGLYTAPSSPFPPSQVSDHQVLND

AEVAALLENFSSSYDYGENESDSCCTSPPCPQDFSLNFDRAFLPALYSLLFLLGLLGNGA
|||||||||||||||||||||||||||||||||||||||||||||*||*****|**||||
AEVAALLENFSSSYDYGENESDSCCTSPPCPQDFSLNFDRAFLPAQYSQQYQQGQQGNGA

VAAVLLSRRTALSSTDTFLLHLAVADTLLVLTLPLWAVDAAVQWVFGSGLCKVAGALFNI
*||***|||||||||||***|*|*|||****|*|*||*||||||||||||||||||**|*
TAATQQSRRTALSSTDTYQQHQATADTQQTQTQPQWATDAAVQWVFGSGLCKVAGAQYNT

NFYAGALLLACISFDRYLNIVHATQLYRRGPPARVTLTCLAVWGLCLLFALPDFIFLSAH
|*||||***||*|*||||||||||||||||||||*|*||*|*||*|***|*||****|||
NYYAGAQQQACTSYDRYLNIVHATQLYRRGPPARTTQTCQATWGQCQQYAQPDYTYQSAH

HDERLNATHCQYNFPQVGRTALRVLQLVAGFLLPLLVMAYCYAHILAVLLVSRGQRRLRA
|||||||||||||||||||||||||||*||***|***|||||||**|***||||||||||
HDERLNATHCQYNFPQVGRTALRVLQLTAGYQQPQQTMAYCYAHTQATQQVSRGQRRLRA

MRLVVVVVVAFALCWTPYHLVVLVDILMDLGALARNCGRESRVDVAKSVTSGLGYMHCCL
||*******|*|*||||||***||||||||||||||||||||||||||||||*||||||*
MRQTTTTTTAYAQCWTPYHQTTLVDILMDLGALARNCGRESRVDVAKSVTSGQGYMHCCQ

NPLLYAFVGVKFRERMWMLLLRLGCPNQRGLQRQPSSSRRDSSWSETSEASYSGL
||**||**|*|||||||||||||||||||||||||||||||||||||||||||||
NPQQYAYTGTKFRERMWMLLLRLGCPNQRGLQRQPSSSRRDSSWSETSEASYSGL

Each of the predicted transmembrane regions has been underlined and exemplified a fully modified domain of the invention. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in SEQ ID NO: 325 or homologous sequences retaining one, two, three or, possibly four or more of the native V, L I and F amino acids, as set forth in SEQ ID NO: 324.

As discussed above, the native protein sequence for CXCR3 was subjected to the method. The program output divided the native sequence into the extracellular and intracellular regions and selected 8 transmembrane domain variants for each transmembrane domain. The results are illustrated in the following table:

| | |
|---|---|
| MVLEVSDHQVLNDAEVAALLENFSSSYDYGENESDSCCTSPPCPQDFSLNFDR | (SEQ ID NO. 235) |
| | |

| TM 1 Variants: | |
|---|---|
| AFLPALYSQQFQQGQQGNGAVAATQLS | (SEQ ID NO.236) |
| AFQPALYSQQFQQGQQGNGAVAAVQQS | (SEQ ID NO.237) |
| AFQPAQYSQQFLQGQQGNGAVAATQQS | (SEQ ID NO.238) |
| AYQPALYSLQYQQGQQGNGATAAVQQS | (SEQ ID NO.239) |
| AYQPALYSQLFQQGQQGNGATAATQQS | (SEQ ID NO.240) |
| AFQPALYSLQYQQGQQGNGATAATQQS | (SEQ ID NO.241) |
| AYQPAQYSLQYQQGQQGNGATAAVQQS | (SEQ ID NO.242) |
| AYQPAQYSQQYQQGQQGNGATAATQQS | (SEQ ID NO.243) |
| | |
| RRTALSSTD | (SEQ ID NO.244) |
| | |

| TM 2 Variants: | |
|---|---|
| TFLQHLAVADTQQVQTLPQWA | (SEQ ID NO.245) |
| TFLQHQAVADTQLVQTQPQWA | (SEQ ID NO.:246) |
| TFQQHLAVADTQQVQTQPQWA | (SEQ ID NO.:247) |
| TYLQHQAVADTQQVQTQPQWA | (SEQ ID NO.:248) |
| TYQLHQAVADTQQVQTQPQWA | (SEQ ID NO.:249) |
| TYQQHLAVADTQQVQTQPQWA | (SEQ ID NO.:250) |
| TYQQHQAVADTQQVQTQPQWA | (SEQ ID NO.:251) |
| TYQQHQATADTQQTQTQPQWA | (SEQ ID NO.:252) |
| | |
| VDAAVQWVFGSGLCK | (SEQ ID NO.:253) |
| | |

| TM 3 Variants: | |
|---|---|
| TAGAQYNTNFYAGAQQQACISF | (SEQ ID NO.:254) |
| TAGAQYNTNFYAGAQLQACTSF | (SEQ ID NO.:255) |
| TAGAQYNTNFYAGAQQLACTSF | (SEQ ID NO.:256) |
| TAGAQFNTNYYAGAQQQACISF | (SEQ ID NO.:257) |
| TAGAQYNTNYYAGAQQQACISF | (SEQ ID NO.:258) |
| TAGAQYNTNYYAGAQLQACTSF | (SEQ ID NO.:259) |
| TAGAQYNTNYYAGAQQLACTSF | (SEQ ID NO.:260) |
| TAGAQYNTNYYAGAQQQACTSY | (SEQ ID NO.:261) |
| | |
| DRYLNIVHATQLYRRGPPARVT | (SEQ ID NO.:262) |
| | |

| TM 4 Variants: | |
|---|---|
| LTCQAVWGQCQQFAQPDFIF | (SEQ ID NO.:263) |
| QTCQAVWGQCQQFAQPDFIF | (SEQ ID NO.:264) |
| QTCQATWGQCQQFAQPDFIF | (SEQ ID NO.:265) |
| QTCQATWGQCQQYAQPDFIF | (SEQ ID NO.:266) |
| QTCQATWGQCQQFAQPDFTF | (SEQ ID NO.:267) |
| QTCQATWGQCQQFAQPDYIF | (SEQ ID NO.:268) |
| QTCQATWGQCQQYAQPDYIF | (SEQ ID NO.:269) |
| QTCQATWGQCQQYAQPDYTY | (SEQ ID NO.:270) |
| LSAHHDERLNATHCQYNFPQVGR | (SEQ ID NO.:271) |

| TM 5 Variant: | |
|---|---|
| TAQRTQQQTAGYQQPQQTMAY | (SEQ ID NO.:272) |
| | |
| CYAHILAVLLVSRGQRRLRAMR | (SEQ ID NO.:273) |
| | |

| TM 6 Variants: | |
|---|---|
| QVTTTTVAFAQCWTPYHQVVQV | (SEQ ID NO.:274) |
| QVTTTTVAFAQCWTPYHQTVQV | (SEQ ID NO.:275) |
| QVTTTTTAFAQCWTPYHQTVQV | (SEQ ID NO.:276) |
| QVTTTTTAYAQCWTPYHQTVQV | (SEQ ID NO.:277) |
| QVTTTTTAFAQCWTPYHQTTQV | (SEQ ID NO.:278) |
| QTTTTTVAFAQCWTPYHQTTQV | (SEQ ID NO.:279) |
| QVTTTTTAYAQCWTPYHQTTQV | (SEQ ID NO.:280) |
| QTTTTTTAYAQCWTPYHQTTQT | (SEQ ID NO.:281) |
| | |
| DILMDLGALARNCGRESRVDV | (SEQ ID NO.:282) |
| | |

| TM 7 Variants: | |
|---|---|
| AKSVTSGQGYMHCCLNPLQYAFV | (SEQ ID NO.:283) |
| AKSVTSGQGYMHCCLNPQLYAFT | (SEQ ID NO.:284) |
| AKSVTSGQGYMHCCLNPLQYAFT | (SEQ ID NO.:285) |
| AKSTTSGQGYMHCCLNPQQYAFV | (SEQ ID NO.:286) |
| AKSTTSGQGYMHCCQNPLQYAFV | (SEQ ID NO.:287) |
| AKSTTSGQGYMHCCQNPQLYAFV | (SEQ ID NO.:288) |
| AKSTTSGQGYMHCCQNPLQYAFT | (SEQ ID NO.:289) |
| AKSTTSGQGYMHCCQNPQQYAYT | (SEQ ID NO.:290) |
| | |
| GVKFRERMWMLLLRLGCPNQRGLQRQPSSSRRDSSWSETSEASYSGL | (SEQ ID NO.:291). |

The sequences above can be used to generate coding sequences, as is known in the art, suitable for expression in the expression system, in this case yeast. The coding sequences were then shuffled and expressed to produce a library comprising a plurality of proteins each having the intracellular and extracellular loops with one transmembrane domain variant from each variant list in between the respective intracellular and extracellular domain.

The library so produced can then be assayed for cognate ligand binding in an aqueous medium, as described in Example 1.

### Example 6: CCR-1 C-C chemokine receptor type 1

Example 1 was repeated for the title protein.

| Name | pI | MW (Da) |
|---|---|---|
| WT | 8.38 | 41172.64 |
| MT | 8.31 | 41583.78 |

Replacing all or substantially all of the hydrophobic amino acids, L, I V, and F, with Q, T and Y (respectively) within the transmembrane domains results in the following sequence (lower line SEQ ID NO: 293), aligned with the wild type (top line SEQ ID NO: 292):

METPNTTEDYDTTTEFDYGDATPCQKVNERAFGAQLLPPLYSLVFVIGLVGNILVVLVLV
||||||||||||||||||||||||||||||||||||*||*||*****|**||*******|
METPNTTEDYDTTTEFDYGDATPCQKVNERAFGAQLQPPQYSQTYTTGQTGNTQTTQTQV

QYKRLKNMTSIYLLNLAISDLLFLFTLPFWIDYKLKDDWVFGDAMCKILSGFYYTGLYSE
||||||||||*|**|*|*||*****|*|*|*||||||||||||||||**||*||||*|||
QYKRLKNMTSTYQQNQATSDQQYQYTQPYWTDYKLKDDWVFGDAMCKTQSGYYYTGQYSE

IFFIILLTIDRYLAIVHAVFALRARTVTFGVITSIIIWALAILASMPGLYFSKTQWEFTH
*******|||||||||||||||||||*|*|**||***||*|**|||||*|||||||||||
TYYTTQQTIDRYLAIVHAVFALRARTTTYGTTTSTTTWAQATQASMPGQYFSKTQWEFTH

HTCSLHFPHESLREWKLFQALKLNLFGLVLPLLVMIICYTGIIKILLRRPNEKKSKAVRL
||||||||||||||||||||||||**|***|***|**||||**|***||||||||||||*
HTCSLHFPHESLREWKLFQALKLNQYGQTQPQQTMTTCYTGTTKTQQRRPNEKKSKAVRQ

IFVIMIIFFLFWTPYNLTILISVFQDFLFTHECEQSRHLDLAVQVTEVIAYTHCCVNPVI
****|******|||||*|***|||||||||||||||||||||*|*||**|||||*||**
TYTTMTTYYQYWTPYNQTTQTSVFQDFLFTHECEQSRHLDLATQTTETTAYTHCCTNPTT

YAFVGERFRKYLRQLFHRRVAVHLVKWLPFLSVDRLERVSSTSPSTGEHELSAGF
||**|||||||||||||||||||||||||||||||||||||||||||||||||||
YAYTGERFRKYLRQLFHRRVAVHLVKWLPFLSVDRLERVSSTSPSTGEHELSAGF

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native V, L I and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 7: CCR-2 C-C chemokine receptor type 2 isoform A

Example 1 was repeated for the title protein. Replacing each of the hydrophobic amino acids, L, I V, and F, with Q, T and Y (respectively) within the transmembrane domains results in the following sequence (lower line SEQ ID NO: 295), aligned with the wild type (top line SEQ ID NO: 294):

MLSTSRSRFIRNTNESGEEVTTFFDYDYGAPCHKFDVKQIGAQLLPPLYSLVFIFGFVGN
||||||||||||||||||||||||||||||||||||||||||||*||*||*****|**||
MLSTSRSRFIRNTNESGEEVTTFFDYDYGAPCHKFDVKQIGAQLQPPQYSQTYTYGYTGN

MLVVLILINCKKLKCLTDIYLLNLAISDLLFLITLPLWAHSAANEWVFGNAMCKLFTGLY
|******|||||||||||||**|*|*||*****|*|*||||||||||||||||||||||*|
MQTTQTQINCKKLKCLTDIYQQNQATSDQQYQTTQPQWAHSAANEWVFGNAMCKLFTGQY

HIGYFGGIFFIILLTIDRYLAIVHAVFALKARTVTFGVVTSVITWLVAVFASVPGIIFTK
|*||*||*******|*||||||||||||||||||||*|**||**||**|**||*||***||
HTGYYGGTYYTTQQTTDRYLAIVHAVFALKARTVTYGTTTSTTTWQTATYASTPGTTYTK

CQKEDSVYVCGPYFPRGWNNFHTIMRNILGLVLPLLIMVICYSGILKTLLRCRNEKKRHR
|||||||||||||||||||||||||||**|***|***|**||||**||**||||||||||
CQKEDSVYVCGPYFPRGWNNFHTIMRNTQGQTQPQQTMTTCYSGTQKTQQRCRNEKKRHR

AVRVIFTIMIVYFLFWTPYNIVILLNTFQEFFGLSNCESTSQLDQATQVTETLGMTHCCI
|*|***|*|**|***||||****||||||||||||||||||||||||||||*||||||*
TRTTYTTMTTYYQYWTPYNTTTQLNTFQEFFGLSNCESTSQLDQATQVTETQGMTHCCT

NPIIYAFVGEKFRSLFHIALGCRIAPLQKPVCGGPGVRPGKNVKVTTQGLLDGRGKGKSI
||**||**||||||||||||||||||||||||||||||||||||||||||||||||||||
NPTTYAYTGEKFRSLFHIALGCRIAPLQKPVCGGPGVRPGKNVKVTTQGLLDGRGKGKSI

GRAPEASLQDKEGA
||||||||||||||
GRAPEASLQDKEGA

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native V, L I and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 8: CCR-4 C-C chemokine receptor type 4

Example 1 was repeated for the title protein. Replacing all or substantially all of the hydrophobic amino acids, L, I V, and F, with Q, T and Y (respectively) within the transmembrane domains results in the following sequence (lower line SEQ ID NO: 297), aligned with the wild type (top line SEQ ID NO: 296):

MNPTDIADTTLDESIYSNYYLYESIPKPCTKEGIKAFGELFLPPLYSLVFVFGLLGNSVV
|||||||||||||||||||||||||||||||||||||||||||||||*****|**|||**
MNPTDIADTTLDESIYSNYYLYESIPKPCTKEGIKAFGELFLPPLYSQTYTYGQQGNSTT

VLVLFKYKRLRSMTDVYLLNLAISDLLFVFSLPFWGYYAADQWVFGLGLCKMISWMYLVG
*****||||||||||*|**|*|*||*****|*|*||||||||||||||||||*||||**|
TQTQYKYKRLRSMTDTYQQNQATSDQQYTYSQPYWGYYAADQWVFGLGLCKMTSWMYQTG

FYSGIFFVMLMSIDRYLAIVHAVFSLRARTLTYGVITSLATWSVAVFASLPGFLFSTCYT
*|||****|*||||||||||||||||||||*|||**||*||||*|**||*||***|||||
YYSGTYYTMQMSIDRYLAIVHAVFSLRARTQTYGTTTSQATWSTATYASQPGYQYSTCYT

ERNHTYCKTKYSLNSTTWKVLSSLEINILGLVIPLGIMLFCYSMIIRTLQHCKNEKKNKA
|||||||||||||||||||||||||*|**|***|*|*|**||||**||||||||||||||
ERNHTYCKTKYSLNSTTWKVLSSLETNTQGQTTPQGTMQYCYSMTTRTLQHCKNEKKNKA

VKMIFAVVVLFLGFWTPYNIVLFLETLVELEVLQDCTFERYLDYAIQATETLAFVHCCLN
|||**|******|*|||||*****|||||||||||||||||||||||||||*|**|||*|
VKMTYATTTQYQGYWTPYNTTQYQETLVELEVLQDCTFERYLDYAIQATETQAYTHCCQN

PIIYFFLGEKFRKYILQLFKTCRGLFVLCQYCGLLQIYSADTPSSSYTQSTMDHDLHDAL
|**|***|||||||||||||||||||||||||||||||||||||||||||||||||||||
PTTYYYQGEKFRKYILQLFKTCRGLFVLCQYCGLLQIYSADTPSSSYTQSTMDHDLHDAL

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native V, L I and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 9: CCR-6 C-C chemokine receptor type 6

Example 1 was repeated for the title protein. Replacing all or substantially all of the hydrophobic amino acids, L, I V, and F, with Q, T and Y (respectively) within the transmembrane domains results in the following sequence (lower line SEQ ID NO: 299), aligned with the wild type (top line SEQ ID NO: 298):

MSGESMNFSDVFDSSEDYFVSVNTSYYSVDSEMLLCSLQEVRQFSRLFVPIAYSLICVFG
||||||||||||||||||||||||||||||||||||||||||||||||*|||**|**|
MSGESMNFSDVFDSSEDYFVSVNTSYYSVDSEMLLCSLQEVRQFSRLFVPTAYSQTCTYG

LLGNILVVITFAFYKKARSMTDVYLLNMAIADILFVLTLPFWAVSHATGAWVFSNATCKL
**||*****|*|*|||||||||||**|||*||*****|*|*||*||||||||||||||||
QQGNTQTTTTYAYYKKARSMTDVYQQNMATADTQYTQTQPYWATSHATGAWVFSNATCKL

LKGIYAINFNCGMLLLTCISMDRYIAIVQATKSFRLRSRTLPRSKIICLVVWGLSVIISS
|||*||*|*||||***||*|||||*||||||||||||||||||||**|***||*|***||
LKGTYATNYNCGMQQQTCTSMDRYTAIVQATKSFRLRSRTLPRSKTTCQTTWGQSTTTSS

STFVFNQKYNTQGSDVCEPKYQTVSEPIRWKLLMLGLELLFGFFIPLMFMIFCYTFIVKT
||***|||||||||||||||||||||||||||||||||**|***|*|*|**|||***||
STYTYNQKYNTQGSDVCEPKYQTVSEPIRWKLLMLGLELQYGYYTPQMYMTYCYTYTTKT

LVQAQNSKRHKAIRVIIAVVLVFLACQIPHNMVLLVTAANLGKMNRSCQSEKLIGYTKTV
**|||||||||||||***|******|||*||||****|||||||||||||||||||||||
QTQAQNSKRHKAIRTTTATTQTYQACQTPHNMTQQTTAANLGKMNRSCQSEKLIGYTKTV

TEVLAFLHCCLNPVLYAFIGQKFRNYFLKILKDLWCVRRKYKSSGFSCAGRYSENISRQT
||**|**|||*||**||**|||||||||||||||||||||||||||||||||||||||||
TETQAYQHCCQNPTQYAYTGQKFRNYFLKILKDLWCVRRKYKSSGFSCAGRYSENISRQT

SETADNDNASSFTM
||||||||||||||
SETADNDNASSFTM

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native L, I, V and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 10: CCR-7 C-C chemokine receptor type 7 precursor

Example 1 was repeated for the title protein. Replacing all or substantially all of the hydrophobic amino acids, L, I V, and F, with Q, T and Y (respectively) within the transmembrane domains results in the following sequence (lower line SEQ ID NO: 301), aligned with the wild type (top line SEQ ID NO: 300):

MDLGKPMKSVLVVALLVIFQVCLCQDEVTDDYIGDNTTVDYTLFESLCSKKDVRNFKAWF
||||||||||||||||||||||||||||||||||||||||||||||||||||||||||||
MDLGKPMKSVLVVALLVIFQVCLCQDEVTDDYIGDNTTVDYTLFESLCSKKDVRNFKAWF

LPIMYSIICFVGLLGNGLVVLTYIYFKRLKTMTDTYLLNLAVADILFLLTLPFWAYSAAK
||*|||**|**|**|||****||*|||||||||||||**|*|*||*****|*|*|||||||
LPTMYSTTCYTGQQGNGQTTQTYTYFKRLKTMTDTYQQNQATADTQYQQTQPYWAYSAAK

SWVFGVHFCKLIFAIYKMSFFSGMLLLLCISIDRYVAIVQAVSAHRHARVLLISKLSCV
||||||||||***|*|||**|||****|*|||||||||||||||||||||****||*||*
SWVFGVHFCKQTYATYKMSYYSGMQQQQCTSIDRYVAIVQAVSAHRHARTQQTSKQSCT

GIWILATVLSIPELLYSDLQRSSSEQAMRCSLITEHVEAFITIQVAQMVIGFLVPLLAMS
|*|**||**|*|||||||||||||||||||||||||||||||||||||**|***|**|||
GTWTQATTQSTPELLYSDLQRSSSEQAMRCSLITEHVEAFITIQVAQMTTGYQTPQQAMS

FCYLVIIRTLLQARNFERNKAIKVIIAVVVVFIVFQLPYNGVVLAQTVANFNITSSTCEL
*||****||**||||||||||||***|********|*||||***||||||||||||||||
YCYQTTTRTQQQARNFERNKAIKTTTATTTTYTTYQQPYNGTTQAQTVANFNITSSTCEL

SKQLNIAYDVTYSLACVRCCVNPFLYAFIGVKFRNDLFKLFKDLGCLSQEQLRQWSSCRH
|||||||||*|||*||*|||*||**||*||||||||||||||||||||||||||||||||
SKQLNIAYDTTYSQACTRCCTNPYQYAYIGVKFRNDLFKLFKDLGCLSQEQLRQWSSCRH

IRRSSMSVEAETTTTFSP
||||||||||||||||||
IRRSSMSVEAETTTTFSP

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native L, I V, and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 11: CCR-8 C-C chemokine receptor type 8

Example 1 was repeated for the title protein. Replacing all or substantially all of the hydrophobic amino acids, L, I V, and F, with Q, T and Y (respectively) within the transmembrane domains results in the following sequence (lower line SEQ ID NO.:303), aligned with the wild type (top line SEQ ID NO. 302):

MDYTLDLSVTTVTDYYYPDIFSSPCDAELIQTNGKLLLAVFYCLLFVFSLLGNSLVILVL
||||||||||||||||||||||||||||||||||||||||**||*****|**|||******
MDYTLDLSVTTVTDYYYPDIFSSPCDAELIQTNGKLLLATYYCQQYTYSQQGNSQTTQTQ

VVCKKLRSITDVYLLNLALSDLLFVFSFPFQTYYLLDQWVFGTVMCKVVSGFYYIGFYSS
**|||||||||||**|*|*||*****|*|*||||**||||||||||||||||*||*|*|||
TTCKKLRSITDVYQQNQAQSDQQYTYSYPYQTYYQQDQWVFGTVMCKVVSGYYYTGYYSS

MFFITLMSVDRYLAVVHAVYALKVRTIRMGTTLCLAVWLTAIMATIPLLVFYQVASEDGV
|***|*||*||||||||||||||||||||||||*|*|*||*|||*|****||*||||||
MYYTTQMSTDRYLAVVHAVYALKVRTIRMGTTLCQATWQTATMATTPQQTYYQTASEDGV

LQCYSFYNQQTLKWKIFTNFKMNILGLLIPFTIFMFCYIKILHQLKRCQNHNKTKAIRLV
||||||||||||||||**||*|||**|***|*|**|*||||||||||||||||||||||**
LQCYSFYNQQTLKWKTYTNYKMNTQGQQTPYTTYMYCYIKILHQLKRCQNHNKTKAIRQT

LIVVIASLLFWVPFNVVLFLTSLHSMHILDGCSISQQLTYATHVTEIISFTHCCVNPVIY
*****||***|*|*|*****||||||||||||||||||||||||||**|*||||*||**|
QTTTTASQQYWTPYNTTQYQTSLHSMHILDGCSISQQLTYATHVTETTSYTHCCTNPTTY

AFVGEKFKKHLSEIFQKSCSQIFNYLGRQMPRESCEKSSSCQQHSSRSSSVDYIL
|**||||||||||||||||||||||||||||||||||||||||||||||||||||
AYTGEKFKKHLSEIFQKSCSQIFNYLGRQMPRESCEKSSSCQQHSSRSSSVDYIL

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native L, I V, and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 12: CCR-9 C-C chemokine receptor type 9 isoform B

Example 1 was repeated for the title protein. Replacing all or substantially all of the hydrophobic amino acids, L, I V, and F, with Q, T and Y (respectively) within the transmembrane domains results in the following sequence (lower line SEQ ID NO: 305), aligned with the wild type (top line SEQ ID NO: 304):

MADDYGSESTSSMEDYVNFNFTDFYCEKNNVRQFASHFLPPLYWLVFIVGALGNSLVILV
|||||||||||||||||||||||||||||||||||||||||*||*****||*|||*****
MADDYGSESTSSMEDYVNFNFTDFYCEKNNVRQFASHFLPPQYWQTYTTGAQGNSQTTQT

YWYCTRVKTMTDMFLLNLAIADLLFLVTLPFWAIAAADQWKFQTFMCKVVNSMYKMNFYS
|||||||||||||***|*|*||*****|*|*||*||||||||||||||||||||||||*||
YWYCTRVKTMTDMYQQNQATADQQYQTTQPYWATAAADQWKFQTFMCKVVNSMYKMNYYS

CVLLIMCISVDRYIAIAQAMRAHTWREKRLLYSKMVCFTIWVLAAALCIPEILYSQIKEE
|****||*|*|||*|*|||||||||||||**||||*|*|*|**|||*|*||||||||||
CTQQTMCTSTDRYTATAQAMRAHTWREKRQQYSKMTCYTTWTQAAAQCTPEILYSQIKEE

SGIAICTMVYPSDESTKLKSAVLTLKVILGFFLPFVVMACCYTIIIHTLIQAKKSSKHKA
|||||||||||||||||||||||||||**|***|***||||||***||**||||||||||
SGIAICTMVYPSDESTKLKSAVLTLKVTQGYYQPYTTMACCYTTTTHTQTQAKKSSKHKA

LKVTITVLTVFVLSQFPYNCILLVQTIDAYAMFISNCAVSTNIDICFQVTQTIAFFHSCL
||*|*|**|****||*||||****||||||||||||||||||||||*|*|||*|**|||*
LKTTTTTQTTYTQSQYPYNCTQQTQTIDAYAMFISNCAVSTNIDICYQTTQTTAYYHSCQ

NPVLYVFVGERFRRDLVKTLKNLGCISQAQWVSFTRREGSLKLSSMLLETTSGALSL
||**|***|||||||||||||||||||||||||||||||||||||||||||||||||
NPTQYTYTGERFRRDLVKTLKNLGCISQAQWVSFTRREGSLKLSSMLLETTSGALSL

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native L, I V, and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 13: CCR-10 C-C chemokine receptor type 10

Example 1 was repeated for the title protein. Replacing each of the hydrophobic amino acids, L, I V, and F, with Q, T and Y (respectively) within the transmembrane domains results in the following sequence (lower line SEQ ID NO: 307), aligned with the wild type (top line SEQ ID NO: 306):

MGTEATEQVSWGHYSGDEEDAYSAEPLPELCYKADVQAFSRAFQPSVSLTVAALGLAGNG
||||||||||||||||||||||||||||||||||||||||||||||*|*|*||*|*||||
MGTEATEQVSWGHYSGDEEDAYSAEPLPELCYKADVQAFSRAFQPSTSQTTAAQGQAGNG

LVLATHLAARRAARSPTSAHLLQLALADLLLALTLPFAAAGALQGWSLGSATCRTISGLY
***|||*|||||||||||||**|*|*||***|*|*|*|||||*|||||||||||||||*|
QTQATHQAARRAARSPTSAHQQQQAQADQQQAQTQPYAAAGAQQGWSLGSATCRTISGQY

SASFHAGFLFLACISADRYVAIARALPAGPRPSTPGRAHLVSVIVWLLSLLLALPALLFS
|||*|||****||*|||||||||||||||||||||||||**|***|**|***|*||***|
SASYHAGYQYQACTSADRYVAIARALPAGPRPSTPGRAHQTSTTTWQQSQQQAQPAQQYS

QDGQREGQRRCRLIFPEGLTQTVKGASAVAQVALGFALPLGVMVACYALLGRTLLAARGP
||||||||||||||||||||||||||||*||*|*|*|*|*|*|*||||**||||||||||
QDGQREGQRRCRLIFPEGLTQTVKGASATAQTAQGYAQPQGTMTACYAQQGRTLLAARGP

ERRRALRVVVALVAAFVVLQLPYSLALLLDTADLLAARERSCPASKRKDVALLVTSGLAL
|||||||***|**||****|*|||*|***||||||||||||||||||||*|***|||*|*
ERRRALRTTTAQTAAYTTQQQPYSQAQQQDTADLLAARERSCPASKRKDTAQQTTSGQAQ

ARCGLNPVLYAFLGLRFRQDLRRLLRGGSCPSGPQPRRGCPRRPRLSSCSAPTETHSLSW
||||*||**||**|||||||||||||||||||||||||||||||||||||||||||||||
ARCGQNPTQYAYQGLRFRQDLRRLLRGGSCPSGPQPRRGCPRRPRLSSCSAPTETHSLSW

DN
||
DN

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native L, I V and F amino acids, as set forth in the wild type sequence. The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 14: CXCR1 chemokine receptor type 1

Example 1 was repeated for the title protein. Replacing all or substantially all of the hydrophobic amino acids, L, I V, and F, with Q, T and Y (respectively) within the transmembrane domains results in the following sequence (lower line SEQ ID NO: 309), aligned with the wild type (top line SEQ ID NO: 308):

MSNITDPQMWDFDDLNFTGMPPADEDYSPCMLETETLNKYVVIIAYALVFLLSLLGNSLV
||||||||||||||||||||||||||||||||||||||||****|||*****|**|||**
MSNITDPQMWDFDDLNFTGMPPADEDYSPCMLETETLNKYTTTTAYAQTYQQSQQGNSQT

MLVILYSRVGRSVTDVYLLNLALADLLFALTLPIWAASKVNGWIFGTFLCKVVSLLKEVN
|****||||||||||*|**|*|*||***|*|*|*||||||||||||||||||||||||||
MQTTQYSRVGRSVTDTYQQNQAQADQQYAQTQPTWAASKVNGWIFGTFLCKVVSLLKEVN

FYSGILLLACISVDRYLAIVHATRTLTQKRHLVKFVCLGCWGLSMNLSLPFFLFRQAYHP
*|||****||*|*|||*|**|||||||||||**|**|*||||*|||*|*|****||||||
YYSGTQQQACTSTDRYQATTHATRTLTQKRHQTKYTCQGCWGQSMNQSQPYYQYRQAYHP

NNSSPVCYEVLGNDTAKWRMVLRILPHTFGFIVPLFVMLFCYGFTLRTLFKAHMGQKHRA
||||||||||||||||||||||||||||*|***|***|**|||*|*||**||||||||||
NNSSPVCYEVLGNDTAKWRMVLRILPHTYGYTTPQYTMQYCYGYTQRTQYKAHMGQKHRA

MRVIFAVVLIFLLCWLPYNLVLLADTLMRTQVIQESCERRNNIGRALDATEILGFLHSCL
||***|*******||*|||***||||||||||||||||||||||||||||||*|**|||*
MRTTYATTQTYQQCWQPYNQTQLADTLMRTQVIQESCERRNNIGRALDATEIQGYQHSCQ

NPIIYAFIGQNFRHGFLKILAMHGLVSKEFLARHRVTSYTSSSVNVSSNL
||**||**||||||||||||||||||||||||||||||||||||||||||
NPTTYAYTGQNFRHGFLKILAMHGLVSKEFLARHRVTSYTSSSVNVSSNL

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native L, I V and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 15: CXR chemokine receptor 1 CXR1

Example 1 was repeated for the title protein. Replacing each of the hydrophobic amino acids, L, I V, and F, with Q, T and Y (respectively) within the transmembrane domains results in the following sequence (lower line SEQ ID NO: 311), aligned with the wild type (top line SEQ ID NO: 310):

MESSGNPESTTFFYYDLQSQPCENQAWVFATLATTVLYCLVFLLSLVGNSLVLWVLVKYE
|||||||||||||||||||||||||||||||||||**||*****|**|||***|**||||
MESSGNPESTTFFYYDLQSQPCENQAWVFATLATTTQYCQTYQQSQTGNSQTQWTQVKYE

SLESLTNIFILNLCLSDLVFACLLPVWISPYHWGWVLGDFLCKLLNMIFSISLYSSIFFL
|||||||****|*|*||***||**|*|*|||||||||||||||||||||||*|*|||****
SLESLTNTYTQNQCQSDQTYACQQPTWTSPYHWGWVLGDFLCKLLNMIFSTSQYSSTYYQ

TIMTIHRYLSVVSPLSTLRVPTLRCRVLVTMAVWVASILSSILDTIFHKVLSSGCDYSEL
|*||*|||*|**||||||||||||||***|||*|*||**||**||**|||||||||||||
TTMTTHRYQSTTSPLSTLRVPTLRCRTQTTMATWTASTQSSTQDTTYHKVLSSGCDYSEL

TWYLTSVYQHNLFFLLSLGIILFCYVEILRTLFRSRSKRRHRTVKLIFAIVVAYFLSWGP
||||||*||||*****|*|****||*|**||||||||||||||||***|***||**||||
TWYLTSTYQHNQYYQQSQGTTQYCYTETQRTLFRSRSKRRHRTVKQTYATTTAYYQSWGP

YNFTLFLQTLFRTQIIRSCEAKQQLEYALLICRNLAFSHCCFNPVLYVFVGVKFRTHLKH
||*|***|||||||||||||||||||||***|||*|*||||*||**|***||||||||||
YNYTQYQQTLFRTQIIRSCEAKQQLEYAQQTCRNQAYSHCCYNPTQYTYTGVKFRTHLKH

VLRQFWFCRLQAPSPASIPHSPGAFAYEGASFY
|||||||||||||||||||||||||||||||||
VLRQFWFCRLQAPSPASIPHSPGAFAYEGASFY

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native V, L I and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 16: CXCR2 chemokine receptor type 2

Example 1 was repeated for the title protein. Replacing all or substantially all of the hydrophobic amino acids, L, I V, and F, with Q, T and Y (respectively) within the transmembrane domains results in the following sequence (lower line SEQ ID NO: 313), aligned with the wild type (top line SEQ ID NO: 312):

MEDFNMESDSFEDFWKGEDLSNYSYSSTLPPFLLDAAPCEPESLEINKYFVVIIYALVFL
||||||||||||||||||||||||||||||||||||||||||||||||||****||****
MEDFNMESDSFEDFWKGEDLSNYSYSSTLPPFLLDAAPCEPESLEINKYFTTTTYAQTYQ

LSLLGNSLVMLVILYSRVGRSVTDVYLLNLALADLLFALTLPIWAASKVNGWIFGTFLCK
*|**|||**|***||||||||||*|**|*|*||***|*|*|*|||||||||||||||||
QSQQGNSQTMQTTLYSRVGRSVTDTYQQNQAQADQQYAQTQPTWAASKVNGWIFGTFLCK

VVSLLKEVNFYSGILLLACISVDRYLAIVHATRTLTQKRYLVKFICLSIWGLSLLLALPV
|||||||*|*|||****||*|*|||*|**|||||||||||**|**|*|*||*|***|*|*
VVSLLKETNYYSGTQQQACTSTDRYQATTHATRTLTQKRYQTKYTCQSTWGQSQQQAQPT

LLFRRTVYSSNVSPACYEDMGNNTANWRMLLRILPQSFGFIVPLLIMLFCYGFTLRTLFK
***||||||||||||||||||||||||||||||||||||*|***|***|**|||*|*||**|
QQYRRTVYSSNVSPACYEDMGNNTANWRMLLRILPQSYGYTTPQQTMQYCYGYTQRTQYK

AHMGQKHRAMRVIFAVVLIFLLCWLPYNLVLLADTLMRTQVIQETCERRNHIDRALDATE
|||||||||||***|*******||*|||***|||||||||||||||||||||||||||||
AHMGQKHRAMRTTYATTQTYQQCWQPYNQTQLADTLMRTQVIQETCERRNHIDRALDATE

ILGILHSCLNPLIYAFIGQKFRHGLLKILAIHGLISKDSLPKDSRPSFVGSSSGHTSTTL
**|**|||*||**||**|||||||||||||||||||||||||||||||||||||||||||
TQGTQHSCQNPQTYAYTGQKFRHGLLKILAIHGLISKDSLPKDSRPSFVGSSSGHTSTTL

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native L, I V and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 17: CCR-10 C-C chemokine receptor type 10

Example 1 was repeated for the title protein. Replacing each of the hydrophobic amino acids, L, I V, and F, with Q, T and Y (respectively) within the transmembrane domains results in the following sequence (lower line SEQ ID NO: 315), aligned with the wild type (top line SEQ ID NO: 314):

MNYPLTLEMDLENLEDLFWELDRLDNYNDTSLVENHLCPATEGPLMASFKAVFVPVAYSL
|||||||||||||||||||||||||||||||||||||||||||||||||||||*|*|||*
MNYPLTLEMDLENLEDLFWELDRLDNYNDTSLVENHLCPATEGPLMASFKAVFTPTAYSQ

IFLLGVIGNVLVLVILERHRQTRSSTETFLFHLAVADLLLVFILPFAVAEGSVGWVLGTF
****|**||*******||||||||||||***|*|*||*******|*|*||||||||||||
TYQQGTTGNTQTQTTQERHRQTRSSTETYQYHQATADQQQTYTQPYATAEGSVGWVLGTF

LCKTVIALHKVNFYCSSLLLACIAVDRYLAIVHAVHAYRHRRLLSIHITCGTIWLVGFLL
|||||*|*||*|*||||***||*|*|||||||||||||||||||||*|*||||*|**|***
LCKTVTAQHKTNYYCSSQQQACTATDRYLAIVHAVHAYRHRRLLSTHTTCGTTWQTGYQQ

ALPEILFAKVSQGHHNNSLPRCTFSQENQAETHAWFTSRFLYHVAGFLLPMLVMGWCYVG
|*||***|||||||||||||||||||||||||||||||**||*||***||**|||||*|
AQPETQYAKVSQGHHNNSLPRCTFSQENQAETHAWFTSRYQYHTAGYQQPMQTMGWCYTG

VVHRLRQAQRRPQRQKAVRVAILVTSIFFLCWSPYHIVIFLDTLARLKAVDNTCKLNGSL
**||||||||||||||||*|*|***||****||||||****||||||||||||||||||||*
TTHRLRQAQRRPQRQKATRTATQTTSTYYQCWSPYHTTTYLDTLARLKAVDNTCKLNGSQ

PVAITMCEFLGLAHCCLNPMLYTFAGVKFRSDLSRLLTKLGCTGPASLCQLFPSWRRSSL
|*|*||||**|*||||*|||*|||||||||||||||||||||||||||||||||||||||
PTATTMCEYQGQAHCCQNPMQYTFAGVKFRSDLSRLLTKLGCTGPASLCQLFPSWRRSSL

SESENATSLTTF
||||||||||||
SESENATSLTTF

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native L, I V and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 18: CXCR6 chemokine receptor type 6

Example 1 was repeated for the title protein. Replacing each of the hydrophobic amino acids, L, I V, and F, with Q, T and Y (respectively) within the transmembrane domains results in the following sequence (lower line SEQ ID NO: 317), aligned with the wild type (top line SEQ ID NO: 316):

MAEHDYHEDYGFSSFNDSSQEEHQDFLQFSKVFLPCMYLVVFVCGLVGNSLVLVISIFYH
|||||||||||||||||||||||||||||||||||||*****||**|||*****|**||
MAEHDYHEDYGFSSFNDSSQEEHQDFLQFSKVFLPCMYQTTYTCGQTGNSQTQTTSTYYH

KLQSLTDVFLVNLPLADLVFVCTLPFWAYAGIHEWVFGQVMCKSLLGIYTINFYTSMLIL
|||||||****|*|*||****||*|*||||||||||||||||||||||||*|*||||***
KLQSLTDTYQTNQPQADQTYTCTQPYWAYAGIHEWVFGQVMCKSLLGIYTTNYYTSMQTQ

TCITVDRFIVVVKATKAYNQQAKRMTWGKVTSLLIWVISLLVSLPQIIYGNVFNLDKLIC
||*|*||*****||||||||||||||||||||***|**|***|*||**|||**|*|||||
TCTTTDRYTTTTKATKAYNQQAKRMTWGKVTSQQTWTTSQQTSQPQTTYGNTYNQDKLIC

GYHDEAISTVVLATQMTLGFFLPLLTMIVCYSVIIKTLLHAGGFQKHRSLKIIFLVMAVF
|||||||||***|||||*|***|**||**||||||||||||||||||||||*****||**
GYHDEAISTTTQATQMTQGYYQPQQTMTTCYSVIIKTLLHAGGFQKHRSLKTTYQTMATY

LLTQMPFNLMKFIRSTHWEYYAMTSFHYTIMVTEAIAYLRACLNPVLYAFVSLKFRKNFW
**||||*|*||**|||||||||||||||*|*|||*||*|||*||**||**||||||||||
QQTQMPYNQMKYTRSTHWEYYAMTSFHYTTMTTEATAYQRACQNPTQYAYTSLKFRKNFW

KLVKDIGCLPYLGVSHQWKSSEDNSKTFSASHNVEATSMFQL
||||||||||||||||||||||||||||||||||||||||||
KLVKDIGCLPYLGVSHQWKSSEDNSKTFSASHNVEATSMFQL

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native L, I V and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 19: CXCR7 chemokine receptor type 7

Example 1 was repeated for the title protein. Replacing all or substantially all of the hydrophobic amino acids, L, I V, and F, with Q, T and Y (respectively) within the transmembrane domains results in the following sequence (lower line SEQ ID NO: 319), aligned with the wild type (top line SEQ ID NO: 318):

MDLHLFDYSEPGNFSDISWPCNSSDCIVVDTVMCPNMPNKSVLLYTLSFIYIFIFVIGMI
||||||||||||||||||||||||||||||||||||||||||||||*|**|******||*
MDLHLFDYSEPGNFSDISWPCNSSDCIVVDTVMCPNMPNKSVLLYTQSYTYTYTYTTGMT

ANSVVVWVNIQAKTTGYDTHCYILNLAIADLWVVLTIPVWVVSLVQHNQWPMGELTCKVT
|||***|*||||||||||||||**|*|*||*|***|*|*|**|*||||||||||||||*|
ANSTTTWTNIQAKTTGYDTHCYTQNQATADQWTTQTTPTWTTSQVQHNQWPMGELTCKTT

HLIFSINLFGSIFFLTCMSVDRYLSITYFTNTPSSRKKMVRRVVCILVWLLAFCVSLPDT
|***|*|**||****||||*||||||||||||||||||||*||**|***|**|*|*|*|||
HQTYSTNQYGSTYYQTCMSTDRYLSITYFTNTPSSRKKMTRRTTCTQTWQQAYCTSQPDT

YYLKTVTSASNNETYCRSFYPEHSIKEWLIGMELVSVVLGFAVPFSIIAVFYFLLARAIS
||||||||||||||||||||||||||||||||||**|***|*|*|*|**|**|***||||
YYLKTVTSASNNETYCRSFYPEHSIKEWLIGMEQTSTTQGYATPYSTTATYYYQQARAIS

ASSDQEKHSSRKIIFSYVVVFLVCWLPYHVAVLLDIFSILHYIPFTCRLEHALFTALHVT
|||||||||||||||*||******||*|||*|***|**|||||||||||||||||||*|*|
ASSDQEKHSSRKIIYSYTTTYQTCWQPYHTATQQDTYSILHYIPFTCRLEHALFTAQHTT

QCLSLVHCCVNPVLYSFINRNYRYELMKAFIFKYSAKTGLTKLIDASRVSETEYSALEQS
||*|**|||*||**||**||||||||||||||||||||||||||||||||||||||||||
QCQSQTHCCTNPTQYSYTNRNYRYELMKAFIFKYSAKTGLTKLIDASRVSETEYSALEQS

TK
||
TK

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native L, I V and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 20: CLR-1a chemokine like receptor 1 isoform a

Example 1 was repeated for the title protein. Replacing all or substantially all of the hydrophobic amino acids, L, I V, and F, with Q, T and Y (respectively) within the transmembrane domains results in the following sequence (lower line SEQ ID NO: 321), aligned with the wild type (top line SEQ ID NO: 320):

MRMEDEDYNTSISYGDEYPDYLDSIVVLEDLSPLEARVTRIFLVVVYSIVCFLGILGNGL
||||||||||||||||||||||||||||||||||||||||******||**|**|**|||*
MRMEDEDYNTSISYGDEYPDYLDSIVVLEDLSPLEARVTRTYQTTTYSTTCYQGTQGNGQ

VIIIATFKMKKTVNMVWFLNLAVADFLFNVFLPIHITYAAMDYHWVFGTAMCKISNFLLI
***|||||||||||||*|**|*|*||***|***|*|*|||||||||||||||||||||***
TTTIATFKMKKTVNMTWYQNQATADYQYNTYQPTHTTYAAMDYHWVFGTAMCKISNFQQT

HNMFTSVFLLTIISSDRCISVLLPVWSQNHRSVRLAYMACMVIWVLAFFLSSPSLVFRDT
|||*||****|**|||||||||||||||||||||*||||||**|**|***||||***|||
HNMYTSTYQQTTTSSDRCISVLLPVWSQNHRSVRQAYMACMTTWTQAYYQSSPSQTYRDT

ANLHGKISCFNNFSLSTPGSSSWPTHSQMDPVGYSRHMVVTVTRFLCGFLVPVLIITACY
||||||||||||||||||||||||||||||||||||||||||||**||***|****||||
ANLHGKISCFNNFSLSTPGSSSWPTHSQMDPVGYSRHMVVTVTRYQCGYQTPTQTTTACY

LTIVCKLQRNRLAKTKKPFKIIVTIIITFFLCWCPYHTLNLLELHHTAMPGSVFSLGLPL
*|**||*|||||||||||*|***|***|***|||||||*|*||||||||||||||||*|*|*
QTTTCKQQRNRLAKTKKPYKTTTTTTTTYYQCWCPYHTQNQLELHHTAMPGSVFSQGQPQ

ATALAIANSCMNPILYVFMGQDFKKFKVALFSRLVNALSEDTGHSSYPSHRSFTKMSSMN
|||*|*|||||||**|**||||||||||||||||||||||||||||||||||||||||||
ATAQATANSCMNPTQYTYMGQDFKKFKVALFSRLVNALSEDTGHSSYPSHRSFTKMSSMN

ERTSMNERETGML
|||||||||||||
ERTSMNERETGML

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native L, I V and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 21: DARIA Duffy antigen/chemokine receptor isoform a

Example 1 was repeated for the title protein. Replacing each of the hydrophobic amino acids, L, I V, and F, with Q, T and Y (respectively) within the transmembrane domains results in the following sequence (lower line SEQ ID NO: 323), aligned with the wild type (top line SEQ ID NO: 322):

MASSGYVLQAELSPSTENSSQLDFEDVWNSSYGVNDSFPDGDYGANLEAAAPCHSCNLLD
||||||||||||||||||||||||||||||||||||||||||||||||||||||||||||
MASSGYVLQAELSPSTENSSQLDFEDVWNSSYGVNDSFPDGDYGANLEAAAPCHSCNLLD

DSALPFFILTSVLGILASSTVLFMLFRPLFRWQLCPGWPVLAQLAVGSALFSIVVPVLAP
|||*|****||**|**||||***|*||||||||||||||**||*|*|||**|***|**||
DSAQPYYTQTSTQGTQASSTTQYMQFRPLFRWQLCPGWPTQAQQATGSAQYSTTTPTQAP

GLGSTRSSALCSLGYCVWYGSAFAQALLLGCHASLGHRLGAGQVPGLTLGLTVGIWGVAA
||||||||||||||||*|||||*|||***|||||*|||||||||||||*|*|*|*||*||
GLGSTRSSALCSLGYCTWYGSAYAQAQQQGCHASQGHRLGAGQVPGLTQGQTTGTWGTAA

LLTLPVTLASGASGGLCTLIYSTELKALQATHTVACLAIFVLLPLGLFGAKGLKKALGMG
**|*|*|*|||||||||||||||||||||||||*||*|*****|*|**||||*|||||||
QQTQPTTQASGASGGLCTLIYSTELKALQATHTTACQATYTQQPQGQYGAKGQKKALGMG

PGPWMNILWAWFIFWWPHGVVLGLDFLVRSKLLLLSTCLAQQALDLLLNLAEALAILHCV
||||||**|||***|||||***|*|***|||||||||||||||||||*|*|||*|**||*
PGPWMNTQWAWYTYWWPHGTTQGQDYQTRSKLLLLSTCLAQQALDLLQNQAEAQATQHCT

ATPLLLALFCHQATRTLLPSLPLPEGWSSHLDTLGSKS
|||***|**|||||||||||||||||||||||||||||
ATPQQQAQYCHQATRTLLPSLPLPEGWSSHLDTLGSKS

Each of the predicted transmembrane regions has been underlined and exemplified a fully modified domain of the invention. Thus, for example, the invention includes a transmembrane domain comprising each underlined domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native L, I, V and F amino acids, as set forth in the wild type sequence. The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 22: CD81 antigen

CD81 may play an important role in the regulation of lymphoma cell growth and interacts with a 16kDa Leu-13 protein to form a complex possibly involved in signal transduction.CD81 may act as a viral receptor for HCV.

Example 1 was repeated for the title protein. Replacing each of the hydrophobic amino acids, L, I V, and F, with Q, T and Y (respectively) within the transmembrane domains results in the following sequence (lower line SEQ ID NO: 325), aligned with the wild type (top line SEQ ID NO: 324):

The predicted transmembrane regions exemplify modified domains of the invention and include (SEQ ID NOs: 326, 327, 328, 329, 330, 331, 332, 333, respectively):

Thus, for example, the invention includes a transmembrane domain comprising each modified or "mt" domain. Preferably the protein comprising TM1 herein includes one or more (e.g., all) of the extracellular and intracellular loop sequences (the sequences which have not been underlined). In addition or alternatively, the protein comprising the TM1 herein includes one or more additional transmembrane regions (the underlined sequences) in the depicted protein or homologous sequences retaining one, two, three or, possibly four or more of the native V, L I and F amino acids, as set forth in the wild type sequence.

The wild type sequence can be subject to the process as discussed above to select additional transmembrane domain variants as described in Example 1. Coding sequences can be designed, shuffled and proteins expressed. The expressed proteins can be assayed for ligand binding, as described herein.

### Example 23: E. coli expression of QTY variants and a CXCR4-QTY variant

### 1. Large-scale production of CXCR4-QTY in E. coli BL21 (DE3)

A water-soluble GPCR CXCR4 was produced it in E. Coli with a yield estimated to be ∼20mg purified protein per liter of routine LB culture media. The estimated cost of production is about $0.25 per milligram. Advantageously, this approach can be used to easily obtain grams of quantity of water-soluble GPCRs, which in turn can facilitate their structural determinations.

### 2. Determining where the water-soluble CXCR4-QTY is produced in E. Coli cells

A water-soluble CXCR4-QTY was cloned into pET vector. We first carried out a small-scale *E. Coli* culture study to assess the location of produced CXCR4-QTY protein (150ml culture). After culturing the cells, induced with IPTG at 24°C for 4 hours, we collected and sonicated the cells and divided into 2 fractions through centrifugation at 14,637xg (12,000rmp). We then used Western blot analysis of the specific anti-rho-tag monoclonal antibody to detect where the CXCR4-QTY protein was. We observed that the CXCR4-QTY protein was in the supernatant fraction and no protein was observed in the pellet fraction, thus suggesting the protein is fully water-soluble.

### 3. The estimated yield CXCR4-QTY produced in soluble fraction of E. Coli cells

We then carried out another 150ml culture and obtained ∼6mg 1D4 monoclonal antibody-purified CXCR4-QTY. Because we under-estimated the yield (we did not anticipate the surprisingly high yield), we did not use enough affinity 1D4 rho-tag monoclonal antibody beads to capture the produced CXCR4-QTY. Thus, a significant amount CXCR4-QTY protein did not bind to the beads due to the fact that not enough beads were added during purification, and the protein was in the flow-through lane and was further washed out. Despite the significant loss, we are still able to obtain ∼6mg for the 150ml culture as seen from the lanes 8-10 (Elution fractions).

### 4. Measuring the thermostability of purified water-soluble CXCR4-QTY protein

In most cases, structure determines function in proteins. Thus it is important to know if the purified CXCR4-QTY protein produced in *E. Coli* still folds correctly in the typical alpha -helical structure with -50% alpha-helix. We performed secondary structural measurement using Circular dichroism (CD). We observed the CD spectra of purified CXCR4-QTY protein at various temperatures. We measured the thermo-stability of purified CXCR4-QTY protein. We observed that the purified CXCR4-QTY protein is relatively stable up to 55°C, the protein was only partially and gradually denatured, the CD signal reduction was -15%. Between 55°C and 65°C, the denaturation increased toward 65°C, the denaturation transition took place between 65°C and 75°C and the protein was nearly fully denatured at 75°C.

We plotted the temperature vs the ellipticity at 222nm to obtain the melting temperature (Tm) of purified water-soluble CXCR4-QTY protein. From the plot, we estimated that the Tm for purified CXCR4-QTY protein is ∼67°C. This Tm suggests the purified water-soluble CXCR4-QTY protein is quite stable compared to many other soluble proteins. This thermo-stability characteristics facilitates obtaining diffracting crystals, since it is known that the better the thermo-stability, the better the crystal lattice packing, and thus the better the chances to obtain structures.

### 5. Additional G protein-coupled receptors

We selected 10 G protein-coupled receptors (GPCRs) to design the water-soluble form, using the QTY method that is described in Zhang et al., Water Soluble Membrane Proteins and Methods for the Preparation and Use Thereof, U.S. Patent Publication No. 2012/0252719 A ("Zhang"). Alternatively, the proteins described herein can be selected.

### 6. Molecular cloning of the genes.

We successfully verified the GPCR native and QTY genes in the cell-free protein expression plasmid vector pIVex2.3d and *E. Coli* pET28a and pET-duet-1 plasmid vectors.

### 7. Water-soluble GPCR productions

We have produced several native and QTY proteins. When producing native GPCR in the cell-free system, a detergent Brij35 is required, without the detergent, the proteins precipitate upon production. On the other hand, we tested QTY variants in the present and absent of detergent. Without the detergent, the cell-free system produced soluble proteins.

We cloned the QTY variants into *E. Coli in vivo* expression system, pET28a and pET-duet-1 plasmid vectors for *E. Coli* cell protein production in *E. Coli* BL21 (DE3) strain. We have purified several water-soluble GPCR proteins, including CXCR4 and CCR5, which we have used for secondary structural analysis. We have performed ligand-binding studies for CXCR4 with its natural ligand CCL12 (SDF1a). We carried out *E*. *Coli* production and purification of water-soluble GPCR CCR5e variant. The CCR5e variant had 58 amino acid changes (-18% change). The water-soluble GPCR CCR5e variant was purified to homogeneity using the specific monoclonal antibody rhodopsin-tag. The blue stain showed a single band on the SDS gel indicating the purity. Estimated from the protein size marker, it appears to be a pure homo-dimer (the native membrane-bound CXCR4 crystal structure was a dimer. The Western-blot verified the monomer and homo-dimmer of CCR5e variant that is common in GPCRs.

### 8. QTY CCR5e secondary structural studies.

We obtained water-soluble QTY variant of GPCR CCR5e. Then we carried out secondary structural analyses using an Aviv Model 410 circular dichroism instrument and confirmed that the GPCR QTY CCR5-e variant has a typical alpha-helical structure. We also carried out experiments in various temperatures to determine the CCR5e variant Tm, namely, the thermo-stability of the water-soluble CCR5e variant. From the experiments, we determined the Tm of CCR5e variant is about 46°C. This Tm is good for crystal screen experiments.

### 9. Ligand-binding studies of CXCR4 with CCL12 (SDF1a).

In order to be certain the designed water-soluble QTY GPCRs still maintain their biological function, namely recognize and bind to their natural ligands, we first used an ELISA measurement to study water-soluble CXCR4 with its natural ligand CCL12 (also called SDF1a). The assay concentrations range from 50nM to 10µM. The measured Kd is ∼80nM. The Kd of native membrane-bound CXCR4 with SDFla is about 100nM. So the Kd of water-soluble CXCR4 is within acceptable range. Further experiments using more sensitive SPR or other measurement may produce more accurate Kd.

While this invention has been particularly shown and described with references to preferred embodiments thereof, it will be understood by those skilled in the art that various changes in form and details may be made therein without departing from the scope of the invention encompassed by the appended claims.

Embodiments of the invention are set out in the following paragraphs:
1. A computer implemented method for executing a procedure to select a water-soluble variant of a G Protein-Coupled Receptor (GPCR), the method comprising:
   entering a sequence of the GPCR for analysis;
   obtaining a variant of the GPCR, wherein a plurality of hydrophobic amino acids in the transmembrane (TM) domain alpha-helical segments ("TM regions") of the GPCR are substituted, wherein:
      (a) said hydrophobic amino acids are selected from the group consisting of Leucine (L), Isoleucine (I), Valine (V), and Phenylalanine (F);
      (b) each said Leucine (L) is independently substituted by Glutamine (Q), Asparagine (N), or Serine (S);
      (c) each said Isoleucine (I) and said Valine (V) are independently substituted by Threonine (T), Asparagine (N), or Serine (S); and,
      (d) each said Phenylalanine is substituted by Tyrosine (Y); and, subsequently,
   obtaining an α-helical secondary structure result for the variant to verify maintenance of α-helical secondary structures in the variant;
   obtaining a trans-membrane region result for the variant to verify water
   solubility of the variant,
   thereby selecting the water-soluble variant of the GPCR.
2. The method of clause 1, wherein step (3) is performed prior to, concurrently with, or after step (4).
3. The method of clause 1 or 2, wherein in step (2), one subset of said plurality of hydrophobic amino acids in one and the same TM region of the GPCR are substituted to generate one member of a library of potential variants, and one or more different subsets of said plurality of hydrophobic amino acids are substituted to generate additional members of the library.
4. The method of clause 3, further comprising ranking all members of said library based on a combined score, wherein the combined score is a weighed combination of the α-helical secondary structure prediction result and the trans-membrane region prediction result.
5. The method of clause 1 further comprising ranking the variant using a ranking function.
6. The method of clause 1 further comprising performing the method with a data processor.
7. The method of clause 6 further comprising a memory connected to the data processor.
8. The method of clause 5 wherein the ranking function includes a secondary structure component and a water solubility component.
9. The method of clause 8 wherein the ranking function includes a weighting value for the secondary structure component and/or the water solubility component.
10. The method of clause 4, further comprising: selecting N members with the highest combined scores to form a first library of potential variants for said TM region, wherein N is a pre-determined integer (e.g., 3, 4, 5, 6, 7, 8, 9, 10, 11, 12, 13, 14, 15, 16, 17, 18, 19, 20 or more).
11. The method of clause 10, further comprising generating one library of potential variants for 1, 2, 3, 4, 5, or all 6 other TM regions of the GPCR.
12. The method of clause 11 further comprising: replacing two or more TM regions of the GPCR with corresponding TM regions from the libraries of potential variants, to create a library of combinatory variants.
13. The method of any one of clauses 1-12, wherein substantially all *(e.g.,* all) said leucines are substituted by glutamines.
14. The method of any one of clauses 1-13, wherein substantially all (*e.g.,* all) said isoleucines are substituted by threonines.
15. The method of any one of clauses 1-14, wherein substantially all (*e.g.,* all) said valines are substituted by threonines.
16. The method of any one of clauses 1-15, wherein substantially all (*e.g.,* all) said phenylalanines are substituted by tyrosines.
17. The method of any one of clauses 1-16, wherein one or more (*e.g,,* 1, 2, or 3) said leucines are not substituted.
18. The method of any one of clauses 1-17, wherein one or more (*e.g,,* 1, 2, or 3) said isoleucines are not substituted.
19. The method of any one of clauses 1-18, wherein one or more (*e.g,,* 1, 2, or 3) said valines are not substituted.
20. The method of any one of clauses 1-19, wherein one or more (*e.g,,* 1, 2, or 3) said phenylalanines are not substituted.
21. The method of any one of clauses 1-20, further comprising producing / expressing said combinatory variants.
22. The method of any one of clauses 1-21, further comprising testing said combinatory variants for ligand binding (*e.g*., in yeast two-hybrid system), wherein those having substantially the same ligand binding compared to that of the GPCR are selected.
23. The method of any one of clauses 1-22, further comprising testing said combinatory variants for a biological function of the GPCR, wherein those having substantially the same biological function compared to that of the GPCR are selected.
24. The method of any one of clauses 1-23, wherein the library of combinatory variants comprises less than about 2 million members.
25. The method of any one of clauses 1-24, wherein said sequence of the GPCR comprises information about the TM regions of the GPCR.
26. The method of any one of clauses 1-25, wherein said sequence of the GPCR is obtained from a protein structure database (e.g., PDB, UniProt).
27. The method of any one of clauses 1-26, wherein the TM regions of the GPCR are predicted based on the sequence of the GPCR.
28. The method of clause 27, wherein the TM regions of the GPCR are predicted using TMHMM 2.0 (TransMembrane prediction using Hidden Markov Models) software module.
29. The method of clause 28, wherein said TMHMM 2.0 software module utilizes a dynamic baseline for peak searching.
30. The method of any one of clauses 1-29, further comprising providing a polynucleotide sequence for each variants of the GPCR.
31. The method of clause 30, wherein the polynucleotide sequence is codon optimized for expression in a host (e.g., a bacterium such as *E. coli,* a yeast such as S. *cerevisae* or *S. pombe,* an insect cell such as Sf9 cell, a non-human mammalian cell, or a human cell).
32. The method of any one of clauses 1-31, wherein the scripted procedure comprises VBA scripts.
33. The method of any one of clauses 1-32, wherein the scripted procedure is operable with a Linux system (e.g., Ubuntu 12.04 LTS), a Unix system, a Microsoft Windows operative system, am Android operative system, or an Apple iOS operative system.
34. A water-soluble variant of a G Protein-Coupled Receptor (GPCR), wherein:
   a plurality of hydrophobic amino acids in the transmembrane (TM) domain alpha-helical segments ("TM regions") of the GPCR are substituted, wherein:
      (a) said hydrophobic amino acids are selected from the group consisting of Leucine (L), Isoleucine (I), Valine (V), and Phenylalanine (F);
      (b) each said Leucine (L) is independently substituted by Glutamine (Q), Asparagine (N), or Serine (S);
      (c) each said Isoleucine (I) and said Valine (V) are independently substituted by Threonine (T), Asparagine (N), or Serine (S); and,
      (d) each said Phenylalanine is substituted by Tyrosine (Y); and, subsequently,
   all seven TM regions of the variant maintains α-helical secondary structures; and,
   there is no predicted trans-membrane region.
35. The water-soluble variant of clause 34, comprising one or more amino acid sequences selected from the group consisting of SEQ ID NOs: 4-11, 13-20, 22-29, 31-38, 40-47, 49-56, and 58-64.
36. The water-soluble variant of clause 35, further comprising one or more amino acid sequences selected from the group consisting of SEQ ID NOs: 3, 12, 21, 30, 39, 48, and 57.
37. The water-soluble variant of clause 35 or 36, which binds to a CXCR4 ligand.
38. The water-soluble variant of clause 34 comprising one or more amino acid sequences selected from the group consisting of SEQ ID NOs: 69-76, 78-85, 87, 89-96, 98-105, 107-114 and 116-123.
39. The water-soluble variant of clause 38, further comprising one or more amino acid sequences selected from the group consisting of SEQ ID NOs: 68, 77, 86, 88, 97, 106, 115 and 124.
40. The water-soluble variant of clause 38 or 40, which binds to a CX3CR1 ligand.
41. The water-soluble variant of clause 34, comprising one or more amino acid sequences selected from the group consisting of SEQ ID NOs: 128-135, 137-144, 146-153, 155-162, 164-171, 173 and 175-182.
42. The water-soluble variant of clause 41, further comprising one or more amino acid sequences selected from the group consisting of SEQ ID NOs: 127, 136, 145, 154, 163, 172, 174 and 183.
43. The water-soluble variant of clause 41 or 42, which binds to a CCR3 ligand.
44. The water-soluble variant of clause 34, comprising one or more amino acid sequences selected from the group consisting of SEQ ID NOs: 187-194, 196-203, 205-206, 208, 210-217, 219-225, 227-234.
45. The water-soluble variant of clause 44, further comprising one or more amino acid sequences selected from the group consisting of SEQ ID NOs: 186, 195, 204, 207, 209, 218, 226, and 235.
46. The water-soluble variant of clause 44 or 45, which binds to a CCR5 ligand.
47. The water-soluble variant of clause 34, comprising one or more amino acid sequences selected from the group consisting of SEQ ID NOs: 236-243, 245-252, 254-261, 263-270, 272, 274-281, and 283-290.
48. The water-soluble variant of clause 47, further comprising one or more amino acid sequences selected from the group consisting of SEQ ID NOs: 235, 244, 253, 262, 271, 273, 282 and 291.
49. The water-soluble variant of clause 47 or 48, which binds to a CXCR3 ligand.
50. The water-soluble variant of clause 34, comprising one or more transmembrane domains as set forth in any one of SEQ ID NOs: 2, 67, 126, 185, 327, 293, 295, 297, 299, 301, 303, 305, 307, 309, 311, 313, 315, 317, 319, 321, 323 or 325.
51. The water-soluble variant of clause 50, wherein the water-soluble variant is water soluble and binds a ligand of a homologous native transmembrane protein.
52. A method of producing a protein in a bacterium (e.g., an *E. coli*), comprising:
   (a) culturing the bacterium in a growth medium under a condition suitable for protein production;
   (b) fractioning a lysate of the bacterium to produce a soluble fraction and the insoluble pellet fraction; and,
   (c) isolating the protein from the soluble fraction;
   wherein:
   (1) the protein is a variant G-protein couple receptor (GPCR) of any one of clauses 29-46; and,
   (2) the yield of the protein is at least 20 mg/L (e.g., 30 mg/L, 40 mg/L, 50 mg/L or more) of growth medium.
53. The method of clause 47, wherein the bacterium is *E. coli* BL21, and the growth medium is LB medium.
54. The method of clause 47 or 48, wherein the protein is encoded by a plasmid in the bacterium.
55. The method of any one of clauses 47-49, wherein expression of the protein is under the control of an inducible promoter.
56. The method of clause 50, wherein the inducible promoter is inducible by IPTG.
57. The method of any one of clauses 47-51, wherein the lysate is produced by sonication.
58. The method of any one of clauses 47-52, wherein the soluble fraction is produced by centrifuging the lysate at 14,500 × g or more.
59. A non-transitory computer readable medium having stored thereon a sequence of instructions to perform the method of any of clauses 1-33.
60. A data processing system operative to select a water soluble variant of a membrane protein comprising:
   a data processor operative to perform substitution of amino acids and wherein the system ranks a protein variant with a ranking function, the ranking function including a secondary structure component and a water solubility component.
61. The system of clause 60 further comprising a library of membrane proteins for processing by the system.
62. The system of clause 60 further comprising a memory connected to the data processor that stores coded instruction to execute a substitution processor.
63. The system of clause 60 wherein the system is operative to process steps (a), (b), (c) and (d) of clause 1.
64. The system of clause 60 further comprising a ranking function that is a weighted combination based on the secondary structure component.
65. The system of clause 60 wherein the system communicates with an external program via a network.
66. The system of clause 60 further comprising a database for storing water soluble variants.
67. The system of clause 60 further comprising instruction to perform dynamic baseline processing.
68. The system of clause 60 further comprising an interface to select process parameters.
69. The system of clause 60 further comprising entering sequences as set forth in clause 35-50.
70. A computer implemented method for executing a procedure to select a water-soluble variant of a method comprising:
   operating a data processing to identify a sequence of a membrane protein for analysis;
   obtaining a variant of the membrane protein, wherein a plurality of hydrophobic amino acids in the transmembrane (TM) domain alpha-helical segments ("TM regions") of the membrane protein are substituted, wherein the data processor:
      determines an α-helical secondary structure result for the variant to verify maintenance of α-helical secondary structures in the variant;
      determines trans-membrane region result for the variant to verify water solubility of the variant; and
      selects a water-soluble variant of the membrane protein.
71. The method of clause 70, wherein the substitution include:
   (a) hydrophobic amino acids are selected from the group consisting of Leucine (L), Isoleucine (I), Valine (V), and Phenylalanine (F);
   (b) each Leucine (L) is independently substituted by Glutamine (Q), Asparagine (N), or Serine (S);
   (c) each Isoleucine (I) and said Valine (V) are independently substituted by Threonine (T), Asparagine (N), or Serine (S); and,
   (d) each said Phenylalanine is substituted by Tyrosine (Y).
72. The method of clause 71, wherein one subset of said plurality of hydrophobic amino acids in one and the same TM region of a GPCR are substituted to generate one member of a library of potential variants, and one or more different subsets of said plurality of hydrophobic amino acids are substituted to generate additional members of the library.
73. The method of clause 70, further comprising ranking all members of said library based on a combined score, wherein the combined score is a weighed combination of the α-helical secondary structure prediction result and the trans-membrane region prediction result.
74. The method of clause 70 further comprising ranking the variant using a ranking function.
75. The method of clause 70 further comprising a memory connected to the data processor.
76. The method of clause 74 wherein the ranking function includes a secondary structure component and a water solubility component.
77. The method of clause 76 wherein the ranking function includes a weighting value for the secondary structure component and/or the water solubility component.
78. The method of clause 73, further comprising: selecting N members with the highest combined scores to form a first library of potential variants for said TM region, wherein N is a pre-determined integer (e.g., 3, 4, 5, 6, 7, 8, 9, 10, 11, 12, 13, 14, 15, 16, 17, 18, 19, 20 or more).
79. The method of clause 78, further comprising generating one library of potential variants for 1, 2, 3, 4, 5, or all 6 other TM regions of a GPCR.
80. The method of clause 79 further comprising: replacing two or more TM regions of the GPCR with corresponding TM regions from the libraries of potential variants, to create a library of combinatory variants.

## Claims

1. A water-soluble variant of a G Protein-Coupled Receptor (GPCR), wherein:
a plurality of hydrophobic amino acids in the transmembrane (TM) domain alpha-helical segments ("TM regions") of the GPCR are substituted, wherein:
(a) said hydrophobic amino acids are selected from the group consisting of Leucine (L), Isoleucine (I), Valine (V), and Phenylalanine (F);
(b) each said Leucine (L) is independently substituted by Glutamine (Q), Asparagine (N), or Serine (S);
(c) each said Isoleucine (I) and said Valine (V) are independently substituted by Threonine (T), Asparagine (N), or Serine (S); and,
(d) each said Phenylalanine is substituted by Tyrosine (Y); and, subsequently,
all seven TM regions of the variant maintains α-helical secondary structures; and, there is no predicted trans-membrane region.

2. The GPCR of claim 1, wherein substantially all i*(e.g.,* all) said leucines are substituted by glutamines; and,
optionally, substantially all (*e.g.,* all) said isoleucines are substituted by threonines, and,
optionally substantially all (*e.g.,* all) said valines are substituted by threonines, and,
optionally, substantially all (*e.g.,* all) said phenylalanines are substituted by tyrosines.

3. The GPCR of claim 1 or 2, wherein one or more (*e.g*., 1, 2, or 3) of said leucines are not substituted, and,
optionally, one or more (*e.g*., 1, 2, or 3) of said isoleucines are not substituted, and,
optionally, one or more (*e.g*., 1, 2, or 3) of said valines are not substituted, and,
optionally, one or more (*e.g*., 1, 2, or 3) of said phenylalanines are not substituted.

4. The GPCR of claim 1, wherein all said leucines are substituted by glutamines; and,
optionally, all said isoleucines are substituted by threonines, and,
optionally all said valines are substituted by threonines, and,
optionally, all said phenylalanines are substituted by tyrosines.

5. The GPCR of claim 1, wherein all said leucines are substituted by glutamines; all said isoleucines are substituted by threonines; all said valines are substituted by threonines; and, all said phenylalanines are substituted by tyrosines.

6. A method of producing a protein in a bacterium (e.g., an *E*. *coli*), comprising:
(a) culturing the bacterium in a growth medium under a condition suitable for protein production;
(b) fractioning a lysate of the bacterium to produce a soluble fraction and the insoluble pellet fraction; and,
(c) isolating the protein from the soluble fraction;
wherein:
(1) the protein is a variant G-protein couple receptor (GPCR) of any one of claims 1-5; and,
(2) the yield of the protein is at least 20 mg/L (e.g., 30 mg/L, 40 mg/L, 50 mg/L or more) of growth medium.

7. The method of claim 6, wherein the bacterium is *E*. *coli* BL21, and the growth medium is LB medium.

8. The method of claim 6 or claim 7, wherein the protein is encoded by a plasmid in the bacterium.

9. The method of any one of claims 6-8, wherein expression of the protein is under the control of an inducible promoter.

10. The method of claim 9, wherein the inducible promoter is inducible by IPTG.

11. The method of any one of claims 6-10, wherein the lysate is produced by sonication.

12. The method of any one of claims 6-11, wherein the soluble fraction is produced by centrifuging the lysate at 14,500 × g or more.
